# EUROPEAN PATENT APPLICATION

(11) **EP 2 679 677 A1**
(43) Date of publication of application: **01.01.2014**
(21) Application number: 12174467.6
(22) Date of filing: 29.06.2012
(51) Int. Cl.: C12N 9/24, C07K 19/00, A61K 39/04

(54) **Composition for use in Mycobacteria therapy**

(71) Applicant: Lysando Aktiengesellschaft, 9497 Triesenberg (LI)
(72) Inventor: Miller, Stefan, 93055 Regensburg (DE)
(74) Representative: Dehmel, Albrecht

(57) **Abstract**

The present invention relates to a composition having the activity of degrading the cell wall of a *Mycobacterium species*. Moreover, the present invention relates in particular to said composition for use as a medicament, a disinfectant, a feed additive, or a food additive as well as the use of said composition in the treatment or prevention of diseases caused by an infection with a *Mycobacterium species.*

SEQUENCE LISTING

<110>  LYSANDO AG

<120>  COMPOSITION FOR USE IN MYCOBACTERIA THERAPY

<130>  LYS-011 EP

<140>  unkown
<141>  2012-06-29

<160>  76

<170>  PatentIn version 3.3

<210>  1
<211>  547
<212>  PRT
<213>  Artificial sequence

<220>
<223>  TM4gp29 Lysin A

<400>  1

Met Ser Phe Thr Arg Phe Leu Gln Asp Asp Pro Leu Leu Thr Arg Glu
1               5                   10                  15

Gln Val Met Ala Glu Leu Ile Arg Val Ala Asp Glu Leu Asn Met Pro
            20                  25                  30

Asp Lys Arg Gly Ala Cys Val Ile Ala Gly Met Thr Ile Ser Gln Glu
        35                  40                  45

Val Gly Val Lys Asp Asn Asp Pro Pro Phe Glu Arg Arg Phe Trp Cys
    50                  55                  60

Pro Ala Asn Arg Ala Asp Pro Glu Ser Phe Asn Tyr Pro His Asp Ser
65                  70                  75                  80

Glu Ser Asn Asp Gly Arg Ser Val Gly Tyr Phe Gln Gln Gln Lys Gly
                85                  90                  95

Pro Asn Gly Glu Leu Trp Trp Gly Thr Thr Ala Ser Glu Met Asn Leu
            100                 105                 110

His Ser Ala Ala Thr Gln Phe Met Thr Arg Leu Lys Ala Ala Gly Tyr
        115                 120                 125

Asn Ala Ser Asn Ala Gln Ala Ala Asn Asp Ser Ala Gln Ala Ile Gln
    130                 135                 140

Arg Ser Gly Val Pro Gln Ala Tyr Lys Gln Trp Trp Asp Asp Ile Asn
145                 150                 155                 160

Arg Leu Tyr Asp Lys Val Lys Gly Ser Gly Gly Gly Pro Ala Pro Ala
                165                 170                 175

Pro Lys Pro Pro Gln Ser Gly Pro Trp Thr Gly Asp Pro Val Trp Leu
            180                 185                 190

Ala Asp Val Leu Arg Ala Glu Gly Leu Asn Val Val Glu Leu Pro Gly
        195                 200                 205

Trp Leu Asp Arg Gly His Gly Asp Met Gly Arg Leu Trp Gly Val Val
    210                 215                 220

Cys His His Thr Gly Ser Asp Asn Thr Pro Ser Ser Glu Ile Ala Phe
225                 230                 235                 240

His Pro Ser Leu Gly Leu Cys Ser Gln Ile His Leu Ala Arg Asn Gly
                245                 250                 255

Thr Val Thr Leu Cys Gly Val Gly Ile Ala Trp His Ala Gly Val Gly
            260                 265                 270

Ser Tyr Pro Gly Leu Pro Glu Asp Asn Ala Asn Ala Val Thr Ile Gly
        275                 280                 285

Ile Glu Ala Gln Asn Ser Gly Thr Tyr Asp Gly Ala Pro His Arg Thr
    290                 295                 300

Asn Trp Pro Asp Ala Gln Tyr Asp Ala Tyr Val Lys Cys Cys Ala Ala
305                 310                 315                 320

Ile Cys Arg Arg Leu Gly Val Arg Ala Asp His Val Ile Ser His Lys
                325                 330                 335

Glu Trp Ala Gly Arg Lys Gln Gly Lys Trp Asp Pro Gly Ala Ile Asp
            340                 345                 350

Met Asn Ile Phe Arg Ala Asp Val Gln Arg Arg Ile Asp Ala His Gln
        355                 360                 365

Pro Asn Gly Glu Asp Asp Phe Met Ala Ala Leu Ser Ala Asp Glu Gln
    370                 375                 380

Arg Glu Val Leu Asn Leu Leu Arg Val Leu Ala Asp Arg Arg Phe Val
385                 390                 395                 400

Ser Arg Ser Pro Phe Arg His Leu Gly Glu Gly Pro Ser Glu Thr Val
                405                 410                 415

Ala Gly Phe Gly Leu Asn Thr Asp Gly Leu Asn His Ala Gln Tyr Thr

420                 425                 430

Ile Glu Leu Ala Arg Leu Gly Asp Pro Thr His Leu Ala Leu Leu Arg
        435                 440                 445

Glu Val Ala Ser Ala Glu Gly Asp Ser Arg Tyr Pro Asp Arg Gln Tyr
    450                 455                 460

Asp Ala Lys Leu Ala Lys Arg Val Leu Ala Glu Ile Glu Gly Ala Ala
465                 470                 475                 480

Thr Ala Pro Ala Lys Pro Ser Thr Pro Ser Ala Pro Thr Glu Pro Ala
                485                 490                 495

Pro Glu Ala Pro Thr Pro Pro Val Lys Ala Ala Cys Ala Leu Ser Ala
            500                 505                 510

Ala Gly Cys Val Val Ala Gly Ser Thr Ser Gly Gly Gly Cys Ala Leu
        515                 520                 525

Ser Thr Asp Gly Thr Gly Lys Cys Val Val Thr Ala Ala Thr Asp Gly
    530                 535                 540

Gly Ala Ala
545

<210>  2
<211>  1644
<212>  DNA
<213>  Artificial sequence

<220>
<223>  TM4gp29 Lysin A

<400>  2
atgagtttca cccggttcct gcaggatgac ccgctgctca cccgcgagca agtgatggcc      60

gagctgattc gggtcgccga cgagctgaac atgcccgaca agcgcggcgc ctgcgtcatt     120

gcgggcatga cgatttcgca agaggtcggc gtaaaggaca acgacccgcc gttcgagcgg     180

cggttctggt gcccggccaa ccgcgccgac cccgaatcgt tcaactaccc gcacgactcg     240

gaatcgaacg acggccgctc ggtcggctac ttccagcagc agaaggggcc taacggcgag     300

ctgtggtggg gcacaacggc atccgagatg aacctgcaca gcgccgcgac gcagtttatg     360

acgcggctca aggcggccgg atacaacgcg agcaacgccc aggcggcgaa cgactcggcg     420

caggcgatcc agcggtcggg cgtcccgcag gcgtacaagc aatggtggga cgacattaac     480

cgcctgtacg acaaggtgaa gggctcgggc ggtggcccgg cgcccgcgcc taagccgccg     540

cagtcggggc cgtggaccgg cgacccggtg tggctggccg acgtgctgcg cgccgagggg     600

ctgaacgtcg tcgagctgcc cggctggctc gaccgcgggc acggcgacat gggccgcttg     660

tggggcgtgg tgtgccatca caccggcagc gataacaccc cgtcgagcga gattgcgttt 720

cacccgtcgc tcggcctgtg ctcgcagatt cacctggcgc gcaacggaac tgtgacgctg 780

tgcggtgtcg gcatcgcctg gcatgcgggc gtcggcagct atcccggcct gcccgaggac 840

aacgccaacg cggtcactat cggcatcgag gcccaaaaca gcggcaccta tgacggcgca 900

ccgcaccgga cgaattggcc tgacgcgcaa tacgacgcct atgtgaagtg ctgcgccgcg 960

atctgccgcc gcctcggcgt gcgcgccgat cacgtgatca gtcacaagga atgggccggg 1020

cgcaagcaag gcaaatggga tccaggcgcc atcgacatga acatctttcg cgccgacgta 1080

cagcggcgca tcgacgccca tcaaccaaac ggagaggacg atttcatggc cgcactatca 1140

gccgacgagc agcgcgaggt gctgaacctg ctgcgcgtcc tggccgaccg gcggttcgtc 1200

agccgcagcc cgttccgcca ccttggcgag gggccgagcg aaactgtcgc cgggttcggg 1260

ctcaacaccg acggcctcaa tcacgcgcag tacacgattg agcttgcgcg cctgggcgac 1320

ccgacgcacc tcgccctgct gcgcgaggtc gccagcgccg agggtgactc gcgctatccc 1380

gaccggcagt acgacgccaa gctcgccaag cgcgtgctcg ccgaaatcga gggcgccgca 1440

acggcaccgg ccaagccgag cacgccgagc gccccgaccg agcccgcccc cgaggcgccc 1500

acgccgccgg tcaaggccgc gtgtgcgctg tctgcggccg ggtgcgtggt ggctggctcg 1560

acctcgggcg gtggctgcgc cctgtccacc gacggcaccg gcaagtgcgt tgtgaccgcc 1620

gcgaccgacg gcggggccgc ctga 1644

<210> 3
<211> 514
<212> PRT
<213> Artificial sequence

<220>
<223> BXZ2gp11 Lysin A

<400> 3

Met Thr Glu Lys Val Leu Pro Tyr Asp Arg Ser Ile Val Thr Gln Glu
1               5                   10                  15

Thr Gly Trp Trp Cys Gly Pro Ala Ala Thr Gln Val Val Leu Asn Ser
            20                  25                  30

Arg Gly Ile Ile Val Pro Glu Ala Thr Leu Ala Ala Glu Ile Glu Ala
        35                  40                  45

Ile Glu Asn Pro Gly Arg Gly Asp Asp Arg Asp Gly Thr Asp Tyr Val
    50                  55                  60

Gly Leu Ile Glu Gln Val Leu Asp Arg Arg Val Pro Gln Ala Arg Tyr
65                  70                  75                  80

Thr Ser Val Tyr Leu Thr Asn Asp Pro Pro Thr Gln Ala Gln Lys Asp
                85                  90                  95

Arg Leu Trp Glu His Ile Val Arg Ser Ile Asn Ala Gly Tyr Gly Val
            100                 105                 110

Val Met Asn Trp Val Ala Pro Pro Ser Asn Lys Pro Arg Gly Val Lys
        115                 120                 125

Gly Ser Val Ser Pro Arg Tyr Ser Gly Gly Thr Thr Tyr His Tyr Val
    130                 135                 140

Ala Cys Met Gly Tyr Asp Asp Thr Pro Gly Ala Arg Ala Val Trp Ile
145                 150                 155                 160

Ala Asp Ser Gly Phe Gln Pro Gln Gly Tyr Trp Ile Ser Phe Asp Gln
                165                 170                 175

Cys Ala Thr Leu Ile Pro Pro Lys Gly Tyr Ala Tyr Ala Asp Ala Ala
            180                 185                 190

Pro Ala Ala Pro Ala Pro Ala Pro Thr Pro Val Val Asp Ala Ala Pro
        195                 200                 205

Ile Leu Ala Arg Ala Ala Gly Ile Ser Glu Ala Lys Ala Arg Glu Ile
    210                 215                 220

Leu Pro Thr Met Arg Asp Gly Leu Lys Gln Ala Asp Cys Thr Thr Val
225                 230                 235                 240

Asn Arg Ile Ala Met Phe Ile Ala Gln Thr Gly His Glu Ser Asp Asp
                245                 250                 255

Phe Arg Ala Thr Glu Glu Tyr Ala Asn Gly Pro Leu Asp Gln Glu Arg
            260                 265                 270

Trp Ile Tyr Lys Gly Arg Thr Trp Ile Gln Ile Thr Trp Arg Glu His
        275                 280                 285

Tyr Ala Arg Phe Gly Lys Trp Cys Phe Asp Arg Gly Leu Val Thr Asp
    290                 295                 300

Pro Asp Val Phe Val Lys Asn Pro Arg Ala Leu Ala Asp Leu Lys Trp
305                 310                 315                 320

Ala Gly Ile Gly Ala Ala Trp Tyr Trp Thr Val Glu Arg Pro Asp Ile
                325                 330                 335

Asn Ala Leu Cys Asp Arg Arg Asp Ile Glu Thr Val Ser Arg Arg Ile
            340                 345                 350

Asn Gly Thr Asn Pro Asn Thr Gly Arg Ala Asn His Ile Glu Glu Arg
        355                 360                 365

Ile Ala Arg Trp Asn Arg Ala Leu Ala Val Gly Asp Asp Leu Leu Gln
    370                 375                 380

Leu Ile Arg Glu Glu Glu Asp Gly Phe Leu Ser Ala Leu Thr Pro Ala
385                 390                 395                 400

Glu Gln Arg Ala Leu Tyr Asn Glu Ile Met Lys Lys Gly Pro Thr Arg
                405                 410                 415

Ser Phe Met Ala Glu Asp Gln Asn Gln Ile Glu Thr Leu Leu Gly Phe
            420                 425                 430

Val Tyr Asn Ile Asp Gly Asn Ile Trp Asn Asp Ala Val Thr Arg Ala
        435                 440                 445

Tyr Leu Phe Asp Val Pro Leu Ala Val Glu Tyr Val Glu Arg Val Ala
    450                 455                 460

Arg Asp Gly Val His Pro Lys Ser Trp Ala Phe Gln Gln Leu Asp Gly
465                 470                 475                 480

Lys Gly Glu Arg Trp Leu Ala Lys Phe Gly Gln Glu Tyr Cys Lys Gly
                485                 490                 495

Leu Ile Arg Phe Lys Lys Lys Leu Asn Asp Leu Leu Glu Pro Tyr Gly
            500                 505                 510

Glu Asn

<210>  4
<211>  1545
<212>  DNA
<213>  Artificial sequence

<220>
<223>  Bxz2gp11 Lysin A

<400>  4
atgacggaga aggtacttcc ctacgaccgc agcatcgtca cgcaggagac cggctggtgg      60

tgtggccctg cggccaccca ggtcgtgctc aactcgcgag gcatcatcgt cccggaggcc     120

acgctcgctg ccgagatcga ggccatcgag aaccccggac ggggtgacga ccgcgacggc     180

accgactacg tcggcctgat cgagcaggtg ctggatcgcc gtgtgccgca ggcgcgctac     240

acgtcggtct acctgacgaa cgatccgccc acgcaggctc agaaggaccg gctgtgggag 300

cacatcgtcc ggtcgatcaa cgcgggctac ggcgtggtca tgaactgggt cgcgcctccg 360

tcgaacaagc cacgcggagt gaagggctcg gtgagcccgc gctactcggg cggcaccacg 420

taccactacg tcgcgtgcat gggctacgac gacacccccg gtgctcgggc ggtctggatc 480

gccgacagcg gcttccagcc gcagggctac tggatctcgt tcgaccagtg cgccacgctg 540

atcccgccga agggctacgc gtacgccgac gccgcaccgg ctgctcccgc acccgcaccg 600

acccccgtgg tcgacgccgc gccgatcctg gcgcgtgctg cgggcatctc cgaggccaag 660

gcccgcgaga tcctgccgac gatgcgtgac gggctgaagc aggccgactg caccaccgtc 720

aaccggatcg cgatgttcat cgcccagacc ggccacgagt ccgacgactt ccgggccacc 780

gaggagtacg ccaacggtcc cctggaccag gagcgctgga tctacaaggg acgcacctgg 840

attcagatca cctggcgcga gcactacgcc cggttcggga agtggtgctt cgaccgcggc 900

ctggtgaccg accccgacgt gttcgtcaag aacccgcgtg cgctggccga tctgaagtgg 960

gccggcatcg gcgcggcctg gtactggacg gtcgagcgcc cggacatcaa cgcgctgtgc 1020

gaccgccgcg acatcgagac ggtctcgcga cggatcaacg ggacgaaccc gaacaccgga 1080

cgcgccaacc acatcgaaga gcggatcgcc cgctggaacc gcgcactcgc ggtcggtgac 1140

gacctgctgc aacttatccg agaggaggag gacggcttct tgtccgcact cacacccgct 1200

gaacagcgcg ctctctacaa cgagatcatg aagaagggtc cgacccggtc gttcatggcc 1260

gaggaccaga accagatcga gacgctgctc ggcttcgtct acaacatcga cggcaacatc 1320

tggaacgacg cggtgacccg cgcctacctg ttcgacgtgc cactggctgt tgagtacgtc 1380

gagcgcgttg ctcgcgacgg cgtccacccg aagtcgtggg cgttccagca gctcgacggc 1440

aagggcgagc gctggctggc caagttcggc caggagtact gcaagggcct gatccgcttc 1500

aagaagaagc tgaacgacct gcttgagccg tacggggaga actga 1545

<210> 5
<211> 493
<212> PRT
<213> Artificial sequence

<220>
<223> D29gp10 Lysin A

<400> 5

Met Thr Leu Ile Val Thr Arg Asp His Ala Gln Trp Val His Asp Met
1               5                   10                  15

Cys Arg Ala Arg Ala Gly Asn Arg Tyr Gly Tyr Gly Gly Ala Phe Thr
            20                  25                  30

Leu Asn Pro Arg Asp Thr Thr Asp Cys Ser Gly Leu Val Leu Gln Thr
        35                  40                  45

Ala Ala Trp Tyr Gly Gly Arg Lys Asp Trp Ile Gly Asn Arg Tyr Gly
    50                  55                  60

Ser Thr Glu Ser Phe Arg Leu Asp His Lys Ile Val Tyr Asp Leu Gly
65                  70                  75                  80

Phe Arg Arg Leu Pro Pro Gly Gly Val Ala Ala Leu Gly Phe Thr Pro
                85                  90                  95

Val Met Leu Val Gly Leu Gln His Gly Gly Gly Gly Arg Tyr Ser His
            100                 105                 110

Thr Ala Cys Thr Leu Met Thr Met Asp Ile Pro Gly Gly Pro Val Lys
        115                 120                 125

Val Ser Gln Arg Gly Val Asp Trp Glu Ser Arg Gly Glu Val Asn Gly
    130                 135                 140

Val Gly Val Phe Leu Tyr Asp Gly Ala Arg Ala Trp Asn Asp Pro Leu
145                 150                 155                 160

Phe His Asp Phe Trp Tyr Leu Asp Ala Lys Leu Glu Asp Gly Pro Thr
                165                 170                 175

Gln Ser Val Asp Ala Ala Glu Ile Leu Ala Arg Ala Thr Gly Leu Ala
            180                 185                 190

Tyr Asn Arg Ala Val Ala Leu Leu Pro Ala Val Arg Asp Gly Leu Ile
        195                 200                 205

Gln Ala Asp Cys Thr Asn Pro Asn Arg Ile Ala Met Trp Leu Ala Gln
    210                 215                 220

Ile Gly His Glu Ser Asp Asp Phe Lys Ala Thr Ala Glu Tyr Ala Ser
225                 230                 235                 240

Gly Asp Ala Tyr Asp Thr Arg Thr Asp Leu Gly Asn Thr Pro Glu Val
                245                 250                 255

Asp Gly Asp Gly Arg Leu Tyr Lys Gly Arg Ser Trp Ile Met Ile Thr
            260                 265                 270

Gly Lys Asp Asn Tyr Arg Asp Phe Ser Arg Trp Ala His Gly Arg Gly
        275                 280                 285

Leu Val Pro Thr Pro Asp Tyr Phe Val Val His Pro Leu Glu Leu Ser
    290                 295                 300

Glu Leu Arg Trp Ala Gly Ile Gly Ala Ala Trp Tyr Trp Thr Val Glu
305                 310                 315                 320

Arg Pro Asp Ile Asn Ala Leu Ser Asp Arg Arg Asp Leu Glu Thr Val
                325                 330                 335

Thr Arg Arg Ile Asn Gly Gly Leu Thr Asn Leu Asp Asp Arg Arg Arg
            340                 345                 350

Arg Tyr Asn Leu Ala Leu Ala Val Gly Asp Gln Leu Leu Thr Leu Ile
        355                 360                 365

Gly Asp Asp Asp Glu Leu Ala Asp Pro Thr Ile Gln Arg Phe Ile Arg
    370                 375                 380

Glu Ile His Gly Ala Leu Phe Asn Thr Val Val Thr Gln Ser Pro Tyr
385                 390                 395                 400

Gly Asp Pro Gln Asn Pro Asp Gly Ser Glu Pro Arg Ser Asn Leu Trp
                405                 410                 415

Gln Leu His Glu Leu Ile Lys Asn Gly Asp Gly Met Gly His Ala Arg
            420                 425                 430

Tyr Val Glu Glu Ser Ala Arg Ala Gly Asp Leu Arg Glu Leu Glu Arg
        435                 440                 445

Val Val Arg Ala Ala Lys Gly Leu Gly Arg Asp Arg Ser Pro Glu Phe
    450                 455                 460

Ile Ala Arg Ala Arg Asn Val Leu Ala Gln Ile Glu Ala Ala Asn Pro
465                 470                 475                 480

Glu Tyr Leu Gln Ala Tyr Ile Ala Arg Asn Gly Ala Leu
                485                 490

<210>  6
<211>  1482
<212>  DNA
<213>  Artificial sequence

<220>
<223>  D29gp10 Lysin A

<400>  6
   atgacgctca tagtcacacg cgaccacgcg cagtgggtcc acgacatgtg ccgcgctcgc      60
   gctggcaaca ggtacggcta cggcggggcg ttcacactca acccccgaga caccaccgac     120
   tgctcgggtc tggttctgca gacggcagcc tggtacggcg gtcggaagga ctggatcgga     180
   aaccggtacg gctcgactga gagcttccgg ctcgaccaca agatcgtcta cgacctcggg     240
   ttcaggcgac tccctccggg aggcgttgcg gccctgggat tcaccccggt catgctcgtc 300
   gggctccagc acggcggcgg gggccggtac tcgcacaccg cttgcacgct gatgacgatg 360
   gacatccccg gtggcccggt gaaggtctcg caacgaggcg tcgactggga gtcccgagga 420
   gaagtcaacg gcgtgggggt gttcctctac gacggcgcac gcgcctggaa cgacccgctc 480
   ttccacgact tctggtacct ggacgcgaag cttgaagacg gcccgacgca gagtgtcgac 540
   gctgccgaaa tcctcgctcg cgcaacgggt ctcgcgtaca accgagcggt agcactgctg 600
   ccggccgtgc gtgacggcct catccaggcc gactgcacca acccgaatcg catcgcgatg 660
   tggctcgccc agatcggcca tgagtcagac gatttcaagg ccactgcgga gtacgccagc 720
   ggggacgcct acgacacccg aaccgacctc ggcaacaccc cggaggtcga cggagacggt 780
   cggctctaca agggccggtc ctggatcatg atcacgggca aggacaacta ccgggacttc 840
   tcccggtggg ctcacggcag gggcctggtc cccacgcccg actacttcgt ggttcacccg 900
   ctggagctgt cggagctgcg ctgggcaggc atcggtgccg cctggtactg gaccgtcgag 960
   cgcccagaca tcaacgcact cagcgaccgc cgcgacctcg aaacggtcac gcgccggatc 1020
   aacggcgggc tcaccaacct cgatgaccgc cgacgccggt acaacctggc cctcgctgtg 1080
   ggcgaccaac tactgactct gatcggagat gacgacgaat tggctgatcc aacgattcag 1140
   cggttcatcc gcgagatcca cggggcgctg ttcaacaccg tcgtgacgca gtccccctac 1200
   ggcgacccgc agaacccgga cggctcggag ccccggagca acctctggca gctccatgag 1260
   ctgatcaaga acggcgacgg catggggcac gcccgctacg tcgaggaatc ggcgcgagcc 1320
   ggtgacctcc gcgagctgga gcgagttgtc cgcgccgcca agggacttgg tagggatcgc 1380
   tcccccgagt tcatcgcacg cgctcggaac gtgctggccc agatcgaggc agccaacccc 1440
   gagtacctac aggcgtacat cgccaggaat ggagccctat ga 1482

<210> 7
<211> 292
<212> PRT
<213> Artificial sequence

<220>
<223> L5gp10 Lysin A

<400> 7

Met Thr Phe Thr Val Thr Arg Glu Arg Ala Gln Trp Val His Asp Met
1               5                   10                  15

Ala Arg Ala Arg Asp Gly Leu Pro Tyr Ala Tyr Gly Gly Ala Phe Thr
            20                  25                  30

Asn Asn Pro Arg Val Ser Thr Asp Cys Ser Gly Leu Val Leu Gln Thr
        35                  40                  45

Gly Ala Trp Tyr Gly Gly Arg Thr Asp Trp Val Gly Asn Arg Tyr Gly
    50                  55                  60

Ser Thr Glu Ser Phe Arg Leu Asp His Lys Ile Val Tyr Asp Leu Gly
65                  70                  75                  80

Phe Lys Arg Met Pro Arg Gly Gly Pro Ala Ala Leu Pro Ile Lys Pro
                85                  90                  95

Val Met Leu Val Gly Leu Gln His Gly Gly Gly Gly Val Tyr Ser His
            100                 105                 110

Thr Ala Cys Thr Leu Met Thr Met Asp His Pro Gly Gly Pro Val Lys
        115                 120                 125

Met Ser Asp Arg Gly Val Asp Trp Glu Ser His Gly Asn Arg Asn Gly
    130                 135                 140

Val Gly Val Glu Leu Tyr Glu Gly Ala Arg Ala Trp Asn Asp Pro Leu
145                 150                 155                 160

Phe His Asp Phe Trp Tyr Leu Asp Ala Val Leu Glu Asp Glu Gly Asp
                165                 170                 175

Asp Asp Glu Leu Ala Asp Pro Val Leu Gly Lys Met Ile Arg Glu Ile
            180                 185                 190

His Ala Cys Leu Phe Asn Gln Thr Ala Ser Thr Ser Asp Leu Ala Thr
        195                 200                 205

Pro Gly Glu Gly Ala Ile Trp Gln Leu His Gln Lys Ile His Ser Ile
    210                 215                 220

Asp Gly Met Leu His Pro Ile His Ala Glu Arg Arg Ala Arg Ala Gly
225                 230                 235                 240

Asp Leu Gly Glu Leu His Arg Ile Val Leu Ala Ala Lys Gly Leu Gly
                245                 250                 255

Val Lys Arg Asp Glu Val Thr Lys Arg Val Tyr Gln Ser Ile Leu Ala
            260                 265                 270

Asp Ile Glu Arg Asp Asn Pro Glu Val Leu Gln Arg Tyr Ile Ala Glu
        275                 280                 285

Arg Gly Gly Leu
    290

<210> 8
<211> 879
<212> DNA
<213> Artificial sequence

<220>
<223> L5gp10 Lysin A

<400> 8
atgaccttca cagtcacccg cgagagagcg cagtgggtcc acgacatggc ccgcgctcgc 60

gacggtctcc cctacgcgta cggcggggcg ttcaccaaca acccgagggt gtcgactgac 120

tgctctggcc tggtgctgca gaccggggct tggtatggag gtcgcaccga ctgggtcgga 180

aaccgttacg gctcaaccga atcgttccgg ctcgaccaca agatcgtcta cgacctaggg 240

ttcaagcgga tgccccgagg cgggccagcg gccttgccga tcaagccggt gatgctcgtc 300

gggctccagc acggaggcgg cggggtctac tcgcacaccg cttgcacgtt gatgacgatg 360

gaccaccccg gtggcccggt caagatgtcc gaccgaggcg tcgactggga gtcccacggc 420

aaccgcaacg gcgtaggcgt cgaactttac gagggcgcac gggcatggaa cgaccctctg 480

ttccatgact tttggtacct ggacgcagtc ctcgaagacg aaggagacga tgacgaattg 540

gctgacccag ttctagggaa gatgatccgc gagatccacg cgtgcctgtt caatcagacc 600

gcgtcgacca gcgatctggc gacccctggt gaaggcgcta tctggcagct acaccagaag 660

atccactcga ttgacggcat gctccacccg atccacgctg agcggcgcgc tcgcgcaggc 720

gatctcggtg agctgcaccg aatcgtgttg gccgcgaagg gcttgggcgt gaagcgcgac 780

gaggtgacca agcgggtcta ccagagcatc ctcgccgaca tcgagcggga caaccccgaa 840

gtacttcagc gatacatcgc agaaagaggt ggcctatga 879

<210> 9
<211> 400
<212> PRT
<213> Artificial sequence

<220>
<223> TM4gp30 Lysin B

<400> 9

Met Ala Trp Val Gly Trp Gln Leu Gly Met Gln Gly Glu Gln Val Lys
1               5                   10                  15

Val Ile Gln Gln Lys Leu Ile Ala Lys Tyr Gln Trp Val Arg Asp Arg
            20                  25                  30

Tyr Pro Arg Leu Thr Ala Ser Gly Val Tyr Asp Val Asn Thr Gln Ala
        35                  40                  45

Ala Ile Val Glu Phe Gln Phe Arg Ala Gly Leu Pro Val Thr Gly Ile
    50                  55                  60

Ala Asp Tyr Ala Thr Gln Val Arg Leu Gly Ala Val Ala Pro Ala Pro
65                  70                  75                  80

Pro Pro Arg Gln Arg Ile Met Val Leu Thr Phe Ser Gly Thr Ser Ala
                85                  90                  95

Asp Met Trp Thr Gly Tyr Pro Ala Asp Val Ala Arg Ala Leu Asp Pro
            100                 105                 110

Ser Ile Phe Tyr Trp Gln Pro Val Cys Tyr Gly Pro Asn Gly Ile Pro
        115                 120                 125

Ala Ile Phe Pro Met Gly Ser Ser Ala Lys Ser Gly Glu Val Glu Gly
    130                 135                 140

Leu Arg Leu Leu Asp Glu Lys Ala Arg Asp Phe Asp Tyr Ile Val Leu
145                 150                 155                 160

Ile Gly Tyr Ser Gln Gly Ala Leu Pro Ala Ser Arg Leu Met Arg Arg
                165                 170                 175

Ile Leu Ser Gly Asp Leu Gln Arg Phe Lys Ser Lys Leu Ile Ala Gly
            180                 185                 190

Val Thr Phe Gly Asn Pro Met Arg Glu Lys Gly His Thr Phe Pro Gly
        195                 200                 205

Gly Ala Asp Pro Gly Gly His Gly Leu Asp Pro Gln Cys Leu Val Asn
    210                 215                 220

Thr Pro Asp Trp Trp His Asp Tyr Ala Ala Lys Gly Asp Ile Tyr Thr
225                 230                 235                 240

Val Gly Ser Gly Ser Asn Asp Glu Lys Ala Asn Ala Asp Met Thr Phe
                245                 250                 255

Ile Tyr Gln Leu Val Gln Gly Asp Ile Leu Gly Met Met Phe Gly Thr
            260                 265                 270

Gly Asn Pro Leu Asp Ile Leu Gly Leu Leu Gly Gly Leu Gly Gly Gly
        275                 280                 285

Leu Leu Gly Gly Leu Gly Gly Gly Leu Leu Gly Gly Gly Lys Gly Gly
    290                 295                 300

Leu Gln Leu Pro Ser Gly Leu Val Leu Pro Gly Val Gln Gly Gly Ala
305                 310                 315                 320

Leu Thr Asp His Gln Arg Gly Leu Val Glu Ala Val Leu Ala Leu Leu
                325                 330                 335

Ala Asn Pro Phe Ala Glu Val Pro Ala Ala Val Lys Ala Ile Val Ser
            340                 345                 350

Gly Val Gly Phe Ile Ala Thr Asn Pro Pro Thr Ala Pro His Ile Glu
        355                 360                 365

Tyr His Ile Arg Glu Ala Ala Pro Gly Val Thr Tyr Phe Gln His Ala
    370                 375                 380

Ile Asp Tyr Leu Arg Gln Val Gly Ala Ser Val Ala Ala Arg Ala Ala
385                 390                 395                 400

<210>  10
<211>  1203
<212>  DNA
<213>  Artificial sequence

<220>
<223>  TM4gp30 Lysin B

<400>  10
atggcctggg tcggttggca gctcggcatg cagggggagc aggtcaaggt gatacagcaa      60

aagctgatcg ccaagtacca gtgggtgcgt gaccgttacc cgcggctgac ggccagcggc     120

gtctatgacg tgaacacgca ggccgcgatc gtcgagtttc agttccgcgc agggcttccc     180

gtcaccggca tagctgacta tgcgacgcag gttcggctcg gcgcggtggc cccggcgccg     240

ccgccgcggc agcgcatcat ggtgctgacg tttagcggca cctcggccga catgtggacc     300

ggctatccgg ccgacgtcgc gcgtgcgctc gacccgtcga tcttctactg gcagccagtg     360

tgctacggcc ccaacggcat cccggcgata ttcccgatgg gttccagcgc caagagcggc     420

gaggtcgagg ggctgcggct gctcgacgag aaggcgcgcg atttcgacta catcgtgctt     480

atcggatact cgcagggcgc gctgcccgcg tcgcggctca tgcggcgcat cctgtcgggc     540

gacctgcagc ggttcaagtc caagctgatc gccggtgtca cgttcggcaa cccgatgcgc     600

gagaaggggc acacgttccc cggcggcgcc gaccccggcg ggcacggcct cgacccgcag     660

tgcctcgtga atacgcccga ctggtggcac gactacgccg ccaagggcga catttacacc     720

gtcggctcgg gcagtaacga cgagaaggcc aacgccgaca tgacgttcat ttaccagctc     780

gtgcagggcg acattctcgg catgatgttc ggcaccggca acccgctcga cattctcggc     840

ctgctcggcg gcctcggtgg cggcctgctc ggcggcctgg gcggtggcct gctcggtggc     900

ggcaagggtg gcctgcagtt gccgagcggc ctggtgctcc ccggcgtcca gggcggcgcg     960

ctcaccgacc accagcgcgg cctcgtcgag gcggtgctgg cgctgctcgc taacccgttc    1020

gccgaggttc cggcggcggt caaggcgatt gtgtccggtg tcgggttcat cgccaccaac    1080

ccgccgacgg cgccgcacat cgagtaccac attcgcgagg ctgcgcccgg cgtgacgtat 1140

ttccagcacg cgatcgacta cctgcgccag gtcggcgcgt ccgtcgccgc tcgcgcggcc 1200

tga 1203

<210> 11
<211> 321
<212> PRT
<213> Artificial sequence

<220>
<223> Bxz2gp12 Lysin B

<400> 11

Met Pro Leu Arg Val Gly Ser Asn Asp Ala Asn Thr Gly Gly Leu Val
1               5                   10                  15

Ser Arg Trp Gln Lys Thr Met Leu Ala Arg Tyr Ala Ala Tyr Ala Lys
            20                  25                  30

Ala Tyr Asp Gly Gly Pro Leu Arg Val Asp Gly Tyr Phe Gly Tyr Asp
        35                  40                  45

Asp Ala Asp Val Gln Arg Glu Tyr Glu Arg Arg Thr His Gln Val Val
    50                  55                  60

Asp Gly Glu Val Ser Asp Ala Asp Leu Arg Ala Leu Gly Leu Glu Ala
65                  70                  75                  80

Ala Lys Arg Trp Leu Phe Thr Val His Gly Thr Gly Gln Ala Asp Pro
                85                  90                  95

Leu Gly Pro Gly Leu Pro Ala Asp Thr Ala Arg Ala Val Leu Asp Lys
            100                 105                 110

Tyr Thr Trp Gln Pro Ile Gly Asn Tyr Pro Ala Arg Ala Phe Pro Met
        115                 120                 125

Trp Ser Ser Ile Met Asp Gly Ala Arg Glu Leu Arg Ser Gln Ile Ala
    130                 135                 140

Ser Lys Ser Gly Glu Val Asn Leu Ala Gly Tyr Ser Gln Gly Ala Val
145                 150                 155                 160

Val Val Gly Gln Val Leu Lys His Asp Ile Met Asp Pro Lys Gly Ser
                165                 170                 175

Leu His His Arg Leu Gly Asp Val Arg Lys Val Val Leu Trp Gly Asn
            180                 185                 190

Pro Met Arg Gln Arg Gly Ile Ala His Phe Asp Glu Trp Ile His Pro
      195                 200                 205

Val Ala Gly Pro Asp Ser Tyr Gly Ile Leu Asp Asp Arg Leu Glu Gly
    210                 215                 220

Leu Glu Lys Ala Pro Phe Glu Ile Arg Asp Tyr Ala His Ala Gly Asp
225                 230                 235                 240

Met Tyr Ala Ser Ile Thr Asp Gly Asp Lys Asp Glu Tyr Lys Ile Ala
                245                 250                 255

Ile Cys Lys Ile Val Met Thr Ala Thr Asp Phe Tyr Arg Gly Pro Asn
            260                 265                 270

Ser Val Val Ser Gln Leu Ile Glu Leu Gly Gln Arg Pro Leu Thr Glu
      275                 280                 285

Gly Ile Ala Met Ala Leu Ala Ile Ile Asp Thr Leu Arg Phe Phe Thr
    290                 295                 300

Asn Thr Ala His Gly Tyr Asn Ile Gly Pro Ala Ile Asp Phe Leu Arg
305                 310                 315                 320

Ser

<210>  12
<211>  966
<212>  DNA
<213>  Artificial sequence

<220>
<223>  Bxz2gp12 Lysin B

<400>  12
atgcccctgc gggtcgggtc gaacgacgcc aataccggcg ggctggtgag ccgttggcag      60

aagacgatgc tggcccgcta cgcggcctac gccaaggcgt acgacggcgg gccgctgcga     120

gtcgacggct atttcggcta cgacgacgct gacgttcagc gcgagtacga acgccgcacc     180

caccaggtgg tcgacggtga ggtcagtgac gccgacctcc gggctctggg cctggaggcg     240

gcgaagcgct ggctgttcac ggtccacggc accggacagg ccgacccgct gggtccggga     300

ctccccgccg acacggcgcg ggcggtgctc gacaagtaca cctggcagcc catcggcaac     360

taccccgctc gggcgttccc gatgtggtcc tcgatcatgg acggtgccag ggagcttcgc     420

tcccagatcg cgtcaaagtc cggtgaggtc aacctggcgg gctactcgca aggcgcggtg     480

gtcgtcggcc aggtgctcaa gcacgacatc atggacccga agggcagcct gcaccacagg     540

ctcggcgatg tccgcaaggt agtgctctgg ggaaatccca tgcgccagag gggaatcgct     600

cacttcgatg agtggattca cccggtggca ggcccagact cgtacggcat cctcgatgac 660

cggctcgaag ggctggagaa ggcaccgttc gagatccggg actacgcgca cgctggtgac 720

atgtacgcct ccatcacgga cggcgacaag gacgagtaca agatcgcgat ctgcaagatc 780

gtcatgacgg cgacggactt ctaccgaggc ccgaactccg ttgtgtccca actgatcgag 840

cttggacagc gtccgctcac cgagggcatc gcaatggccc tggcgatcat cgacacgctg 900

cggttcttca cgaacaccgc gcacggctac aacatcggac cagctatcga cttcctgcgt 960

agctga 966

<210> 13
<211> 254
<212> PRT
<213> Artificial sequence

<220>
<223> D29gp12 Lysin B

<400> 13

Met Ser Lys Pro Trp Leu Phe Thr Val His Gly Thr Gly Gln Pro Asp
1               5                   10                  15

Pro Leu Gly Pro Gly Leu Pro Ala Asp Thr Ala Arg Asp Val Leu Asp
            20                  25                  30

Ile Tyr Arg Trp Gln Pro Ile Gly Asn Tyr Pro Ala Ala Ala Phe Pro
        35                  40                  45

Met Trp Pro Ser Val Glu Lys Gly Val Ala Glu Leu Ile Leu Gln Ile
    50                  55                  60

Glu Leu Lys Leu Asp Ala Asp Pro Tyr Ala Asp Phe Ala Met Ala Gly
65                  70                  75                  80

Tyr Ser Gln Gly Ala Ile Val Val Gly Gln Val Leu Lys His His Ile
                85                  90                  95

Leu Pro Pro Thr Gly Arg Leu His Arg Phe Leu His Arg Leu Lys Lys
            100                 105                 110

Val Ile Phe Trp Gly Asn Pro Met Arg Gln Lys Gly Phe Ala His Ser
        115                 120                 125

Asp Glu Trp Ile His Pro Val Ala Ala Pro Asp Thr Leu Gly Ile Leu
    130                 135                 140

Glu Asp Arg Leu Glu Asn Leu Glu Gln Tyr Gly Phe Glu Val Arg Asp
145                 150                 155                 160

Tyr Ala His Asp Gly Asp Met Tyr Ala Ser Ile Lys Glu Asp Asp Leu
                165                 170                 175

His Glu Tyr Glu Val Ala Ile Gly Arg Ile Val Met Lys Ala Ser Gly
            180                 185                 190

Phe Ile Gly Gly Arg Asp Ser Val Val Ala Gln Leu Ile Glu Leu Gly
        195                 200                 205

Gln Arg Pro Ile Thr Glu Gly Ile Ala Leu Ala Gly Ala Ile Ile Asp
    210                 215                 220

Ala Leu Thr Phe Phe Ala Arg Ser Arg Met Gly Asp Lys Trp Pro His
225                 230                 235                 240

Leu Tyr Asn Arg Tyr Pro Ala Val Glu Phe Leu Arg Gln Ile
                245                 250

<210>  14
<211>  765
<212>  DNA
<213>  Artificial sequence

<220>
<223>  D29gp12 Lysin B

<400>  14
atgagcaagc cctggctgtt caccgttcac ggcacgggcc agcccgatcc cctcgggcct      60

ggcctgcctg ccgatacggc acgcgacgta cttgacatct accggtggca gcccatcggc     120

aactaccccg ctgcggcctt cccgatgtgg ccgtcggtcg agaagggtgt cgccgagctg     180

atcctgcaga tcgagctgaa gctggacgcg gacccctacg cggacttcgc gatggcgggt     240

tactcgcagg gagccatcgt ggttggccag gtgctcaagc accacatcct gcctccgacg     300

ggcaggctcc acaggttcct gcaccggctc aagaaggtca tcttctgggg taatcccatg     360

cggcagaagg gctttgccca ctctgacgag tggatccacc cggtcgctgc ccctgacacc     420

ctcggaatcc tcgaggaccg gctcgaaaac ctggagcagt acggcttcga ggtccgcgac     480

tacgcccacg acggtgacat gtacgcctcc atcaaagagg acgacctgca cgaatacgag     540

gtcgccatcg gccggatcgt gatgaaggcc agcggcttca tcggtggccg ggactccgtg     600

gtagcccagc tcatcgagct tggccagcgt ccgatcaccg agggaattgc gttggcggga     660

gccatcatcg acgccctcac gttcttcgcc cgctctcgta tgggcgacaa gtggccgcac     720

ctctacaacc gctacccggc ggtcgagttc ctacgacaga tctga                     765

<210>  15
<211>  254
<212>  PRT
<213>  Artificial sequence

<220>
<223>  L5gp12 Lysin B

<400>  15

Met Ser Lys Pro Trp Leu Phe Thr Val His Gly Thr Gly Gln Pro Asp
1               5                   10                  15

Pro Leu Gly Pro Gly Leu Pro Ala Asp Thr Ala Arg Asp Val Leu Asp
            20                  25                  30

Ile Tyr Arg Trp Gln Pro Ile Gly Asn Tyr Pro Ala Ala Ala Phe Pro
        35                  40                  45

Met Trp Pro Ser Val Glu Lys Gly Val Ala Glu Leu Ile Leu Gln Ile
    50                  55                  60

Glu Leu Lys Leu Asp Ala Asp Pro Tyr Ala Asp Phe Ala Leu Ala Gly
65                  70                  75                  80

Tyr Ser Gln Gly Ala Ile Val Val Gly Gln Val Leu Lys His His Ile
                85                  90                  95

Ile Asn Pro Arg Gly Arg Leu His Arg Phe Leu His Arg Leu Arg Lys
            100                 105                 110

Val Ile Phe Trp Gly Asn Pro Met Arg Gln Lys Gly Phe Ala His Thr
        115                 120                 125

Asp Glu Trp Ile His Gln Val Ala Ala Ser Asp Thr Met Gly Ile Leu
    130                 135                 140

Glu Asp Arg Leu Glu Asn Leu Glu Gln Tyr Gly Phe Glu Val Arg Asp
145                 150                 155                 160

Tyr Ala His Asp Gly Asp Met Tyr Ala Ser Ile Lys Glu Asp Asp Met
                165                 170                 175

His Glu Tyr Glu Val Ala Ile Gly Arg Ile Val Met Ser Ala Arg Arg
            180                 185                 190

Phe Ile Gly Gly Lys Asp Ser Val Ile Ala Gln Leu Ile Glu Leu Gly
        195                 200                 205

Gln Arg Pro Ile Trp Glu Gly Ile Ala Met Ala Arg Ala Ile Ile Asp
    210                 215                 220

Ala Leu Thr Phe Phe Ala Lys Ser Thr Gln Gly Pro Ser Trp Pro His
225                 230                 235                 240

Leu Tyr Asn Arg Phe Pro Ala Val Glu Phe Leu Arg Arg Ile
                245                 250

<210>  16
<211>  765
<212>  DNA
<213>  Artificial sequence

<220>
<223>  L5gp12 Lysin B

<400>  16
atgagcaagc cctggctgtt caccgtccac ggcacaggcc agcccgaccc gctcgggcct      60

ggtctgcctg ccgataccgc acgggacgta cttgacatct accggtggca gcccatcggc     120

aactacccgg cagcggcgtt cccgatgtgg ccgtcggtcg aaaagggtgt cgctgagctg     180

atcctgcaga tcgagctgaa gctggacgca gatccgtacg cggacttcgc gctggccggc     240

tactcgcagg gagccatcgt ggtgggccag gtgctcaagc accacatcat caacccgaga     300

ggtcgactgc accggttcct gcaccggctc aggaaggtca tcttctgggg taatccgatg     360

cggcagaagg gctttgccca caccgacgag tggattcacc aggtcgctgc ctcggacacg     420

atgggcatcc tcgaggaccg actggagaac ctcgagcagt acggctttga ggtccgcgac     480

tacgcgcacg acggcgacat gtacgcctcc atcaaggagg acgacatgca cgagtacgag     540

gtggccattg gccgaatcgt gatgagcgct aggcgattca tcggaggtaa ggactccgtc     600

atcgcccagc tcatcgagct tggacagcgt ccgatctggg agggaatcgc gatggccaga     660

gccatcatcg acgccctcac gttcttcgcc aagtcgaccc aaggcccgag ctggccgcat     720

ttgtacaacc gcttcccggc ggtcgagttc ctacgacgaa tctga                     765

<210>  17
<211>  37
<212>  PRT
<213>  Artificial sequence

<220>
<223>  LL-37

<400>  17

Leu Leu Gly Asp Phe Phe Arg Lys Ser Lys Glu Lys Ile Gly Lys Glu
1               5                   10                  15

Phe Lys Arg Ile Val Gln Arg Ile Lys Asp Phe Leu Arg Asn Leu Val
            20                  25                  30

Pro Arg Thr Glu Ser
        35

<210>  18
<211>  111
<212>  DNA

<213>  Artificial sequence

<220>
<223>  LL-37

<400>  18
ttattgggtg atttctttcg gaagagcaaa gaaaagatag gaaaggagtt taaacgaatt      60

gttcaacgta tcaaagactt cctaaggaat cttgtaccaa gaacagaaag t              111


<210>  19
<211>  30
<212>  PRT
<213>  Artificial sequence

<220>
<223>  alpha-defensin

<400>  19

Asp Cys Tyr Cys Arg Ile Pro Ala Cys Ile Ala Gly Glu Arg Arg Tyr
1               5                   10                  15


Gly Thr Cys Ile Tyr Gln Gly Arg Leu Trp Ala Phe Cys Cys
            20                  25                  30


<210>  20
<211>  90
<212>  DNA
<213>  Artificial sequence

<220>
<223>  alpha-defensin

<400>  20
gattgttatt gtagaatacc agcatgcatt gcgggagaac gtaggtacgg aacatgcatc      60

tatcaaggtc gattatgggc tttttgttgc                                      90


<210>  21
<211>  38
<212>  PRT
<213>  Artificial sequence

<220>
<223>  beta-defensin

<400>  21

Asn Pro Val Ser Cys Val Arg Asn Lys Gly Ile Cys Val Pro Ile Arg
1               5                   10                  15


Cys Pro Gly Ser Met Lys Gln Ile Gly Thr Cys Val Gly Arg Ala Val
            20                  25                  30


Lys Cys Cys Arg Lys Lys
        35

<210>  22
<211>  114
<212>  DNA
<213>  Artificial sequence

<220>
<223>  beta-defensin

<400>  22
aatccagtaa gctgtgttcg taataagggt atttgcgttc caatacgatg cccaggaagt      60

atgaaacaaa tcggtacatg cgtaggaaga gcagtaaagt gttgtaggaa aaaa          114


<210>  23
<211>  25
<212>  PRT
<213>  Artificial sequence

<220>
<223>  Hepcidin

<400>  23

Asp Thr His Phe Pro Ile Cys Ile Phe Cys Cys Gly Cys Cys His Arg
1               5                   10                  15


Ser Lys Cys Gly Met Cys Cys Lys Thr
            20                  25


<210>  24
<211>  75
<212>  DNA
<213>  Artificial sequence

<220>
<223>  Hepcidin

<400>  24
gatacacact ttccaatatg cattttctgt tgcggttgct gtcatagaag taaatgtgga      60

atgtgctgta agaca                                                      75


<210>  25
<211>  27
<212>  PRT
<213>  Artificial sequence

<220>
<223>  NK-2

<400>  25

Lys Ile Leu Arg Gly Val Cys Lys Lys Ile Met Arg Thr Phe Leu Arg
1               5                   10                  15


Arg Ile Ser Lys Asp Ile Leu Thr Gly Lys Lys
            20                  25


<210>  26

<211>  81
<212>  DNA
<213>  Artificial sequence

<220>
<223>  NK-2

<400>  26
aaaatcttac gaggtgtatg taaaaagatt atgagaacat ttttgcgtag gataagtaaa      60

gatatactaa caggaaagaa g                                                81


<210>  27
<211>  24
<212>  PRT
<213>  Artificial sequence

<220>
<223>  Ci-MAM-A24

<400>  27

Trp Arg Ser Leu Gly Arg Thr Leu Leu Arg Leu Ser His Ala Leu Lys
1               5                   10                  15


Pro Leu Ala Arg Arg Ser Gly Trp
            20


<210>  28
<211>  72
<212>  DNA
<213>  Artificial sequence

<220>
<223>  Ci-MAM-A24

<400>  28
tggcgaagtt taggaagaac actacttcgg ttgagccatg cattgaaacc attagctagg      60

cgtagtggtt gg                                                          72


<210>  29
<211>  14
<212>  PRT
<213>  Artificial sequence

<220>
<223>  TAT48-60

<400>  29

Gly Arg Lys Lys Arg Arg Gln Arg Arg Arg Pro Pro Gln Cys
1               5                   10


<210>  30
<211>  11
<212>  PRT
<213>  Artificial sequence

<220>

<223>  TAT47-57

<400>  30

Tyr Gly Arg Lys Lys Arg Arg Gln Arg Arg Arg
1               5                   10



<210>  31
<211>  9
<212>  PRT
<213>  Artificial sequence

<220>
<223>  TAT47-55

<400>  31

Tyr Gly Arg Lys Lys Arg Arg Gln Arg
1               5



<210>  32
<211>  11
<212>  PRT
<213>  Artificial sequence

<220>
<223>  PTD3

<400>  32

Tyr Ala Arg Lys Ala Arg Arg Gln Ala Arg Arg
1               5                   10



<210>  33
<211>  9
<212>  PRT
<213>  Artificial sequence

<220>
<223>  PolyArginine

<400>  33

Arg Arg Arg Arg Arg Arg Arg Arg Arg
1               5



<210>  34
<211>  20
<212>  PRT
<213>  Artificial sequence

<220>
<223>  CADY

<400>  34

Gly Leu Trp Arg Ala Leu Trp Arg Leu Leu Arg Ser Leu Trp Arg Leu
1               5                   10                  15


Leu Trp Arg Ala

            20

<210>  35
<211>  21
<212>  PRT
<213>  Artificial sequence

<220>
<223>  PepFect6

<400>  35

Ala Gly Tyr Leu Leu Gly Lys Ile Asn Leu Lys Ala Leu Ala Ala Leu
1               5                   10                  15

Ala Lys Lys Ile Leu
            20

<210>  36
<211>  14
<212>  PRT
<213>  Artificial sequence

<220>
<223>  RXR

<220>
<221>  misc_feature
<222>  (2)..(2)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (13)..(13)
<223>  Xaa can be any naturally occurring amino acid

<400>  36

Arg Xaa Arg Arg Xaa Arg Arg Xaa Arg Arg Xaa Arg Xaa Asx
1               5                   10

<210>  37
<211>  16
<212>  PRT
<213>  Artificial sequence

<220>
<223>  Antennapedia

<400>  37

Arg Gln Ile Lys Ile Trp Phe Gln Asn Arg Arg Met Lys Trp Lys Lys
1               5                   10                  15


<210>  38
<211>  30
<212>  PRT
<213>  Artificial sequence

<220>
<223>  Kala Syn

<400>  38

Trp Glu Ala Lys Leu Ala Lys Ala Leu Ala Lys Ala Leu Ala Lys His
1               5                   10                  15


Leu Ala Lys Ala Leu Ala Lys Ala Leu Lys Ala Cys Glu Ala
            20                  25                  30


<210>  39
<211>  22
<212>  PRT
<213>  Artificial sequence

<220>
<223>  M918

<400>  39

Met Val Thr Val Leu Phe Arg Arg Leu Arg Ile Arg Arg Ala Ser Gly
1               5                   10                  15


Pro Pro Arg Val Arg Val
            20


<210>  40
<211>  18
<212>  PRT
<213>  Artificial sequence

<220>
<223>  MAP

<400>  40

Lys Leu Ala Leu Lys Leu Ala Leu Lys Ala Leu Lys Ala Ala Leu Lys
1               5                   10                  15


Leu Ala



<210>  41

<211>  16
<212>  PRT
<213>  Artificial sequence

<220>
<223>  Penetratin

<400>  41

Arg Gln Ile Lys Ile Trp Phe Gln Asn Arg Arg Met Lys Trp Lys Lys
1               5                   10                  15


<210>  42
<211>  12
<212>  PRT
<213>  Artificial sequence

<220>
<223>  PTD5-Syn

<400>  42

Arg Arg Gln Arg Arg Thr Ser Lys Leu Met Lys Arg
1               5                   10


<210>  43
<211>  18
<212>  PRT
<213>  Artificial sequence

<220>
<223>  Pvec

<400>  43

Leu Leu Ile Ile Leu Arg Arg Arg Ile Arg Lys Gln Ala His Ala His
1               5                   10                  15


Ser Lys




<210>  44
<211>  8
<212>  PRT
<213>  Artificial sequence

<220>
<223>  Poly Arg 8

<400>  44

Arg Arg Arg Arg Arg Arg Arg Arg
1               5


<210>  45
<211>  13
<212>  PRT
<213>  Artificial sequence

<220>

<223>  TAT48-60

<400>  45

Gly Arg Lys Lys Arg Arg Gln Arg Arg Arg Pro Pro Gln
1               5                   10


<210>  46
<211>  27
<212>  PRT
<213>  Artificial sequence

<220>
<223>  Transportan

<400>  46

Gly Trp Thr Leu Asn Ser Ala Gly Tyr Leu Leu Gly Lys Ile Asn Leu
1               5                   10                  15


Lys Ala Leu Ala Ala Leu Ala Lys Lys Ile Leu
            20                  25


<210>  47
<211>  21
<212>  PRT
<213>  Artificial sequence

<220>
<223>  Transportan10

<400>  47

Ala Gly Tyr Leu Leu Gly Lys Ile Asn Leu Lys Ala Leu Ala Ala Leu
1               5                   10                  15


Ala Lys Lys Ile Leu
            20


<210>  48
<211>  9
<212>  PRT
<213>  Artificial sequence

<220>
<223>  TAT-9

<400>  48

Arg Lys Lys Arg Arg Gln Arg Arg Arg
1               5


<210>  49
<211>  603
<212>  PRT
<213>  Artificial sequence

<220>
<223>  TM4gp29/LL-37/TAT47-57

<400> 49

Met Ser Phe Thr Arg Phe Leu Gln Asp Asp Pro Leu Leu Thr Arg Glu
1               5                   10                  15

Gln Val Met Ala Glu Leu Ile Arg Val Ala Asp Glu Leu Asn Met Pro
            20                  25                  30

Asp Lys Arg Gly Ala Cys Val Ile Ala Gly Met Thr Ile Ser Gln Glu
        35                  40                  45

Val Gly Val Lys Asp Asn Asp Pro Pro Phe Glu Arg Arg Phe Trp Cys
    50                  55                  60

Pro Ala Asn Arg Ala Asp Pro Glu Ser Phe Asn Tyr Pro His Asp Ser
65                  70                  75                  80

Glu Ser Asn Asp Gly Arg Ser Val Gly Tyr Phe Gln Gln Gln Lys Gly
                85                  90                  95

Pro Asn Gly Glu Leu Trp Trp Gly Thr Thr Ala Ser Glu Met Asn Leu
            100                 105                 110

His Ser Ala Ala Thr Gln Phe Met Thr Arg Leu Lys Ala Ala Gly Tyr
        115                 120                 125

Asn Ala Ser Asn Ala Gln Ala Ala Asn Asp Ser Ala Gln Ala Ile Gln
    130                 135                 140

Arg Ser Gly Val Pro Gln Ala Tyr Lys Gln Trp Trp Asp Asp Ile Asn
145                 150                 155                 160

Arg Leu Tyr Asp Lys Val Lys Gly Ser Gly Gly Gly Pro Ala Pro Ala
                165                 170                 175

Pro Lys Pro Pro Gln Ser Gly Pro Trp Thr Gly Asp Pro Val Trp Leu
            180                 185                 190

Ala Asp Val Leu Arg Ala Glu Gly Leu Asn Val Val Glu Leu Pro Gly
        195                 200                 205

Trp Leu Asp Arg Gly His Gly Asp Met Gly Arg Leu Trp Gly Val Val
    210                 215                 220

Cys His His Thr Gly Ser Asp Asn Thr Pro Ser Ser Glu Ile Ala Phe
225                 230                 235                 240

His Pro Ser Leu Gly Leu Cys Ser Gln Ile His Leu Ala Arg Asn Gly
                245                 250                 255

Thr Val Thr Leu Cys Gly Val Gly Ile Ala Trp His Ala Gly Val Gly
            260                 265                 270

Ser Tyr Pro Gly Leu Pro Glu Asp Asn Ala Asn Ala Val Thr Ile Gly
        275                 280                 285

Ile Glu Ala Gln Asn Ser Gly Thr Tyr Asp Gly Ala Pro His Arg Thr
    290                 295                 300

Asn Trp Pro Asp Ala Gln Tyr Asp Ala Tyr Val Lys Cys Cys Ala Ala
305                 310                 315                 320

Ile Cys Arg Arg Leu Gly Val Arg Ala Asp His Val Ile Ser His Lys
                325                 330                 335

Glu Trp Ala Gly Arg Lys Gln Gly Lys Trp Asp Pro Gly Ala Ile Asp
            340                 345                 350

Met Asn Ile Phe Arg Ala Asp Val Gln Arg Arg Ile Asp Ala His Gln
        355                 360                 365

Pro Asn Gly Glu Asp Asp Phe Met Ala Ala Leu Ser Ala Asp Glu Gln
    370                 375                 380

Arg Glu Val Leu Asn Leu Leu Arg Val Leu Ala Asp Arg Arg Phe Val
385                 390                 395                 400

Ser Arg Ser Pro Phe Arg His Leu Gly Glu Gly Pro Ser Glu Thr Val
                405                 410                 415

Ala Gly Phe Gly Leu Asn Thr Asp Gly Leu Asn His Ala Gln Tyr Thr
            420                 425                 430

Ile Glu Leu Ala Arg Leu Gly Asp Pro Thr His Leu Ala Leu Leu Arg
        435                 440                 445

Glu Val Ala Ser Ala Glu Gly Asp Ser Arg Tyr Pro Asp Arg Gln Tyr
    450                 455                 460

Asp Ala Lys Leu Ala Lys Arg Val Leu Ala Glu Ile Glu Gly Ala Ala
465                 470                 475                 480

Thr Ala Pro Ala Lys Pro Ser Thr Pro Ser Ala Pro Thr Glu Pro Ala
                485                 490                 495

Pro Glu Ala Pro Thr Pro Pro Val Lys Ala Ala Cys Ala Leu Ser Ala
            500                 505                 510

Ala Gly Cys Val Val Ala Gly Ser Thr Ser Gly Gly Gly Cys Ala Leu
        515                 520                 525

Ser Thr Asp Gly Thr Gly Lys Cys Val Val Thr Ala Ala Thr Asp Gly
    530                 535                 540

Gly Ala Ala Gly Ser Gly Ser Leu Leu Gly Asp Phe Phe Arg Lys Ser
545                 550                 555                 560

Lys Glu Lys Ile Gly Lys Glu Phe Lys Arg Ile Val Gln Arg Ile Lys
                565                 570                 575

Asp Phe Leu Arg Asn Leu Val Pro Arg Thr Glu Ser Gly Ala Gly Ala
            580                 585                 590

Tyr Gly Arg Lys Lys Arg Arg Gln Arg Arg Arg
        595                 600

<210>  50
<211>  1809
<212>  DNA
<213>  Artificial sequence

<220>
<223>  TM4gp29/LL-37/TAT47-57

<400>  50
atgagtttca cacgtttttt acaagatgac ccactattaa cacgtgagca agttatggcg      60

gagttaatac gggttgctga cgaacttaat atgccagaca agcgtggagc gtgcgtaata     120

gctggaatga caattagcca agaggttggt gttaaagaca atgatccacc atttgagagg     180

cgtttctggt gcccagctaa tcgtgccgat ccagaaagtt tcaattatcc acacgatagc     240

gaaagcaatg atggtaggag cgttggatat tttcaacaac aaaagggtcc aaatggagaa     300

ttgtggtggg gtacaacagc tagcgaaatg aatttacaca gtgcagcgac acaattcatg     360

acaaggctaa aggcggctgg atacaatgcc agtaatgcgc aagccgctaa tgatagcgca     420

caagccatac aacgaagcgg tgttccacaa gcatataagc aatggtggga tgacataaat     480

cggctttatg acaaagtaaa gggaagcggt ggaggtccag cgccagcccc aaaaccacca     540

caaagcggtc catggacagg agatccagta tggttagccg atgtactaag ggcagaagga     600

ttgaatgttg tagaattacc aggttggcta gaccgaggac atggtgatat gggacggttg     660

tggggtgttg tatgtcatca cacaggaagc gataatacac caagcagtga aatcgccttt     720

catccaagcc ttggattatg cagccaaata catttggcca gaaatggaac agttacattg     780

tgtggagttg gtattgcatg gcacgcaggt gttggtagtt atccaggtct accagaggac     840

aatgcaaatg ctgttacaat tggtattgaa gctcaaaata gcggaacata tgacggtgcg     900

ccacatcgga caaattggcc agacgcgcaa tacgatgctt acgttaaatg ctgtgcagct     960

atctgtcggc gattgggagt aagggcggac catgtaatca gtcacaaaga atgggcaggt 1020

aggaaacaag gtaaatggga cccaggtgca atagatatga atatatttag agccgatgta 1080

caacgtcgga tcgatgccca tcaaccaaat ggagaagacg attttatggc ggctttgagc 1140

gcagacgaac aacgagaggt attgaatctt ttgcgtgtac tagccgatcg gagattcgta 1200

agtaggagtc cattcaggca ccttggagaa ggtccaagcg aaacagtagc aggatttggt 1260

cttaatacag acggattgaa tcatgcccaa tatacaattg aacttgcccg attaggtgac 1320

ccaacacact tggcactttt aagagaggta gcgagtgcgg aaggtgacag cagataccca 1380

gatcggcaat acgacgccaa gcttgcgaaa agggttctag cagaaattga aggagcagcc 1440

acagcgccag ctaagccaag tacaccaagt gcgccaacag agccagcccc agaagctcca 1500

acaccaccag ttaaggctgc gtgcgcctta agtgcggctg gatgtgttgt agcgggtagt 1560

acaagtggtg gaggttgcgc actaagtaca gatggtacag gaaagtgtgt agttacagcc 1620

gctacagacg gtggagcagc tggaagtgga agtcttttag gagacttctt tcgtaagagc 1680

aaagagaaaa tcggaaaaga atttaaacgt atcgtacaac gaattaagga tttcctaaga 1740

aatctagtac cacgaacaga gagtggtgca ggagcttacg gtagaaagaa acggcgacaa 1800

agacgaaga 1809

<210> 51
<211> 595
<212> PRT
<213> Artificial sequence

<220>
<223> TM4gp29/LL-37/TAT47-57

<400> 51

Met Ser Phe Thr Arg Phe Leu Gln Asp Asp Pro Leu Leu Thr Arg Glu
1               5                   10                  15

Gln Val Met Ala Glu Leu Ile Arg Val Ala Asp Glu Leu Asn Met Pro
            20                  25                  30

Asp Lys Arg Gly Ala Cys Val Ile Ala Gly Met Thr Ile Ser Gln Glu
        35                  40                  45

Val Gly Val Lys Asp Asn Asp Pro Pro Phe Glu Arg Arg Phe Trp Cys
    50                  55                  60

Pro Ala Asn Arg Ala Asp Pro Glu Ser Phe Asn Tyr Pro His Asp Ser
65                  70                  75                  80

Glu Ser Asn Asp Gly Arg Ser Val Gly Tyr Phe Gln Gln Gln Lys Gly
                85                  90                  95

Pro Asn Gly Glu Leu Trp Trp Gly Thr Thr Ala Ser Glu Met Asn Leu
            100                 105                 110

His Ser Ala Ala Thr Gln Phe Met Thr Arg Leu Lys Ala Ala Gly Tyr
        115                 120                 125

Asn Ala Ser Asn Ala Gln Ala Ala Asn Asp Ser Ala Gln Ala Ile Gln
    130                 135                 140

Arg Ser Gly Val Pro Gln Ala Tyr Lys Gln Trp Trp Asp Asp Ile Asn
145                 150                 155                 160

Arg Leu Tyr Asp Lys Val Lys Gly Ser Gly Gly Gly Pro Ala Pro Ala
                165                 170                 175

Pro Lys Pro Pro Gln Ser Gly Pro Trp Thr Gly Asp Pro Val Trp Leu
            180                 185                 190

Ala Asp Val Leu Arg Ala Glu Gly Leu Asn Val Val Glu Leu Pro Gly
        195                 200                 205

Trp Leu Asp Arg Gly His Gly Asp Met Gly Arg Leu Trp Gly Val Val
    210                 215                 220

Cys His His Thr Gly Ser Asp Asn Thr Pro Ser Ser Glu Ile Ala Phe
225                 230                 235                 240

His Pro Ser Leu Gly Leu Cys Ser Gln Ile His Leu Ala Arg Asn Gly
                245                 250                 255

Thr Val Thr Leu Cys Gly Val Gly Ile Ala Trp His Ala Gly Val Gly
            260                 265                 270

Ser Tyr Pro Gly Leu Pro Glu Asp Asn Ala Asn Ala Val Thr Ile Gly
        275                 280                 285

Ile Glu Ala Gln Asn Ser Gly Thr Tyr Asp Gly Ala Pro His Arg Thr
    290                 295                 300

Asn Trp Pro Asp Ala Gln Tyr Asp Ala Tyr Val Lys Cys Cys Ala Ala
305                 310                 315                 320

Ile Cys Arg Arg Leu Gly Val Arg Ala Asp His Val Ile Ser His Lys
                325                 330                 335

Glu Trp Ala Gly Arg Lys Gln Gly Lys Trp Asp Pro Gly Ala Ile Asp
            340                 345                 350

Met Asn Ile Phe Arg Ala Asp Val Gln Arg Arg Ile Asp Ala His Gln
        355                 360                 365

Pro Asn Gly Glu Asp Asp Phe Met Ala Ala Leu Ser Ala Asp Glu Gln
    370                 375                 380

Arg Glu Val Leu Asn Leu Leu Arg Val Leu Ala Asp Arg Arg Phe Val
385                 390                 395                 400

Ser Arg Ser Pro Phe Arg His Leu Gly Glu Gly Pro Ser Glu Thr Val
                405                 410                 415

Ala Gly Phe Gly Leu Asn Thr Asp Gly Leu Asn His Ala Gln Tyr Thr
            420                 425                 430

Ile Glu Leu Ala Arg Leu Gly Asp Pro Thr His Leu Ala Leu Leu Arg
        435                 440                 445

Glu Val Ala Ser Ala Glu Gly Asp Ser Arg Tyr Pro Asp Arg Gln Tyr
    450                 455                 460

Asp Ala Lys Leu Ala Lys Arg Val Leu Ala Glu Ile Glu Gly Ala Ala
465                 470                 475                 480

Thr Ala Pro Ala Lys Pro Ser Thr Pro Ser Ala Pro Thr Glu Pro Ala
                485                 490                 495

Pro Glu Ala Pro Thr Pro Pro Val Lys Ala Ala Cys Ala Leu Ser Ala
            500                 505                 510

Ala Gly Cys Val Val Ala Gly Ser Thr Ser Gly Gly Gly Cys Ala Leu
        515                 520                 525

Ser Thr Asp Gly Thr Gly Lys Cys Val Val Thr Ala Ala Thr Asp Gly
    530                 535                 540

Gly Ala Ala Leu Leu Gly Asp Phe Phe Arg Lys Ser Lys Glu Lys Ile
545                 550                 555                 560

Gly Lys Glu Phe Lys Arg Ile Val Gln Arg Ile Lys Asp Phe Leu Arg
                565                 570                 575

Asn Leu Val Pro Arg Thr Glu Ser Tyr Gly Arg Lys Lys Arg Arg Gln
            580                 585                 590

Arg Arg Arg
        595

<210>  52

<211>  1785
<212>  DNA
<213>  Artificial sequence

<220>
<223>  TM4gp29/LL-37/TAT47-57

<400>  52
atgagtttta caaggttcct acaagatgac ccactattga cacgagaaca agtaatggct      60

gagctaatac gagtagcaga tgaattaaat atgccagata agaggggagc ttgtgttatc     120

gccggtatga caatcagtca agaagttgga gttaaggaca atgatccacc attcgagcgt     180

aggttctggt gtccagcgaa tcgtgctgat ccagaaagtt ttaattaccc acacgacagc     240

gaaagcaatg acggacgtag tgtaggttat ttccaacaac aaaagggacc aaatggagag     300

ctttggtggg gtacaacagc tagtgagatg aatctacata gtgcagcgac acaatttatg     360

acaagactta aagctgccgg ttataatgcc agtaatgcgc aagcggctaa tgacagtgca     420

caagctatac aacgtagtgg tgtaccacaa gcctataagc aatggtggga tgacataaat     480

aggttatacg ataaggtaaa gggtagtggt ggaggtccag caccagcacc aaaaccacca     540

caaagcggtc catggacagg agatccagtt tggttggcgg acgtattacg agcagaaggt     600

ttgaatgtag ttgaacttcc aggatggctt gacagaggac acggtgacat gggacgtctt     660

tggggtgtag tttgtcacca tacaggtagt gacaatacac caagtagcga aattgccttt     720

cacccaagtt tgggactttg cagccaaatt catcttgcca ggaatggtac agttacacta     780

tgcggagtag gaatagcttg gcacgccggt gttggtagct atccaggttt accagaagat     840

aatgctaatg cagtaacaat tggaattgaa gcacaaaata gcggaacata cgacggtgca     900

ccacatagaa caaattggcc agacgctcaa tacgatgcgt atgttaaatg ctgtgctgcc     960

atatgccggc gattaggtgt acgggctgac catgttatta gccacaagga atgggccgga    1020

agaaaacaag gaaaatggga cccaggagct atcgatatga atatctttcg ggcggatgta    1080

caacggcgaa ttgatgctca tcaaccaaat ggagaagatg actttatggc ggccttaagt    1140

gccgacgaac aacgtgaggt tttaaatctt ttacgggtat tggcggaccg gcgattcgtt    1200

agtaggagcc cattccgaca tttgggtgag ggaccaagcg agacagtagc gggattcggt    1260

ttgaatacag atggtttgaa tcatgcacaa tatacaatag aattggcaag attgggtgac    1320

ccaacacacc tagcgctttt aagggaagta gcaagtgcag aaggtgatag ccgttatcca    1380

gatcgtcaat acgatgccaa gctagcgaaa cgagttttag cagaaattga aggagccgcg    1440

acagcgccag ccaaaccaag cacaccaagt gctccaacag aaccagcacc agaggcgcca    1500

acaccaccag ttaaagctgc ctgtgcccta agtgcagcgg gatgcgtagt tgccggaagc    1560

acaagcggag gtggatgcgc tttgagcaca gacggaacag gtaaatgtgt agttacagct    1620

gcgacagatg gtggagcagc attactaggt gactttttca ggaaaagcaa agaaaagatc    1680

ggtaaagagt ttaaaagaat agtacaacgg atcaaggatt ttcttagaaa tctagttcca    1740

cggacagaga gctacggaag aaagaagcgg agacaaaggc gacgt                    1785

<210>  53
<211>  555
<212>  PRT
<213>  Artificial sequence

<220>
<223>  Bzx2gp11/alpha-defensin/PTD3

<400>  53

Met Thr Glu Lys Val Leu Pro Tyr Asp Arg Ser Ile Val Thr Gln Glu
1               5                   10                  15

Thr Gly Trp Trp Cys Gly Pro Ala Ala Thr Gln Val Val Leu Asn Ser
            20                  25                  30

Arg Gly Ile Ile Val Pro Glu Ala Thr Leu Ala Ala Glu Ile Glu Ala
        35                  40                  45

Ile Glu Asn Pro Gly Arg Gly Asp Asp Arg Asp Gly Thr Asp Tyr Val
    50                  55                  60

Gly Leu Ile Glu Gln Val Leu Asp Arg Arg Val Pro Gln Ala Arg Tyr
65                  70                  75                  80

Thr Ser Val Tyr Leu Thr Asn Asp Pro Pro Thr Gln Ala Gln Lys Asp
                85                  90                  95

Arg Leu Trp Glu His Ile Val Arg Ser Ile Asn Ala Gly Tyr Gly Val
            100                 105                 110

Val Met Asn Trp Val Ala Pro Pro Ser Asn Lys Pro Arg Gly Val Lys
        115                 120                 125

Gly Ser Val Ser Pro Arg Tyr Ser Gly Gly Thr Thr Tyr His Tyr Val
    130                 135                 140

Ala Cys Met Gly Tyr Asp Asp Thr Pro Gly Ala Arg Ala Val Trp Ile
145                 150                 155                 160

Ala Asp Ser Gly Phe Gln Pro Gln Gly Tyr Trp Ile Ser Phe Asp Gln
                165                 170                 175

Cys Ala Thr Leu Ile Pro Pro Lys Gly Tyr Ala Tyr Ala Asp Ala Ala
            180                 185                 190

Pro Ala Ala Pro Ala Pro Ala Pro Thr Pro Val Val Asp Ala Ala Pro
        195                 200                 205

Ile Leu Ala Arg Ala Ala Gly Ile Ser Glu Ala Lys Ala Arg Glu Ile
    210                 215                 220

Leu Pro Thr Met Arg Asp Gly Leu Lys Gln Ala Asp Cys Thr Thr Val
225                 230                 235                 240

Asn Arg Ile Ala Met Phe Ile Ala Gln Thr Gly His Glu Ser Asp Asp
                245                 250                 255

Phe Arg Ala Thr Glu Glu Tyr Ala Asn Gly Pro Leu Asp Gln Glu Arg
            260                 265                 270

Trp Ile Tyr Lys Gly Arg Thr Trp Ile Gln Ile Thr Trp Arg Glu His
        275                 280                 285

Tyr Ala Arg Phe Gly Lys Trp Cys Phe Asp Arg Gly Leu Val Thr Asp
    290                 295                 300

Pro Asp Val Phe Val Lys Asn Pro Arg Ala Leu Ala Asp Leu Lys Trp
305                 310                 315                 320

Ala Gly Ile Gly Ala Ala Trp Tyr Trp Thr Val Glu Arg Pro Asp Ile
                325                 330                 335

Asn Ala Leu Cys Asp Arg Arg Asp Ile Glu Thr Val Ser Arg Arg Ile
            340                 345                 350

Asn Gly Thr Asn Pro Asn Thr Gly Arg Ala Asn His Ile Glu Glu Arg
        355                 360                 365

Ile Ala Arg Trp Asn Arg Ala Leu Ala Val Gly Asp Asp Leu Leu Gln
    370                 375                 380

Leu Ile Arg Glu Glu Glu Asp Gly Phe Leu Ser Ala Leu Thr Pro Ala
385                 390                 395                 400

Glu Gln Arg Ala Leu Tyr Asn Glu Ile Met Lys Lys Gly Pro Thr Arg
                405                 410                 415

Ser Phe Met Ala Glu Asp Gln Asn Gln Ile Glu Thr Leu Leu Gly Phe
            420                 425                 430

Val Tyr Asn Ile Asp Gly Asn Ile Trp Asn Asp Ala Val Thr Arg Ala
        435                 440                 445

Tyr Leu Phe Asp Val Pro Leu Ala Val Glu Tyr Val Glu Arg Val Ala
    450                 455                 460

Arg Asp Gly Val His Pro Lys Ser Trp Ala Phe Gln Gln Leu Asp Gly
465                 470                 475                 480

Lys Gly Glu Arg Trp Leu Ala Lys Phe Gly Gln Glu Tyr Cys Lys Gly
                485                 490                 495

Leu Ile Arg Phe Lys Lys Lys Leu Asn Asp Leu Leu Glu Pro Tyr Gly
            500                 505                 510

Glu Asn Asp Cys Tyr Cys Arg Ile Pro Ala Cys Ile Ala Gly Glu Arg
        515                 520                 525

Arg Tyr Gly Thr Cys Ile Tyr Gln Gly Arg Leu Trp Ala Phe Cys Cys
    530                 535                 540

Tyr Ala Arg Lys Ala Arg Arg Gln Ala Arg Arg
545                 550                 555

<210>  54
<211>  1665
<212>  DNA
<213>  Artificial sequence

<220>
<223>  Bzx2gp11/alpha-defensin/PTD3

<400>  54
atgacagaga aagttttacc atacgatcgg agtatagtta cacaagagac aggttggtgg      60

tgtggtccag ccgcaacaca agtagtttta aatagtcgtg gaataatcgt accagaagct     120

acattggccg ctgaaatcga agccatagaa aatccaggtc gtggtgatga ccgagacgga     180

acagattatg taggtcttat tgagcaagtt cttgatcgga gggttccaca agctcgttac     240

acaagcgtat atttaacaaa tgatccacca acacaagcac aaaaggaccg attgtgggaa     300

catattgtaa gaagcataaa tgcaggttac ggagtagtta tgaattgggt agctccacca     360

agtaataagc cacgtggagt taaaggtagc gttagcccac gatatagtgg aggtacaaca     420

tatcactatg tagcgtgcat gggatatgac gatacaccag gtgccagagc agtatggata     480

gcggacagcg gattccaacc acaaggatac tggatcagct ttgaccaatg cgccacactt     540

atcccaccaa aaggttatgc atacgcagac gcggcaccag ctgcaccagc tccagctcca     600

acaccagttg tagacgccgc gccaatattg gcccgggcgg caggtattag cgaagcaaag     660

gcaagggaaa ttcttccaac aatgcgggat ggactaaaac aagcggattg tacaacagtt     720

aatcgaattg caatgtttat agcccaaaca ggacacgaaa gtgatgactt tcgggctaca     780

gaagagtatg caaatggacc attggatcaa gaacgttgga tttacaaagg tcgaacatgg     840

atacaaatta catggaggga acactacgct cgtttcggta aatggtgttt cgaccgtgga     900

cttgttacag atccagatgt atttgttaaa aatccacgag ctttggccga cctaaaatgg     960

gcgggtatag gagcggcttg gtactggaca gtagaacggc cagatatcaa tgccttatgc 1020

gatcgaagag acatcgaaac agttagtaga aggataaatg gaacaaatcc aaatacaggt 1080

agagcgaatc atatcgagga acgaattgcg aggtggaatc gtgcactagc agtaggtgac 1140

gatttacttc aactaattag agaagaggaa gacggttttt taagtgcttt gacaccagcc 1200

gagcaacgag cgttgtataa tgaaatcatg aagaaaggac caacacgtag tttcatggct 1260

gaggatcaaa atcaaatcga gacattattg ggattcgtat acaatataga cggaaatatc 1320

tggaatgacg ctgtaacacg ggcttattta tttgatgttc cattagccgt agaatacgtt 1380

gagagggttg cgagggatgg agtacatcca aagagctggg catttcaaca actagacgga 1440

aagggagaaa ggtggctagc caagttcgga caagaatact gtaaaggtct aatccggttc 1500

aagaaaaagt tgaatgatct acttgaacca tatggagaga atgactgcta ctgtcggatt 1560

ccagcgtgca ttgctggtga aaggagatat ggtacatgta tatatcaagg tagactttgg 1620

gccttttgtt gctacgcgag aaaggccaga aggcaagcgc gtcga 1665

<210> 55
<211> 555
<212> PRT
<213> Artificial sequence

<220>
<223> PTD3/alpha-defensin/Bzx2gp11

<400> 55

Tyr Ala Arg Lys Ala Arg Arg Gln Ala Arg Arg Asp Cys Tyr Cys Arg
1               5                   10                  15

Ile Pro Ala Cys Ile Ala Gly Glu Arg Arg Tyr Gly Thr Cys Ile Tyr
            20                  25                  30

Gln Gly Arg Leu Trp Ala Phe Cys Cys Met Thr Glu Lys Val Leu Pro
        35                  40                  45

Tyr Asp Arg Ser Ile Val Thr Gln Glu Thr Gly Trp Trp Cys Gly Pro
    50                  55                  60

Ala Ala Thr Gln Val Val Leu Asn Ser Arg Gly Ile Ile Val Pro Glu
65                  70                  75                  80

Ala Thr Leu Ala Ala Glu Ile Glu Ala Ile Glu Asn Pro Gly Arg Gly
                85                  90                  95

Asp Asp Arg Asp Gly Thr Asp Tyr Val Gly Leu Ile Glu Gln Val Leu
            100                 105                 110

Asp Arg Arg Val Pro Gln Ala Arg Tyr Thr Ser Val Tyr Leu Thr Asn
        115                 120                 125

Asp Pro Pro Thr Gln Ala Gln Lys Asp Arg Leu Trp Glu His Ile Val
    130                 135                 140

Arg Ser Ile Asn Ala Gly Tyr Gly Val Val Met Asn Trp Val Ala Pro
145                 150                 155                 160

Pro Ser Asn Lys Pro Arg Gly Val Lys Gly Ser Val Ser Pro Arg Tyr
                165                 170                 175

Ser Gly Gly Thr Thr Tyr His Tyr Val Ala Cys Met Gly Tyr Asp Asp
            180                 185                 190

Thr Pro Gly Ala Arg Ala Val Trp Ile Ala Asp Ser Gly Phe Gln Pro
        195                 200                 205

Gln Gly Tyr Trp Ile Ser Phe Asp Gln Cys Ala Thr Leu Ile Pro Pro
    210                 215                 220

Lys Gly Tyr Ala Tyr Ala Asp Ala Ala Pro Ala Ala Pro Ala Pro Ala
225                 230                 235                 240

Pro Thr Pro Val Val Asp Ala Ala Pro Ile Leu Ala Arg Ala Ala Gly
                245                 250                 255

Ile Ser Glu Ala Lys Ala Arg Glu Ile Leu Pro Thr Met Arg Asp Gly
            260                 265                 270

Leu Lys Gln Ala Asp Cys Thr Thr Val Asn Arg Ile Ala Met Phe Ile
        275                 280                 285

Ala Gln Thr Gly His Glu Ser Asp Asp Phe Arg Ala Thr Glu Glu Tyr
    290                 295                 300

Ala Asn Gly Pro Leu Asp Gln Glu Arg Trp Ile Tyr Lys Gly Arg Thr
305                 310                 315                 320

Trp Ile Gln Ile Thr Trp Arg Glu His Tyr Ala Arg Phe Gly Lys Trp
                325                 330                 335

Cys Phe Asp Arg Gly Leu Val Thr Asp Pro Asp Val Phe Val Lys Asn
            340                 345                 350

Pro Arg Ala Leu Ala Asp Leu Lys Trp Ala Gly Ile Gly Ala Ala Trp
        355                 360                 365

Tyr Trp Thr Val Glu Arg Pro Asp Ile Asn Ala Leu Cys Asp Arg Arg
    370                 375                 380

Asp Ile Glu Thr Val Ser Arg Arg Ile Asn Gly Thr Asn Pro Asn Thr
385                 390                 395                 400

Gly Arg Ala Asn His Ile Glu Glu Arg Ile Ala Arg Trp Asn Arg Ala
                405                 410                 415

Leu Ala Val Gly Asp Asp Leu Leu Gln Leu Ile Arg Glu Glu Glu Asp
            420                 425                 430

Gly Phe Leu Ser Ala Leu Thr Pro Ala Glu Gln Arg Ala Leu Tyr Asn
        435                 440                 445

Glu Ile Met Lys Lys Gly Pro Thr Arg Ser Phe Met Ala Glu Asp Gln
    450                 455                 460

Asn Gln Ile Glu Thr Leu Leu Gly Phe Val Tyr Asn Ile Asp Gly Asn
465                 470                 475                 480

Ile Trp Asn Asp Ala Val Thr Arg Ala Tyr Leu Phe Asp Val Pro Leu
                485                 490                 495

Ala Val Glu Tyr Val Glu Arg Val Ala Arg Asp Gly Val His Pro Lys
            500                 505                 510

Ser Trp Ala Phe Gln Gln Leu Asp Gly Lys Gly Glu Arg Trp Leu Ala
        515                 520                 525

Lys Phe Gly Gln Glu Tyr Cys Lys Gly Leu Ile Arg Phe Lys Lys Lys
    530                 535                 540

Leu Asn Asp Leu Leu Glu Pro Tyr Gly Glu Asn
545                 550                 555

<210>  56
<211>  1665
<212>  DNA
<213>  Artificial sequence

<220>
<223>  PTD3/alpha-defensin/Bzx2gp11

<400>  56
tatgcacgca aagcgagacg acaagcccgt cgcgactgct attgtcgcat ccctgcatgt      60

atagcaggtg agcgtcggta cgggacatgc atttatcaag gtaggttatg ggctttttgt     120

tgcatgacgg agaaggtttt gccttacgat cgtagtatag ttacccagga aaccggctgg     180

tggtgtggac cagcagccac gcaggtggtc ttgaatagca ggggcatcat tgtgccagag     240

gcgactcttg ccgctgaaat tgaggctatc gagaatccag ggaggggaga tgacagagat     300

ggtactgatt acgtaggctt aatagaacag gtccttgatc gacgtgtgcc tcaagctagg     360

tatacgtctg tttatttaac aaacgatccc ccaactcaag cgcagaaaga ccgcttatgg 420

gaacacatag tacggagcat caatgcgggt tacggcgttg tcatgaactg ggtggcccca 480

ccttctaaca aaccccgtgg ggtcaagggt tccgtatctc cccggtattc aggcggaact 540

acatatcatt atgtcgcttg catgggttac gacgatacac cgggagccag agctgtatgg 600

atagcagatt ccggattcca acctcagggc tattggatta gtttcgacca gtgcgctacg 660

ttaataccgc ctaagggata tgcctacgca gacgccgctc cggcggctcc cgcaccagca 720

ccgacgccgg tagttgacgc cgcaccgatt ttggcgaggg ctgcggggat aagtgaagcg 780

aaagcacgag agatcttacc cactatgcga gacggcctta agcaggcgga ctgcacaacg 840

gtaaacagga tcgcgatgtt tatagctcaa accgggcatg aaagcgatga cttcagggca 900

accgaggaat acgctaatgg tccacttgat caagagcggt ggatttacaa aggcagaacc 960

tggatacaaa ttacgtggcg cgagcactac gcccgatttg ggaagtggtg ttttgatcgg 1020

ggtctcgtca cagatccaga tgtgtttgtg aaaaacccca gagctttggc agacctcaaa 1080

tgggcgggga tcggcgcagc ctggtactgg actgtcgagc gtccggatat taatgcgctg 1140

tgtgacagac gagatattga gaccgtctcg cgacggatca atggaacaaa tcccaataca 1200

gggcgcgcta accatattga agagcgcata gcccggtgga acagagctct ggcagttgga 1260

gacgatctcc tgcaactgat ccgcgaggaa gaggacgggt ttctgtcagc tctaacccca 1320

gcggaacaaa gagcgcttta caacgagatc atgaagaaag ggcctactcg ttcattcatg 1380

gccgaagacc agaatcagat agaaacgcta ttgggctttg tatataacat tgacggtaat 1440

atctggaacg atgccgtaac tcgggcgtat ctattcgacg tgccgctagc cgttgaatac 1500

gtggaaagag ttgcacgaga cggagtacac cctaagtcgt gggcattcca acagctcgat 1560

ggtaaaggag agaggtggct agccaaattc ggtcaggaat actgtaaggg acttatacgt 1620

tttaagaaaa agctcaacga tttgctagaa ccctatgggg aaaat 1665

<210> 57
<211> 585
<212> PRT
<213> Artificial sequence

<220>
<223> PTD3/alpha-defensin/bzx2gp11/alpha-defensin

<400> 57

Tyr Ala Arg Lys Ala Arg Arg Gln Ala Arg Arg Asp Cys Tyr Cys Arg
1               5                   10                  15

Ile Pro Ala Cys Ile Ala Gly Glu Arg Arg Tyr Gly Thr Cys Ile Tyr
            20                  25                  30

Gln Gly Arg Leu Trp Ala Phe Cys Cys Met Thr Glu Lys Val Leu Pro

35                  40                  45

Tyr Asp Arg Ser Ile Val Thr Gln Glu Thr Gly Trp Trp Cys Gly Pro
    50                  55                  60

Ala Ala Thr Gln Val Val Leu Asn Ser Arg Gly Ile Ile Val Pro Glu
65                  70                  75                  80

Ala Thr Leu Ala Ala Glu Ile Glu Ala Ile Glu Asn Pro Gly Arg Gly
                85                  90                  95

Asp Asp Arg Asp Gly Thr Asp Tyr Val Gly Leu Ile Glu Gln Val Leu
            100                 105                 110

Asp Arg Arg Val Pro Gln Ala Arg Tyr Thr Ser Val Tyr Leu Thr Asn
        115                 120                 125

Asp Pro Pro Thr Gln Ala Gln Lys Asp Arg Leu Trp Glu His Ile Val
    130                 135                 140

Arg Ser Ile Asn Ala Gly Tyr Gly Val Val Met Asn Trp Val Ala Pro
145                 150                 155                 160

Pro Ser Asn Lys Pro Arg Gly Val Lys Gly Ser Val Ser Pro Arg Tyr
                165                 170                 175

Ser Gly Gly Thr Thr Tyr His Tyr Val Ala Cys Met Gly Tyr Asp Asp
            180                 185                 190

Thr Pro Gly Ala Arg Ala Val Trp Ile Ala Asp Ser Gly Phe Gln Pro
        195                 200                 205

Gln Gly Tyr Trp Ile Ser Phe Asp Gln Cys Ala Thr Leu Ile Pro Pro
    210                 215                 220

Lys Gly Tyr Ala Tyr Ala Asp Ala Ala Pro Ala Ala Pro Ala Pro Ala
225                 230                 235                 240

Pro Thr Pro Val Val Asp Ala Ala Pro Ile Leu Ala Arg Ala Ala Gly
                245                 250                 255

Ile Ser Glu Ala Lys Ala Arg Glu Ile Leu Pro Thr Met Arg Asp Gly
            260                 265                 270

Leu Lys Gln Ala Asp Cys Thr Thr Val Asn Arg Ile Ala Met Phe Ile
        275                 280                 285

Ala Gln Thr Gly His Glu Ser Asp Asp Phe Arg Ala Thr Glu Glu Tyr
    290                 295                 300

Ala Asn Gly Pro Leu Asp Gln Glu Arg Trp Ile Tyr Lys Gly Arg Thr
305                 310                 315                 320

Trp Ile Gln Ile Thr Trp Arg Glu His Tyr Ala Arg Phe Gly Lys Trp
                325                 330                 335

Cys Phe Asp Arg Gly Leu Val Thr Asp Pro Asp Val Phe Val Lys Asn
            340                 345                 350

Pro Arg Ala Leu Ala Asp Leu Lys Trp Ala Gly Ile Gly Ala Ala Trp
        355                 360                 365

Tyr Trp Thr Val Glu Arg Pro Asp Ile Asn Ala Leu Cys Asp Arg Arg
    370                 375                 380

Asp Ile Glu Thr Val Ser Arg Arg Ile Asn Gly Thr Asn Pro Asn Thr
385                 390                 395                 400

Gly Arg Ala Asn His Ile Glu Glu Arg Ile Ala Arg Trp Asn Arg Ala
                405                 410                 415

Leu Ala Val Gly Asp Asp Leu Leu Gln Leu Ile Arg Glu Glu Glu Asp
            420                 425                 430

Gly Phe Leu Ser Ala Leu Thr Pro Ala Glu Gln Arg Ala Leu Tyr Asn
        435                 440                 445

Glu Ile Met Lys Lys Gly Pro Thr Arg Ser Phe Met Ala Glu Asp Gln
    450                 455                 460

Asn Gln Ile Glu Thr Leu Leu Gly Phe Val Tyr Asn Ile Asp Gly Asn
465                 470                 475                 480

Ile Trp Asn Asp Ala Val Thr Arg Ala Tyr Leu Phe Asp Val Pro Leu
                485                 490                 495

Ala Val Glu Tyr Val Glu Arg Val Ala Arg Asp Gly Val His Pro Lys
            500                 505                 510

Ser Trp Ala Phe Gln Gln Leu Asp Gly Lys Gly Glu Arg Trp Leu Ala
        515                 520                 525

Lys Phe Gly Gln Glu Tyr Cys Lys Gly Leu Ile Arg Phe Lys Lys Lys
    530                 535                 540

Leu Asn Asp Leu Leu Glu Pro Tyr Gly Glu Asn Asp Cys Tyr Cys Arg
545                 550                 555                 560

Ile Pro Ala Cys Ile Ala Gly Glu Arg Arg Tyr Gly Thr Cys Ile Tyr
                565                 570                 575

Gln Gly Arg Leu Trp Ala Phe Cys Cys
            580                 585

<210>  58
<211>  1755
<212>  DNA
<213>  Artificial sequence

<220>
<223>  PTD3/alpha-defensin/Bzx2gp11/alpha-defensin

<400>  58
tatgctcgta aggcgcgccg acaggctcgg cgcgactgtt attgtcggat cccggcgtgc      60

attgccggcg agcgcaggta cggtacgtgt atctaccagg gacggttatg ggcattttgc     120

tgtatgaccg aaaaggtcct accatacgac agaagcatcg tgacacagga gaccggttgg     180

tggtgtggtc ctgcggctac acaggtggtc cttaattctc gaggcattat agtgccggag     240

gccactcttg ccgctgaaat agaagccatt gaaaatcccg gccggggaga tgaccgggac     300

gggacggact acgttgggtt gattgaacaa gtattggatc gccgtgtacc gcaagcgaga     360

tatacgagtg tatacctgac taacgatccc ccgacgcaag cacaaaaaga tcgcctctgg     420

gaacatatag ttaggtccat aaatgcgggt tatggagtcg ttatgaactg ggtagcgccg     480

ccaagtaata aaccacgagg agtcaaaggt agtgtgtcac ctcgatactc aggcgggact     540

acgtatcact atgtagcttg tatgggctac gacgatacac caggcgcaag ggcggtttgg     600

atagcagatt cgggcttcca acctcaagga tattggatct cgtttgatca atgcgcaacc     660

cttattccgc caaagggata tgcgtacgca gacgctgcgc ccgcggcacc agctccggcc     720

cctactcctg ttgtagacgc agcccctata ttagcacgtg cagccgggat ctccgaggca     780

aaggctcgtg agatcttgcc cactatgcga gacgggctaa aacaggcaga ctgcacaacc     840

gttaatagaa tagccatgtt catagcccaa accggtcatg agagcgacga ttttcgggcc     900

acggaggaat acgctaacgg tcccctggac caggaaaggt ggatctataa ggggagaact     960

tggatacaga ttacctggag ggagcattat gcgcgtttcg gcaagtggtg cttcgaccga    1020

ggtttggtca ccgaccctga cgtctttgtt aaaaacccac gcgcactcgc ggacttgaaa    1080

tgggctggaa ttggggcagc ttggtattgg accgtagaaa gacctgatat caacgccctg    1140

tgcgaccgga gagatattga gacagtatca aggcgcatca atggaacgaa cccaaacact    1200

gggagggcca accacattga agagcgtatc gctagatgga atcgtgccct ggccgtgggc    1260

gatgacctat tacaactcat tcgcgaagag gaagatggtt ttcttagcgc gctcacgcct    1320

gctgaacaaa gagctcttta caacgagata atgaagaaag gacccacacg atcttttatg    1380

gcagaggacc aaaaccagat cgaaacacta ttaggtttcg tctataatat agatgggaat    1440

atatggaatg atgcagtgac tcgtgcttat ttattcgatg tacccttggc cgtggagtac 1500

gtcgagaggg tggcgcggga tggagttcac cccaagtctt gggcctttca acagctggat 1560

gggaagggcg agcgttggct agcaaaattc ggtcaggaat actgcaaagg tttaatccgg 1620

tttaaaaaga aactgaacga tctacttgag ccatacggcg aaaatgattg ttattgtaga 1680

ataccggcgt gcattgcggg ggaacgaagg tacggaacat gtatttatca gggacgactc 1740

tgggctttct gctgt 1755

<210> 59
<211> 341
<212> PRT
<213> Artificial sequence

<220>
<223> beta-defensin/L5gp10/TAT47-57

<400> 59

Asn Pro Val Ser Cys Val Arg Asn Lys Gly Ile Cys Val Pro Ile Arg
1               5                   10                  15

Cys Pro Gly Ser Met Lys Gln Ile Gly Thr Cys Val Gly Arg Ala Val
            20                  25                  30

Lys Cys Cys Arg Lys Lys Met Thr Phe Thr Val Thr Arg Glu Arg Ala
        35                  40                  45

Gln Trp Val His Asp Met Ala Arg Ala Arg Asp Gly Leu Pro Tyr Ala
    50                  55                  60

Tyr Gly Gly Ala Phe Thr Asn Asn Pro Arg Val Ser Thr Asp Cys Ser
65                  70                  75                  80

Gly Leu Val Leu Gln Thr Gly Ala Trp Tyr Gly Gly Arg Thr Asp Trp
                85                  90                  95

Val Gly Asn Arg Tyr Gly Ser Thr Glu Ser Phe Arg Leu Asp His Lys
            100                 105                 110

Ile Val Tyr Asp Leu Gly Phe Lys Arg Met Pro Arg Gly Gly Pro Ala
        115                 120                 125

Ala Leu Pro Ile Lys Pro Val Met Leu Val Gly Leu Gln His Gly Gly
    130                 135                 140

Gly Gly Val Tyr Ser His Thr Ala Cys Thr Leu Met Thr Met Asp His
145                 150                 155                 160

Pro Gly Gly Pro Val Lys Met Ser Asp Arg Gly Val Asp Trp Glu Ser

                165                 170                 175

His Gly Asn Arg Asn Gly Val Gly Val Glu Leu Tyr Glu Gly Ala Arg
            180                 185                 190

Ala Trp Asn Asp Pro Leu Phe His Asp Phe Trp Tyr Leu Asp Ala Val
        195                 200                 205

Leu Glu Asp Glu Gly Asp Asp Asp Glu Leu Ala Asp Pro Val Leu Gly
    210                 215                 220

Lys Met Ile Arg Glu Ile His Ala Cys Leu Phe Asn Gln Thr Ala Ser
225                 230                 235                 240

Thr Ser Asp Leu Ala Thr Pro Gly Glu Gly Ala Ile Trp Gln Leu His
                245                 250                 255

Gln Lys Ile His Ser Ile Asp Gly Met Leu His Pro Ile His Ala Glu
            260                 265                 270

Arg Arg Ala Arg Ala Gly Asp Leu Gly Glu Leu His Arg Ile Val Leu
        275                 280                 285

Ala Ala Lys Gly Leu Gly Val Lys Arg Asp Glu Val Thr Lys Arg Val
    290                 295                 300

Tyr Gln Ser Ile Leu Ala Asp Ile Glu Arg Asp Asn Pro Glu Val Leu
305                 310                 315                 320

Gln Arg Tyr Ile Ala Glu Arg Gly Gly Leu Tyr Gly Arg Lys Lys Arg
                325                 330                 335

Arg Gln Arg Arg Arg
            340

<210> 60
<211> 1023
<212> DNA
<213> Artificial sequence

<220>
<223> beta-defensin/L5gp10/TAT47-57

<400> 60
aacccggtca gctgtgtcag gaacaaggga atatgtgttc ccattcgctg cccggggagc 60

atgaaacaaa ttggtacatg cgtagggcgt gcggtgaagt gctgtcgaaa gaaaatgacc 120

tttactgtga cacgggaaag ggctcagtgg gtccatgata tggcgcgcgc tagggacggt 180

ctaccttatg catatggagg ggcgtttacg aataacccac gcgtttcgac agactgttcg 240

ggcctggtcc tgcagactgg agcatggtat ggtggccgaa cagattgggt tgggaatcga 300

tatgggtcta cggagtcatt tagactcgac cataaaattg tatatgattt aggttttaaa 360

cgtatgcctc gggggggacc ggcagctctg cccatcaagc cagtaatgtt agtaggtcta 420

caacacggcg gtggaggggt gtactcccat actgcgtgca cgttaatgac catggaccat 480

ccaggcgggc cagtgaaaat gtctgatcgt ggagtagact gggaaagtca tgggaaccgc 540

aacggagttg gcgttgaact gtacgagggc gcccgggctt ggaatgatcc cttgttccac 600

gatttctggt acttggatgc cgtattggag gatgagggtg acgatgacga gctagccgat 660

ccggtccttg gcaaaatgat acgagagatc cacgcctgtt tattcaatca aaccgcttcc 720

actagtgatt tggcgacccc tggcgaaggt gccatatggc agctccatca gaagatccac 780

tcaatcgacg gtatgcttca ccctatccac gccgagagaa gggcacgcgc aggagactta 840

ggagaactac atagaatagt gctcgctgcg aaagggcttg gcgttaagcg ggacgaggtc 900

acgaagcgtg tataccaaag tattttggca gatattgaaa gagacaatcc cgaagtgctt 960

cagagataca tagcagaaag gggaggcctc tatggtagaa agaaacgaag gcaacgtcga 1020

cgg 1023

<210> 61
<211> 370
<212> PRT
<213> Artificial sequence

<220>
<223> alpha-defensin/Hepcidin/L5gp10/TAT47-57

<400> 61

Asn Pro Val Ser Cys Val Arg Asn Lys Gly Ile Cys Val Pro Ile Arg
1               5                   10                  15

Cys Pro Gly Ser Met Lys Gln Ile Gly Thr Cys Val Gly Arg Ala Val
            20                  25                  30

Lys Cys Cys Arg Lys Lys Gly Ala Gly Ala Asp Thr His Phe Pro Ile
        35                  40                  45

Cys Ile Phe Cys Cys Gly Cys Cys His Arg Ser Lys Cys Gly Met Cys
    50                  55                  60

Cys Lys Thr Met Thr Phe Thr Val Thr Arg Glu Arg Ala Gln Trp Val
65                  70                  75                  80

His Asp Met Ala Arg Ala Arg Asp Gly Leu Pro Tyr Ala Tyr Gly Gly
                85                  90                  95

Ala Phe Thr Asn Asn Pro Arg Val Ser Thr Asp Cys Ser Gly Leu Val
            100                 105                 110

Leu Gln Thr Gly Ala Trp Tyr Gly Gly Arg Thr Asp Trp Val Gly Asn
        115                 120                 125

Arg Tyr Gly Ser Thr Glu Ser Phe Arg Leu Asp His Lys Ile Val Tyr
    130                 135                 140

Asp Leu Gly Phe Lys Arg Met Pro Arg Gly Gly Pro Ala Ala Leu Pro
145                 150                 155                 160

Ile Lys Pro Val Met Leu Val Gly Leu Gln His Gly Gly Gly Gly Val
                165                 170                 175

Tyr Ser His Thr Ala Cys Thr Leu Met Thr Met Asp His Pro Gly Gly
            180                 185                 190

Pro Val Lys Met Ser Asp Arg Gly Val Asp Trp Glu Ser His Gly Asn
        195                 200                 205

Arg Asn Gly Val Gly Val Glu Leu Tyr Glu Gly Ala Arg Ala Trp Asn
    210                 215                 220

Asp Pro Leu Phe His Asp Phe Trp Tyr Leu Asp Ala Val Leu Glu Asp
225                 230                 235                 240

Glu Gly Asp Asp Asp Glu Leu Ala Asp Pro Val Leu Gly Lys Met Ile
                245                 250                 255

Arg Glu Ile His Ala Cys Leu Phe Asn Gln Thr Ala Ser Thr Ser Asp
            260                 265                 270

Leu Ala Thr Pro Gly Glu Gly Ala Ile Trp Gln Leu His Gln Lys Ile
        275                 280                 285

His Ser Ile Asp Gly Met Leu His Pro Ile His Ala Glu Arg Arg Ala
    290                 295                 300

Arg Ala Gly Asp Leu Gly Glu Leu His Arg Ile Val Leu Ala Ala Lys
305                 310                 315                 320

Gly Leu Gly Val Lys Arg Asp Glu Val Thr Lys Arg Val Tyr Gln Ser
                325                 330                 335

Ile Leu Ala Asp Ile Glu Arg Asp Asn Pro Glu Val Leu Gln Arg Tyr
            340                 345                 350

Ile Ala Glu Arg Gly Gly Leu Tyr Gly Arg Lys Lys Arg Arg Gln Arg
        355                 360                 365

Arg Arg
    370


<210>  62
<211>  1110
<212>  DNA
<213>  Artificial sequence

<220>
<223>  alpha-defensin/Hepcidin/L5gp10/TAT47-57

<400>  62
aatcctgtta gctgcgttcg caataaaggc atctgtgtgc caatacgatg tccaggtagt      60

atgaaacaaa tcggaacatg cgtgggaaga gccgttaagt gttgccggaa gaaaggagct     120

ggtgcggata cacacttccc aatatgcatt ttctgttgcg ggtgttgcca tcgttctaaa     180

tgtggtatgt gctgtaagac catgacattt acggtaacac gggagcgagc ccaatgggta     240

catgacatgg cgcgcgccag agatggatta ccgtacgcat acggcggtgc atttacaaac     300

aatccgaggg tatccaccga ctgttctggg ctcgtattac aaacgggcgc gtggtacggt     360

ggcaggacgg attgggtagg gaaccggtat ggaagcactg agtcgtttag attggaccac     420

aagatagtgt atgacctcgg ctttaaaaga atgccccggg gaggccctgc tgcgttaccc     480

atcaagccag tcatgttagt tgggctacag cacggtggag gcggggtcta ttcacacacg     540

gcttgcactt taatgactat ggaccatccc gggggccccg ttaagatgag cgaccgtggg     600

gtcgattggg agtcccatgg aaatcgcaac ggggtaggtg tggaattgta tgaaggcgca     660

agagcctgga acgacccgct cttccatgat ttctggtatc ttgatgcggt tttggaagac     720

gagggcgatg acgatgaact agctgatcca gtgctgggta agatgatacg tgaaatccat     780

gcatgtctat ttaaccagac tgcatcgacc tcagacctcg cgactcctgg agagggagca     840

atatggcaac tgcaccagaa aatccacagt attgatggga tgctgcaccc tattcatgcg     900

gaacgtaggg ctagggccgg tgatttgggt gagctacatc gaattgtctt ggctgccaaa     960

gggcttggag tcaagcggga cgaggtgacc aaacgcgtat accagagtat tctggcagat    1020

attgaaaggg acaatccgga agtccttcaa cgatatatag ctgagcgtgg cggtctttac    1080

gggcgaaaga aacgtaggca gcgccgaaga                                     1110


<210>  63
<211>  456
<212>  PRT
<213>  Artificial sequence

<220>
<223>  TM4gp30/LL-37/TAT47-57

<400>  63

<210>  64
<211>  1368
<212>  DNA
<213>  Artificial sequence

<220>
<223>  TM4gp30/LL-37/TAT47-57

<400>  64
atggcttggg tcggctggca gcttggtatg cagggtgaac aggttaaggt aatccagcaa      60

aagttgatag ccaagtacca gtgggtgcgt gatagatatc cccggttgac cgcaagtggg     120

gtttacgacg tcaacaccca agcagcgatt gttgagtttc aatttagagc cggacttcca     180

gtcacgggca tagccgacta tgcgacacag gtgcggcttg gagctgtcgc accagctcca     240

ccccctaggc aacggattat ggtactcaca tttagcggga cgtcggctga tatgtggaca 300

ggctacccgg ctgacgttgc aagagcccta gatccttcaa ttttttattg gcaacccgtt 360

tgttatggcc caaacggtat tcccgcgata ttcccgatgg ggtcgagtgc caagtccggg 420

gaagttgaag gcttgcgact attagacgag aaagctcgcg atttcgacta cattgtcctg 480

atcgggtata gtcagggtgc gttgcctgcg agcaggttga tgcgtcgcat cctatcaggt 540

gatctgcaaa ggttcaaatc aaaactgata gcaggagtga cgtttgggaa cccgatgcga 600

gaaaaaggtc acacctttcc tggcggggct gacccgggtg gccacggctt agacccacag 660

tgcctcgtga atactccgga ttggtggcac gactacgcgg ccaaaggcga tatctacaca 720

gtaggttctg gatctaatga tgagaaggcc aatgcggaca tgactttcat atatcaatta 780

gtccaaggag acatactcgg tatgatgttc ggtacgggta accctttgga tatattaggg 840

ttgctgggag gcctaggcgg aggtctactt ggaggcctag gtgggggctt gcttggaggt 900

ggaaaggggg gattacaact gcctagcgga cttgttttac ctggggttca ggggggtgca 960

ttaaccgacc atcaacgtgg acttgtagaa gcggtgctgg ctttactcgc aaatcccttc 1020

gcggaagtac ccgccgcggt gaaggcaatc gtaagtggcg tcggtttcat cgccactaat 1080

ccgccaactg ccccacatat tgagtaccat attagggagg cagctcccgg cgtgacatat 1140

ttccaacatg caatcgatta tctcaggcaa gtcggagcct ccgtggctgc acgggctgcg 1200

ggatctggat cactactggg agattttttc cgaaagtcta aagagaaaat agggaaagaa 1260

tttaagcgaa ttgtacagcg catcaaggat tttctacgaa acctcgtacc gcgtactgag 1320

agcggggcag gggcttacgg tcgcaaaaaa cgccggcaga gacgtaga 1368

<210> 65
<211> 448
<212> PRT
<213> Artificial sequence

<220>
<223> TM4gp30/LL-37/TAT47-57

<400> 65

Met Ala Trp Val Gly Trp Gln Leu Gly Met Gln Gly Glu Gln Val Lys
1               5                   10                  15

Val Ile Gln Gln Lys Leu Ile Ala Lys Tyr Gln Trp Val Arg Asp Arg
            20                  25                  30

Tyr Pro Arg Leu Thr Ala Ser Gly Val Tyr Asp Val Asn Thr Gln Ala
        35                  40                  45

Ala Ile Val Glu Phe Gln Phe Arg Ala Gly Leu Pro Val Thr Gly Ile
    50                  55                  60

Ala Asp Tyr Ala Thr Gln Val Arg Leu Gly Ala Val Ala Pro Ala Pro
65                  70                  75                  80

Pro Pro Arg Gln Arg Ile Met Val Leu Thr Phe Ser Gly Thr Ser Ala
                85                  90                  95

Asp Met Trp Thr Gly Tyr Pro Ala Asp Val Ala Arg Ala Leu Asp Pro
            100                 105                 110

Ser Ile Phe Tyr Trp Gln Pro Val Cys Tyr Gly Pro Asn Gly Ile Pro
        115                 120                 125

Ala Ile Phe Pro Met Gly Ser Ser Ala Lys Ser Gly Glu Val Glu Gly
    130                 135                 140

Leu Arg Leu Leu Asp Glu Lys Ala Arg Asp Phe Asp Tyr Ile Val Leu
145                 150                 155                 160

Ile Gly Tyr Ser Gln Gly Ala Leu Pro Ala Ser Arg Leu Met Arg Arg
                165                 170                 175

Ile Leu Ser Gly Asp Leu Gln Arg Phe Lys Ser Lys Leu Ile Ala Gly
            180                 185                 190

Val Thr Phe Gly Asn Pro Met Arg Glu Lys Gly His Thr Phe Pro Gly
        195                 200                 205

Gly Ala Asp Pro Gly Gly His Gly Leu Asp Pro Gln Cys Leu Val Asn
    210                 215                 220

Thr Pro Asp Trp Trp His Asp Tyr Ala Ala Lys Gly Asp Ile Tyr Thr
225                 230                 235                 240

Val Gly Ser Gly Ser Asn Asp Glu Lys Ala Asn Ala Asp Met Thr Phe
                245                 250                 255

Ile Tyr Gln Leu Val Gln Gly Asp Ile Leu Gly Met Met Phe Gly Thr
            260                 265                 270

Gly Asn Pro Leu Asp Ile Leu Gly Leu Leu Gly Gly Leu Gly Gly Gly
        275                 280                 285

Leu Leu Gly Gly Leu Gly Gly Gly Leu Leu Gly Gly Gly Lys Gly Gly
    290                 295                 300

Leu Gln Leu Pro Ser Gly Leu Val Leu Pro Gly Val Gln Gly Gly Ala
305                 310                 315                 320

Leu Thr Asp His Gln Arg Gly Leu Val Glu Ala Val Leu Ala Leu Leu

325                 330                 335

Ala Asn Pro Phe Ala Glu Val Pro Ala Ala Val Lys Ala Ile Val Ser
            340                 345                 350

Gly Val Gly Phe Ile Ala Thr Asn Pro Pro Thr Ala Pro His Ile Glu
        355                 360                 365

Tyr His Ile Arg Glu Ala Ala Pro Gly Val Thr Tyr Phe Gln His Ala
    370                 375                 380

Ile Asp Tyr Leu Arg Gln Val Gly Ala Ser Val Ala Ala Arg Ala Ala
385                 390                 395                 400

Leu Leu Gly Asp Phe Phe Arg Lys Ser Lys Glu Lys Ile Gly Lys Glu
                405                 410                 415

Phe Lys Arg Ile Val Gln Arg Ile Lys Asp Phe Leu Arg Asn Leu Val
            420                 425                 430

Pro Arg Thr Glu Ser Tyr Gly Arg Lys Lys Arg Arg Gln Arg Arg Arg
        435                 440                 445

<210>  66
<211>  1344
<212>  DNA
<213>  Artificial sequence

<220>
<223>  TM4gp30/LL-37/TAT47-57

<400>  66
atggcctggg ttggctggca gttaggaatg cagggtgaac aggtgaaggt aatacagcaa      60

aaacttatag ccaagtacca gtgggtcagg gatagatatc cccgcctaac cgcgagtggg     120

gtttacgacg tgaatactca ggcagctatc gttgagttcc aatttagagc tggcctacca     180

gtaactggta tagcagacta tgctactcaa gtgcggttag gggcggtcgc gcctgctccg     240

ccaccccgtc aacgaatcat ggtactgaca ttttcaggca catcagcgga tatgtggact     300

ggatatcccg cagacgtcgc gagggctctt gacccctcaa tattctattg gcagcccgtg     360

tgctacgggc ccaacggaat tccggccata ttccctatgg ggtcctctgc aaaatctggc     420

gaggttgagg gtttgcgtct actggacgaa aaagcccgag actttgatta catagtgctg     480

ataggttata gtcaaggagc gctcccggcg agtaggttaa tgcggcgaat tctgagcggc     540

gatctccaac ggtttaaaag caagcttatc gcaggtgtaa cctttgggaa tcctatgcgg     600

gagaagggac atacatttcc tggaggtgcc gacccaggtg gacacggctt agatcctcaa     660

tgtctcgtca ataccccgga ttggtggcac gattacgccg cgaagggaga tatctacaca     720

gttggaagcg gtagcaatga cgaaaaagct aacgcggaca tgacatttat ttatcagctg     780

gtgcaggggg acatcctagg catgatgttc ggaacgggta accctctgga tatattgggt 840

cttctcgggg gcttaggcgg gggcttattg ggcggtctag ggggcggtct tctgggcgga 900

gggaagggag gtctacagtt accctctgga ctagtgttgc cgggggtaca aggaggtgct 960

ttgactgatc atcagagagg gcttgtagaa gcagtcctcg ctttgctcgc caatccgttt 1020

gctgaagttc cagcggccgt taaggccatt gtgtccggcg tcggattcat cgcaacgaac 1080

ccacctaccg caccgcatat cgaatatcat attcgcgagg cagccccagg ggtcacgtat 1140

ttccaacacg cgattgatta cttgagacaa gtaggggcct cggttgcagc tcgtgctgca 1200

ttacttggtg attttttccg caaatcgaag gagaagattg gtaaagaatt caaacgtatc 1260

gtccaacgca ttaaagactt cctacgaaac ttggtaccaa ggacggagag ttacggacgc 1320

aaaaagcggc gacaaaggag acgt 1344

<210> 67
<211> 295
<212> PRT
<213> Artificial sequence

<220>
<223> D29gp12/alpha-defensin/PTD3

<400> 67

Met Ser Lys Pro Trp Leu Phe Thr Val His Gly Thr Gly Gln Pro Asp
1               5                   10                  15

Pro Leu Gly Pro Gly Leu Pro Ala Asp Thr Ala Arg Asp Val Leu Asp
            20                  25                  30

Ile Tyr Arg Trp Gln Pro Ile Gly Asn Tyr Pro Ala Ala Ala Phe Pro
        35                  40                  45

Met Trp Pro Ser Val Glu Lys Gly Val Ala Glu Leu Ile Leu Gln Ile
    50                  55                  60

Glu Leu Lys Leu Asp Ala Asp Pro Tyr Ala Asp Phe Ala Met Ala Gly
65                  70                  75                  80

Tyr Ser Gln Gly Ala Ile Val Val Gly Gln Val Leu Lys His His Ile
                85                  90                  95

Leu Pro Pro Thr Gly Arg Leu His Arg Phe Leu His Arg Leu Lys Lys
            100                 105                 110

Val Ile Phe Trp Gly Asn Pro Met Arg Gln Lys Gly Phe Ala His Ser
        115                 120                 125

Asp Glu Trp Ile His Pro Val Ala Ala Pro Asp Thr Leu Gly Ile Leu

130                 135                 140

Glu Asp Arg Leu Glu Asn Leu Glu Gln Tyr Gly Phe Glu Val Arg Asp
145                 150                 155                 160

Tyr Ala His Asp Gly Asp Met Tyr Ala Ser Ile Lys Glu Asp Asp Leu
                165                 170                 175

His Glu Tyr Glu Val Ala Ile Gly Arg Ile Val Met Lys Ala Ser Gly
            180                 185                 190

Phe Ile Gly Gly Arg Asp Ser Val Val Ala Gln Leu Ile Glu Leu Gly
        195                 200                 205

Gln Arg Pro Ile Thr Glu Gly Ile Ala Leu Ala Gly Ala Ile Ile Asp
    210                 215                 220

Ala Leu Thr Phe Phe Ala Arg Ser Arg Met Gly Asp Lys Trp Pro His
225                 230                 235                 240

Leu Tyr Asn Arg Tyr Pro Ala Val Glu Phe Leu Arg Gln Ile Asp Cys
                245                 250                 255

Tyr Cys Arg Ile Pro Ala Cys Ile Ala Gly Glu Arg Arg Tyr Gly Thr
            260                 265                 270

Cys Ile Tyr Gln Gly Arg Leu Trp Ala Phe Cys Cys Tyr Ala Arg Lys
        275                 280                 285

Ala Arg Arg Gln Ala Arg Arg
    290                 295

<210>  68
<211>  885
<212>  DNA
<213>  Artificial sequence

<220>
<223>  D29gp12/alpha-defensin/PTD3

<400>  68
atgagtaaac cttggctatt cacagtgcac ggtacgggtc agcctgaccc actaggtcct      60

gggcttccag cagatactgc gcgcgatgtg ctcgacattt atcgctggca acccatcgga     120

aattatcctg cagcggcttt tccgatgtgg ccgtccgttg aaaaaggggt agcagagctc     180

atattacaga tcgaactgaa attggatgct gacccgtatg cagattttgc gatggcgggg     240

tatagccagg gagccatcgt tgtgggccaa gttcttaagc accatattct accaccgacc     300

ggacgtctcc ataggttcct gcatcgttta aaaaaggtca tttttggggg caaccccatg     360

cggcaaaagg gtttcgctca ctcggatgag tggatccacc cagtagctgc acccgacacg     420

ttaggtatcc tggaggatcg tctggagaac ttagagcagt acggatttga agtcagagac 480

tacgctcatg atggtgatat gtatgcgagc attaaggaag acgatcttca tgagtacgaa 540

gtggcgatag gcagaatagt tatgaaagcc tctggattta tcggcgggag ggatagtgtc 600

gtagcccaat tgattgaatt gggccaacgg cccataactg agggtattgc cctagctgga 660

gctataatcg acgcattaac cttctttgcc cgatcacgga tgggcgacaa atggccacac 720

ctctataata gataccctgc ggtagaattc cttcgtcaga ttgactgtta ctgccgcata 780

ccggcgtgca tagcagggga aaggcgctac gggacatgca tatatcaggg acgattgtgg 840

gccttctgtt gttacgccag gaaggcacga agacaagctc gacgg 885

<210> 69
<211> 295
<212> PRT
<213> Artificial sequence

<220>
<223> PTD3/alpha-defensin/D29gp12

<400> 69

Tyr Ala Arg Lys Ala Arg Arg Gln Ala Arg Arg Asp Cys Tyr Cys Arg
1               5                   10                  15

Ile Pro Ala Cys Ile Ala Gly Glu Arg Arg Tyr Gly Thr Cys Ile Tyr
            20                  25                  30

Gln Gly Arg Leu Trp Ala Phe Cys Cys Met Ser Lys Pro Trp Leu Phe
        35                  40                  45

Thr Val His Gly Thr Gly Gln Pro Asp Pro Leu Gly Pro Gly Leu Pro
    50                  55                  60

Ala Asp Thr Ala Arg Asp Val Leu Asp Ile Tyr Arg Trp Gln Pro Ile
65                  70                  75                  80

Gly Asn Tyr Pro Ala Ala Ala Phe Pro Met Trp Pro Ser Val Glu Lys
                85                  90                  95

Gly Val Ala Glu Leu Ile Leu Gln Ile Glu Leu Lys Leu Asp Ala Asp
            100                 105                 110

Pro Tyr Ala Asp Phe Ala Met Ala Gly Tyr Ser Gln Gly Ala Ile Val
        115                 120                 125

Val Gly Gln Val Leu Lys His His Ile Leu Pro Pro Thr Gly Arg Leu
    130                 135                 140

His Arg Phe Leu His Arg Leu Lys Lys Val Ile Phe Trp Gly Asn Pro

145                 150                 155                 160

Met Arg Gln Lys Gly Phe Ala His Ser Asp Glu Trp Ile His Pro Val
                165                 170                 175

Ala Ala Pro Asp Thr Leu Gly Ile Leu Glu Asp Arg Leu Glu Asn Leu
            180                 185                 190

Glu Gln Tyr Gly Phe Glu Val Arg Asp Tyr Ala His Asp Gly Asp Met
        195                 200                 205

Tyr Ala Ser Ile Lys Glu Asp Asp Leu His Glu Tyr Glu Val Ala Ile
    210                 215                 220

Gly Arg Ile Val Met Lys Ala Ser Gly Phe Ile Gly Gly Arg Asp Ser
225                 230                 235                 240

Val Val Ala Gln Leu Ile Glu Leu Gly Gln Arg Pro Ile Thr Glu Gly
                245                 250                 255

Ile Ala Leu Ala Gly Ala Ile Ile Asp Ala Leu Thr Phe Phe Ala Arg
            260                 265                 270

Ser Arg Met Gly Asp Lys Trp Pro His Leu Tyr Asn Arg Tyr Pro Ala
        275                 280                 285

Val Glu Phe Leu Arg Gln Ile
    290                 295

<210>  70
<211>  885
<212>  DNA
<213>  Artificial sequence

<220>
<223>  PTD3/alpha-defensin/D29gp12

<400>  70
tacgcacgga aggctcgcag acaggcccgc agagactgtt attgtcgtat accagcgtgt      60

atcgctgggg agaggcgtta tggcacgtgc atctaccaag ggaggctgtg ggcgttttgc     120

tgcatgagca aaccatggct ctttacggta cacggtactg gacaaccaga cccgctaggc     180

ccaggactac ccgcggatac agccagagat gtattagata tttatcggtg gcaaccgata     240

ggaaactacc ccgctgcagc tttcccaatg tggcctagtg ttgaaaaagg tgtcgccgaa     300

ctgattcttc aaattgaact caaattagac gcggaccctt atgctgattt tgcgatggca     360

ggatattcac agggcgcaat agtcgtgggt caagtgttga agcatcacat cctgcccccg     420

accggtcggt tacacagatt tttgcacagg ctaaagaaag ttattttctg gggtaatcct      480

atgcgccaaa aagggtttgc gcattccgac gagtggatac atccggttgc tgcccctgac     540

actcttggca tccttgagga tcgattggag aacttggaac agtacgggtt cgaagtacga 600

gattacgccc atgatggcga tatgtacgca agcattaagg aggatgacct ccacgagtat 660

gaggtcgcaa ttggaaggat cgtgatgaaa gcaagtggat tcataggtgg acgcgactcg 720

gtggttgccc agttaattga actcggtcag cgaccgataa cagaaggcat cgctctggcc 780

ggggctataa tcgacgcatt aacctttttc gcgcggtctc gtatggggga taagtggccc 840

catctttata atcgataccc tgcggtagaa ttcctacgtc agata 885

<210> 71
<211> 325
<212> PRT
<213> Artificial sequence

<220>
<223> PTD3/alpha-defensin/D29gp12/alpha-defensin

<400> 71

Tyr Ala Arg Lys Ala Arg Arg Gln Ala Arg Arg Asp Cys Tyr Cys Arg
1               5                   10                  15

Ile Pro Ala Cys Ile Ala Gly Glu Arg Arg Tyr Gly Thr Cys Ile Tyr
            20                  25                  30

Gln Gly Arg Leu Trp Ala Phe Cys Cys Met Ser Lys Pro Trp Leu Phe
        35                  40                  45

Thr Val His Gly Thr Gly Gln Pro Asp Pro Leu Gly Pro Gly Leu Pro
    50                  55                  60

Ala Asp Thr Ala Arg Asp Val Leu Asp Ile Tyr Arg Trp Gln Pro Ile
65                  70                  75                  80

Gly Asn Tyr Pro Ala Ala Ala Phe Pro Met Trp Pro Ser Val Glu Lys
                85                  90                  95

Gly Val Ala Glu Leu Ile Leu Gln Ile Glu Leu Lys Leu Asp Ala Asp
            100                 105                 110

Pro Tyr Ala Asp Phe Ala Leu Ala Gly Tyr Ser Gln Gly Ala Ile Val
        115                 120                 125

Val Gly Gln Val Leu Lys His His Ile Ile Asn Pro Arg Gly Arg Leu
    130                 135                 140

His Arg Phe Leu His Arg Leu Arg Lys Val Ile Phe Trp Gly Asn Pro
145                 150                 155                 160

Met Arg Gln Lys Gly Phe Ala His Thr Asp Glu Trp Ile His Gln Val

165                 170                 175

Ala Ala Ser Asp Thr Met Gly Ile Leu Glu Asp Arg Leu Glu Asn Leu
        180                 185                 190

Glu Gln Tyr Gly Phe Glu Val Arg Asp Tyr Ala His Asp Gly Asp Met
    195                 200                 205

Tyr Ala Ser Ile Lys Glu Asp Asp Met His Glu Tyr Glu Val Ala Ile
    210                 215                 220

Gly Arg Ile Val Met Ser Ala Arg Arg Phe Ile Gly Gly Lys Asp Ser
225                 230                 235                 240

Val Ile Ala Gln Leu Ile Glu Leu Gly Gln Arg Pro Ile Trp Glu Gly
                245                 250                 255

Ile Ala Met Ala Arg Ala Ile Ile Asp Ala Leu Thr Phe Phe Ala Lys
        260                 265                 270

Ser Thr Gln Gly Pro Ser Trp Pro His Leu Tyr Asn Arg Phe Pro Ala
    275                 280                 285

Val Glu Phe Leu Arg Arg Ile Asp Cys Tyr Cys Arg Ile Pro Ala Cys
    290                 295                 300

Ile Ala Gly Glu Arg Arg Tyr Gly Thr Cys Ile Tyr Gln Gly Arg Leu
305                 310                 315                 320

Trp Ala Phe Cys Cys
                325

<210>  72
<211>  975
<212>  DNA
<213>  Artificial sequence

<220>
<223>  PTD3/alpha-defensin/D29gp12/alpha-defensin

<400>  72
tacgcccgta aggcccggag acaggccagg cgtgactgtt attgcagaat ccccgcatgc      60

attgctggcg agcgtcgata tggtacgtgt atataccaag gccgcctttg ggccttttgc     120

tgtatgtcaa aaccctggtt attcaccgta cacggaacag gtcagcctga tccactgggt     180

ccggggcttc ctgccgacac agcgcgtgac gtactcgata tctacagatg gcagccaata     240

gggaattacc ccgcggctgc ctttccaatg tggccgtcag tcgagaaggg tgttgcagag     300

cttattttgc agattgagtt aaagctggac gctgatccat atgctgactt tgcactcgct     360

ggatatagtc aaggagcaat cgtcgtagga caggttctga aacatcacat tatcaaccca     420

cgaggacggt tgcatcgctt tttgcatagg ctccggaaag taatattttg gggtaatcct 480

atgaggcaga aggggtttgc acacacggat gaatggattc accaagttgc ggcaagtgac 540

accatgggca tactagagga cagattggaa aacttagaac aatacgggtt cgaggtgagg 600

gattatgcgc acgatggcga tatgtacgcg agcattaaag aagacgatat gcatgaatat 660

gaggtcgcga ttgggcgcat tgtgatgtct gcgagacgat ttataggtgg caaggattcg 720

gttatagccc aactgataga actaggtcaa cgacctatct gggaaggaat cgctatggcg 780

cgtgctatca ttgacgcact aactttcttc gctaaaagca ctcagggacc ctcctggccg 840

catctttata atcggttccc ggccgtggaa ttcctacggc gcatagattg ctattgcagg 900

atccctgcat gtatcgcagg ggagcgacgc tacgggacat gtatatacca aggcagatta 960

tgggctttct gctgt 975

<210> 73
<211> 303
<212> PRT
<213> Artificial sequence

<220>
<223> beta-defensin/D29gp12/TAT47-57

<400> 73

Asn Pro Val Ser Cys Val Arg Asn Lys Gly Ile Cys Val Pro Ile Arg
1               5                   10                  15

Cys Pro Gly Ser Met Lys Gln Ile Gly Thr Cys Val Gly Arg Ala Val
            20                  25                  30

Lys Cys Cys Arg Lys Lys Met Ser Lys Pro Trp Leu Phe Thr Val His
        35                  40                  45

Gly Thr Gly Gln Pro Asp Pro Leu Gly Pro Gly Leu Pro Ala Asp Thr
    50                  55                  60

Ala Arg Asp Val Leu Asp Ile Tyr Arg Trp Gln Pro Ile Gly Asn Tyr
65                  70                  75                  80

Pro Ala Ala Ala Phe Pro Met Trp Pro Ser Val Glu Lys Gly Val Ala
                85                  90                  95

Glu Leu Ile Leu Gln Ile Glu Leu Lys Leu Asp Ala Asp Pro Tyr Ala
            100                 105                 110

Asp Phe Ala Met Ala Gly Tyr Ser Gln Gly Ala Ile Val Val Gly Gln
        115                 120                 125

Val Leu Lys His His Ile Leu Pro Pro Thr Gly Arg Leu His Arg Phe

130                 135                 140

Leu His Arg Leu Lys Lys Val Ile Phe Trp Gly Asn Pro Met Arg Gln
145                 150                 155                 160

Lys Gly Phe Ala His Ser Asp Glu Trp Ile His Pro Val Ala Ala Pro
                165                 170                 175

Asp Thr Leu Gly Ile Leu Glu Asp Arg Leu Glu Asn Leu Glu Gln Tyr
            180                 185                 190

Gly Phe Glu Val Arg Asp Tyr Ala His Asp Gly Asp Met Tyr Ala Ser
        195                 200                 205

Ile Lys Glu Asp Asp Leu His Glu Tyr Glu Val Ala Ile Gly Arg Ile
    210                 215                 220

Val Met Lys Ala Ser Gly Phe Ile Gly Gly Arg Asp Ser Val Val Ala
225                 230                 235                 240

Gln Leu Ile Glu Leu Gly Gln Arg Pro Ile Thr Glu Gly Ile Ala Leu
                245                 250                 255

Ala Gly Ala Ile Ile Asp Ala Leu Thr Phe Phe Ala Arg Ser Arg Met
            260                 265                 270

Gly Asp Lys Trp Pro His Leu Tyr Asn Arg Tyr Pro Ala Val Glu Phe
        275                 280                 285

Leu Arg Gln Ile Tyr Gly Arg Lys Lys Arg Arg Gln Arg Arg Arg
    290                 295                 300

<210>  74
<211>  909
<212>  DNA
<213>  Artificial sequence

<220>
<223>  beta defensin/D29gp12/TAT47-57

<400>  74
aatcctgtaa gttgcgtacg gaacaaaggt atatgtgttc caattagatg cccaggtagc      60

atgaaacaga tagggacctg cgttggacgg gccgtaaaat gttgtcgcaa gaaaatgtct     120

aagccgtggc tattcacggt tcacggtaca ggccaacctg acccactcgg cccaggactt     180

cctgccgata ctgctcgcga cgtgttagat atatatcgtt ggcagccaat tggtaattac     240

ccggctgcag ctttccccat gtggccctcg gtcgagaagg gtgtggccga gctcatattg     300

cagattgaat tgaaattgga tgccgatccg tatgccgact ttgctatggc ggggtacagt     360

caaggcgcga tagtcgtagg ccaagtgcta aagcatcaca tcctccctcc cactggacga     420

ctccaccgtt ttctacatcg acttaaaaag gtcatctttt ggggaaaccc catgaggcaa 480

aagggctttg cacactccga cgagtggatc catccggttg ctgcaccaga cacgttgggt 540

attcttgaag acaggctgga gaacctggaa cagtatgggt tcgaagttcg ggattatgcg 600

catgatgggg acatgtacgc atcaataaaa gaagatgact tacacgaata tgaagtggca 660

attggaagaa tcgtcatgaa ggcgtcagga tttattggag gtagggatag cgtggtagca 720

caactaatcg agctggccca gcgtcccatc accgagggga tagcgcttgc tggggctatt 780

atcgacgccc tgacattctt tgcgcgctct cgaatggggg ataaatggcc tcatttatac 840

aatcgctatc cggcagtcga gttcttaaga cagatatacg gcagaaagaa aaggagacaa 900

cgacggcgt 909

<210> 75
<211> 332
<212> PRT
<213> Artificial sequence

<220>
<223> beta-defensin/Hepcidin/L5gp12/TAT47-57

<400> 75

Asn Pro Val Ser Cys Val Arg Asn Lys Gly Ile Cys Val Pro Ile Arg
1               5                   10                  15

Cys Pro Gly Ser Met Lys Gln Ile Gly Thr Cys Val Gly Arg Ala Val
            20                  25                  30

Lys Cys Cys Arg Lys Lys Gly Ala Gly Ala Asp Thr His Phe Pro Ile
        35                  40                  45

Cys Ile Phe Cys Cys Gly Cys Cys His Arg Ser Lys Cys Gly Met Cys
    50                  55                  60

Cys Lys Thr Met Ser Lys Pro Trp Leu Phe Thr Val His Gly Thr Gly
65                  70                  75                  80

Gln Pro Asp Pro Leu Gly Pro Gly Leu Pro Ala Asp Thr Ala Arg Asp
                85                  90                  95

Val Leu Asp Ile Tyr Arg Trp Gln Pro Ile Gly Asn Tyr Pro Ala Ala
            100                 105                 110

Ala Phe Pro Met Trp Pro Ser Val Glu Lys Gly Val Ala Glu Leu Ile
        115                 120                 125

Leu Gln Ile Glu Leu Lys Leu Asp Ala Asp Pro Tyr Ala Asp Phe Ala
    130                 135                 140

Leu Ala Gly Tyr Ser Gln Gly Ala Ile Val Val Gly Gln Val Leu Lys
145                 150                 155                 160

His His Ile Ile Asn Pro Arg Gly Arg Leu His Arg Phe Leu His Arg
                165                 170                 175

Leu Arg Lys Val Ile Phe Trp Gly Asn Pro Met Arg Gln Lys Gly Phe
            180                 185                 190

Ala His Thr Asp Glu Trp Ile His Gln Val Ala Ala Ser Asp Thr Met
        195                 200                 205

Gly Ile Leu Glu Asp Arg Leu Glu Asn Leu Glu Gln Tyr Gly Phe Glu
    210                 215                 220

Val Arg Asp Tyr Ala His Asp Gly Asp Met Tyr Ala Ser Ile Lys Glu
225                 230                 235                 240

Asp Asp Met His Glu Tyr Glu Val Ala Ile Gly Arg Ile Val Met Ser
                245                 250                 255

Ala Arg Arg Phe Ile Gly Gly Lys Asp Ser Val Ile Ala Gln Leu Ile
            260                 265                 270

Glu Leu Gly Gln Arg Pro Ile Trp Glu Gly Ile Ala Met Ala Arg Ala
        275                 280                 285

Ile Ile Asp Ala Leu Thr Phe Phe Ala Lys Ser Thr Gln Gly Pro Ser
    290                 295                 300

Trp Pro His Leu Tyr Asn Arg Phe Pro Ala Val Glu Phe Leu Arg Arg
305                 310                 315                 320

Ile Tyr Gly Arg Lys Lys Arg Arg Gln Arg Arg Arg
                325                 330

<210>  76
<211>  996
<212>  DNA
<213>  Artificial sequence

<220>
<223>  beta-defensin/Hepcidin/L5gp12/TAT47-57

<400>  76
aatcccgtga gctgtgtaag aaataaaggt atttgtgtgc caattcgatg cccaggaagt      60

atgaagcaga ttggaacatg tgttggccgt gcggtaaagt gctgtaggaa gaaaggcgca     120

ggagctgata ctcacttccc aatatgcatt ttctgctgtg ggtgctgtca tcgctcaaaa     180

tgcggtatgt gttgcaaaac aatgagtaaa ccttggttgt tcactgtcca tgggaccggc     240

caacctgatc ctttggggcc gggtttgcca gcggatacag ctagggatgt cttagatatc 300

taccgttggc aaccgatagg caattacccc gcggctgcgt tccctatgtg gccgagcgtt 360

gaaaagggag tagcggagct gatcctacag attgaactca agctggacgc tgacccctat 420

gctgactttg cacttgcagg gtactcgcaa ggagccattg tggtcggaca agttctaaaa 480

caccatatca taaacccccg aggccgatta catagattcc tacacaggct aaggaaggta 540

atattttggg gtaatcctat gcggcagaag ggctttgctc acactgacga gtggatacac 600

caggtcgcag cttcggatac catgggcata ttagaggatc gccttgagaa cttggaacaa 660

tatggttttg aagttcggga ctatgcccac gacggtgaca tgtacgcctc catcaaggag 720

gatgacatgc atgagtatga agtggcgatc gggcgaattg taatgtcagc ccgcagattc 780

atcggaggga aggattctgt gatcgcacaa ctcatagaac tgggacaacg gccgatatgg 840

gagggtattg ccatggcacg ggcaattata gacgcgctta cgtttttcgc caaatctacg 900

cagggtccat cctggccgca tctctacaac cgttttcccg ccgttgaatt tttaaggaga 960

atctatgggc gcaaaaaacg tagacagcgt cgacgg 996

## Description

The present invention relates to a composition having the activity of degrading the cell wall of a *Mycobacterium species* comprising: (a) a first fusion protein including (i) a first endolysin or a first domain, both having a first enzymatic activity, the enzymatic activity being at least one or more of the following: N-acetyl-b-D-muramidase (lysozyme, lytic transglycosylase), N-acetyl-b-D-glucosaminidase, N-acetylmuramoyl-L-alanine amidase, L-alanoyl-D-glutamate (LD) endopeptidase, c-D-glutamyl-meso-diaminopimelic acid (DL) peptidase, L-alanyl-D-iso-glutaminyl-meso-diaminopimelic acid (D-Ala-m-DAP) (DD) endopeptidase, or m-DAP-m-DAP (LD) endopeptidase, (ii) at least one peptide stretch fused to the N- or C-terminus of the endolysin having the first enzymatic activity or the domain having the first enzymatic activity, wherein the peptide stretch is selected from the group consisting of synthetic amphipathic peptide, synthetic cationic peptide, synthetic polycationic peptide, synthetic hydrophobic peptide, synthetic antimicrobial peptide (AMP) or naturally occurring AMP; and (iii) a protein transduction domain (PTD) being at the N- or C-terminus of the first fusion protein, wherein the PTD is having the characteristic to deliver a cargo from the extracellular to the intracellular space of a cell; and (b) a second fusion protein including (i) a second endolysin or a second domain, both having a second enzymatic activity, the enzymatic activity being at least one or more of the following: lipolytic activity, cutinase, mycolarabinogalactanesterase, or alpha/beta hydrolase; (ii) at least one peptide stretch fused to the N- or C-terminus of the endolysin having a second enzymatic activity or the domain having the second enzymatic activity, wherein the peptide stretch is selected from the group consisting of synthetic amphipathic peptide, synthetic cationic peptide, synthetic polycationic peptide, synthetic hydrophobic peptide, synthetic antimicrobial peptide (AMP) or naturally occurring AMP; and (iii) a protein transduction domain (PTD) being at the N- or C-terminus of the second fusion protein, wherein the PTD is having the characteristic to deliver a cargo from the extracellular to the intracellular space of a cell. Moreover, the present invention relates in particular to said composition for use as a medicament, a disinfectant, a feed additive, or a food additive as well as the use of said composition in the treatment or prevention of diseases caused by an infection with a *Mycobacterium species.* Mycobacteria are classified as Gram positive bacteria. In comparison to most of the Gram-positive bacteria however, the structure of the cell wall of mycobacteria is different in their composition. The complex structure of the cell wall of mycobacteria consists of a mycolic acid-rich outer membrane which is covalently linked to the arabinogalactan-petidoglycan complex (Hoffmann et al., 2008; Zuber et al., 2008). The mycolic acids are alpha-alkyl, beta-hydroxy C₆₀₋₉₀ fatty acids. The distinct composition of the mycolic acids is dependent on the *Mycobacterium species* including short saturated alpha, C₂₀₋₂₅, and a longer meromycolate chain, the beta-hydroxy branch C₆₀, comprising doublebonds, cyclopropane rings and oxygenated groups. The outer membrane is linked with esterification to the terminal pentaarabinofuranosyl components of arabinogalactan (Payne et al., Molecular Microbiology, 73(3), 2009). The arabinogalactan is covalently linked to peptidoglycan. This covalently linked complex is known as mycolyl-arabinogalactan peptidoglycan (mAGP). This mAGP is known as the cell wall core and builds a stable scaffolding to anchor the outer non-covalently associated lipid and glycoplipids including trehalose 6,6'-dimycolate (TDM or cord factor) (Gil et al., Microbiology, 156, 2010). TDM is a secreted molecule which is important for the pathogenesis of mycobacteria (Brennan, 2003). The cell surface of mycobacteria has the characteristics of a highly hydrophobicity and fastness in view of acids due to the special structure of mAGP and TDM in combination with trehalose 6'-monomycolate. These special properties are leading to the fact that mycobacteria are resistant to dehydration and posses a natural impermeability to nutrients and antibacterial drugs (Gil et al., Microbiology, 156, 2010).

*Mycobacterium tuberculosis* is the cause for the tuberculosis, an infectious disease which typically affects the lungs. Tuberculosis is a health and life threatening disease. In 2009, 9.4 million new cases of tuberculosis and 1.7 million deaths are counted (Global Tuberculosis Control WHO Report 2010. World Health Organization; Geneva: 2010). *Mycobacterium tuberculosis* is spread as a primarily respiratory pathogen. Patients with an active infection can transmit the infection by coughing. A major part of infected human patients are not able to eliminate the bacteria completely. This results in the so called "latent" stage, defining a status, wherein the patient is still infected, but does not show any symptoms of the disease. This latent stage however can change in some patients due to a reactivation of the infection resulting in an active stage of tuberculosis. Typically, an infection with *mycobacterium tuberculosis* starts with the inhalation of the bacteria, followed by the presentation by antigen-presenting immune cells, such as macrophages or dendritic cells, in the airway. Infected macrophages include mycobacteria in intracellular vesicles. However, these vesicles are not accessible for a fusion with lysosomes, which would result in a killing of the mycobacteria. After activation of the infected macrophages with a specific T_{H}1-cell, a lysosomal fusion occurs. Further to this first infection step, infected macrophages recruit uninfected macrophages. Thereby a so called granuloma is formed. The structure of such a granuloma, which is also called caseous granuloma because of the "cheese-like" look, comprises macrophages surrounding a necrotic area with adjacent of B and T cells.

Mycobacteria in general can be classified into several major groups for purpose of diagnosis and treatment: *M. tuberculosis* complex, which can cause tuberculosis: *M. tuberculosis, M. bovis, M. africanum, and M. microti; M. leprae,* which causes Hansen's disease or leprosy; Nontuberculous mycobacteria (NTM) define all the other mycobacteria, which can cause pulmonary disease resembling tuberculosis, lymphadenitis, skin disease, or disseminated disease. Mycobacteria not only cause human infections but also animal infections as well, e.g. *Mycobacterium avium, Mycobacterium avium subsp. paratuberculosis, Mycobacerium bovis.*

Mycobacteriophages are a subgroup of bacteriophages, which are bacterial viruses, which target mycobacterial hosts. In view of the special structure and composition of the cell wall of mycobacteria, it is necessary for the mycobacteriophages to degrade the peptidoglycan layer and further to lyse the mycolic acid-rich outer membrane attached to the mAGP complex.

Various types of agents having bactericidal or bacteriostatic activity are known, e.g. antibiotics, endolysins, antimicrobial peptides and defensins. Increasingly microbial resistance to antibiotics, however, is creating difficulties in treating more and more infections caused by bacteria.

Endolysins are peptidoglycan hydrolases encoded by bacteriophages (or bacterial viruses). They are synthesized during late gene expression in the lytic cycle of phage multiplication and mediate the release of progeny virions from infected cells through degradation of the bacterial peptidoglycan. They are either ß(1,4)-glycosylases, transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this concept of combating human pathogens with endolysins received interest. A compelling need to develop totally new classes of antibacterial agents emerged and endolysins used as 'enzybiotics' - a hybrid term of 'enzymes' and 'antibiotics' - seem to meet this need. In 2001, Fischetti and coworkers demonstrated for the first time the therapeutic potential of bacteriophage Cl endolysin towards group A streptococci (Nelson et a1., 2001). Since then many publications have established endolysins as an attractive and complementary alternative to control bacterial infections, particularly by Gram positive bacteria. Subsequently different endolysins against other Gram positive pathogens such as *Streptococcus pneumoniae* (Loeffler et al., 2001), *Bacillus anthracis* (Schuch et al., 2002), *S. agalactiae* (Cheng et al., 2005) and *Staphylococcus aureus* (Rashel et al., 2007) have proven their efficacy as enzybiotics.

Distinct endolysins have been identified in mycobacteriophages (Payne and Hatfull, Plos ONE, 7(3), 2012; Payne et al., Mol Microbiol, 73(3), 2009). These particular endolysins are able to break down the mycobacterial cell wall characterized by the mycol-rich mycobacterial outer membrane attached to an arabinogalactan layer which is in turn linked to the peptidoglycan. These particular phage endolysins can be assigned to two groups, (i) enzymes that cleave the peptidoglycan, and (ii) enzymes that cleave the mycolic acid and arabinogalactan layer.

Antimicrobial peptides (AMPs) represent an important component of the innate immunity against infections against bacteria. Several antimicrobial peptides have been identified which possess an effect against mycobacteria. These antimicrobial peptides are involved not only in the killing of mycobacteria but also in the modulation of the immune defense in form of the secretion of cytokines and chemokines (Shin and Jo, Immune Network, 11(5), 2011).

Antimicrobial peptides (AMPs) represent a wide range of short, cationic or amphipathic, gene encoded peptide antibiotics that can be found in virtually every organism. Different AMPs display different properties, and many peptides in this class are being intensively researched not only as antibiotics, but also as templates for cell penetrating peptides. Despite sharing a few common features (e.g., cationicity, amphipathicity and short size), AMP sequences vary greatly, and at least four structural groups (α-helical, β-sheet, extended and looped) have been proposed to accommodate the diversity of the observed AMP conformations. Likewise, several modes of action as antibiotics have been proposed, and it was shown e.g. that the primary target of many of these peptides is the cell membrane whereas for other peptides the primary target is cytoplasmic invasion and disruption of core metabolic functions. AMPs may become concentrated enough to exhibit cooperative activity despite the absence of specific target binding; for example, by forming a pore in the membrane, as is the case for most AMPs. However, this phenomenon has only been observed in model phospholipid bilayers, and in some cases, AMP concentrations in the membrane that were as high as one peptide molecule per six phospholipid molecules were required for these events to occur. These concentrations are close to, if not at, full membrane saturation. As the minimum inhibitory concentration (MIC) for AMPs are typically in the low micromolar range, scepticism has understandably arisen regarding the relevance of these thresholds and their importance *in vivo* (Melo et al., Nature reviews, Microbiology, 2009, 245).

Cathelicidins are a family of AMPs which are derived from leukocytes and epithelial cells. Currently, the only identified human cathelicidin is hCAP-18/LL-37. Immunstimulatory effects have been reported for cathelicidins (Shin and Jo, Immune Network, 11(5), 2011).

Defensins are a large family of small, cationic or amphipathic, cysteine- and arginine-rich antimicrobial peptides, found in both vertebrates and invertebrates. Defensins are divided into five groups according to the spacing pattern of cysteines: plant, invertebrate, α-, β-, and θ-defensins. The latter three are mostly found in mammals. α -defensins are proteins found in neutrophils and intestinal epithelia. β-defensins are the most widely distributed and are secreted by leukocytes and epithelial cells of many kinds. θ-defensins have been rarely found so far e.g. in leukocytes of rhesus macaques. Defensins are active against bacteria, fungi and many enveloped and nonenveloped viruses. However, the concentrations needed for efficient killing of bacteria are mostly high, i.e. in the µ-molar range. Activity of many peptides may be limited in presence of physiological salt conditions, divalent cations and serum. Depending on the content of hydrophobic amino acid residues defensins also show haemolytic activity.

Hepcidin is a cationic amphipathic bactericidal peptide which is primarily produced in the liver. The expression of Hepcidin is induced during infectious and inflammatory conditions. Crucially, Hepcidin is expressed in macrophages after infection with intracellular pathogens *Mycobacterium avium* and *Mycobacterium tuberculosis.* Further, hepcidin causes damage to *Mycobacterium tuberculosis* and thus exerts immediate antimycobacterial activity (Shin and Jo, Immune Network, 11(5), 2011).

Mycobacteria with its special structure of the cell wall and the infection procedure which results in the intracellular survival of the mycobacteria within macrophages represent challenges for an effective treatment and therapy of mycobacterial infections. There are currently difficulties existing to target mycobacteria which are residing and replicating intracellularly, e.g. mycobacteria which are surviving within infected host cells, such as macrophages.

Thus, there is a need for new antimycobacterial agents.

This object is solved by the subject-matter defined in the claims.

The term "protein" as used herein refers to a linear polymer of amino acid residues linked by peptide bonds in a specific sequence. The amino-acid residues of a protein may be modified by e.g. covalent attachments of various groups such as carbohydrates and phosphate. Other substances may be more loosely associated with the protein, such as heme or lipid, giving rise to the conjugated proteins which are also comprised by the term "protein" as used herein. The protein may be folded in different ways. The various ways in which the protein fold have been elucidated, are in particular with regard to the presence of alpha helices and beta-pleated sheets. The term "protein" as used herein refers to all four classes of proteins being all-alpha, all-beta, alpha/beta and alpha plus beta. Moreover, the term "protein" refers to a complex, wherein the complex refers to a homomer.

The term "fusion protein" as used herein refers to an expression product resulting from the fusion of different nucleic acid sequences. Such a protein may be produced, e.g., in recombinant DNA expression systems. Moreover, the term "fusion protein" as used herein refers to a fusion of a first amino acid sequence having an enzymatic activity, e.g. an endolysin, with a second and a third amino acid sequence. The second amino acid sequence is preferably a peptide stretch, in particular selected from the group consisting of cationic, polycationic, hydrophobic, amphipathic peptides, and antimicrobial peptides. A third amino acid sequence is a protein transduction domain. Preferably, said second and third amino acid sequence is foreign to and not substantially homologous with any domain of the first amino acid sequence. Moreover, the fusion proteins of the present invention also refer to an expression product resulting from the fusion of at least three nucleic acid sequences.

The term "peptide stretch" as used herein refers to any kind of peptide linked to a protein such as an endolysin. In particular the term "peptide stretch" as used herein refers to a peptide stretch selected from the group consisting of cationic, polycationic, hydrophobic, amphipathic peptides, and antimicrobial peptides (AMP), in particular synthetic amphipathic peptide, synthetic cationic peptide, synthetic polycationic peptide, synthetic hydrophobic peptide, synthetic antimicrobial peptide (AMP) or naturally occurring AMP. In the context of the present invention, AMP are understood as peptides, which provide antimycobacterial activity.

However, a peptide stretch in the meaning of the present invention does not refer to His-tags, preferably His₅-tags, His₆-tags, His₇-tags, His₈-tags, His₉-tags, His₁₀-tags, His₁₁-tags, His₁₂-tags, His₁₆-tags and His₂₀-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "peptide stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His₆-tag may, depending on the respective pH, also be positively charged, but is used as affinity purification tool as it binds to immobilized divalent cations and is not used as a peptide stretch according to the present invention.

The term "peptide" as used herein refers to short polypeptides consisting of from about 2 to about 100 amino acid residues, more preferably from about 4 to about 50 amino acid residues, more preferably from about 5 to about 30 amino acid residues, wherein the amino group of one amino acid residue is linked to the carboxyl group of another amino acid residue by a peptide bond. A peptide may have a specific function. A peptide can be a naturally occurring peptide or a synthetically designed and produced peptide. The peptide can be, for example, derived or removed from a native protein by enzymatic or chemical cleavage, or can be prepared using conventional peptide synthesis techniques (e.g., solid phase synthesis) or molecular biology techniques (see Sambrook, J. et al., Molecular Cloning: A Laboratory Manual, Cold Spring Harbor Press, Cold Spring Harbor, N.Y. (1989)). Preferred naturally occurring peptides are e.g. antimicrobial peptides and defensins. Preferred synthetically produced peptides are e.g. polycationic, amphipathic or hydrophobic peptides. A peptide in the meaning of the present invention does not refer to His-tags, Strep-tags, thioredoxin or maltose binding proteins (MBP) or the like, which are used to purify or locate proteins.

The term "enzymatic activity" as used herein refers to the effect exerted by one or more enzyme(s) or enzyme like substance(s). An enzymatic activity refers in particular to the effects which are exerted by endolysins. The term "enzymatic activity" refers further in particular to the effect of distinct group of enzyme or enzymatic substances which are having the activity of degrading the cell wall of a *Mycobacterium species.* A group of these enzymes with this distinct characteristics are named as Lysin A (LysA), of the peptidoglycan-cleavage group, which are known or are proposed to cleave (Payne and Hatfull, Plos ONE, 7(3), 2012; Payne et al., Mol Microbiol, 73(3), 2009):
- N-acetyl-b-D-muramidase (lysozyme, lytic transglycosylase);
- N-acetyl-b-D-glucosaminidase;
- N-acetylmuramoyl-L-alanine amidase;
- L-alanoyl-D-glutamate (LD) endopeptidase;
- c-D-glutamyl-meso-diaminopimelic acid (DL) peptidase;
- D-Ala-m-DAP (DD) endopeptidase; and
- m-DAP-m-DAP (LD) endopeptidase.

A further group of enzymes are named as Lysin B (LysB). These enzymes hydrolyze the linkage of the mycolic acids to the peptidoglycan-arabinogalactan complex and comprise at least the following or other lipolytic activities:
- Esterase (mycolarabinogalactanesterase)
- Cutinase
- α/β hydrolase

LysB like proteins are described e.g. in Mycobacteriophage Lysin B is a novel mycolylarabinogalactan esterase Kimberly Payne, Qingan Sun, James Sacchettini, Graham F. Hatfull Mol Microbiol. 2009 August; 73(3): 367-381*;* Mycobacteriophage Ms6 LysB specifically targets the outer membrane of Mycobacterium smegmatis Filipa Gil, Anna E. Grzegorzewicz, Maria João Catalão, João Vital, Michael R. McNeil, Madalena Pimentel Microbiology. 2010 May; 156(Pt 5): 1497-1504*.*

A person skilled in the art is able to identify an enzymatic activity as mentioned above with applying a suitable test setting for the distinct enzyme or enzymatic activity.

The term "endolysin" as used herein refers to an enzyme which is a peptidoglycan hydrolase naturally encoded by bacteriophages or bacterial viruses and which is suitable to hydrolyse bacterial cell walls. According to the present invention "endolysins" may derive from mycobacteriophages. Thus, "endolysins" are in particular enzymes such as Lysin A, LysA, or Lysin A like enzymes or Lysin B, LysB, or Lys B like enzymes. "Endolysins" comprise at least one "enzymatically active domain" (EAD) having at least one or more of the following activities: N-acetyl-b-D-muramidase (lysozyme, lytic transglycosylase), N-acetyl-b-D-glucosaminidase, N-acetyl-muramoyl-L-alanine-amidase (amidase) peptidase, L-alanoyl-D-glutamate (LD) endopeptidase, L-alanyl-D-iso-glutaminyl-meso-diaminopimelic acid (D-Ala-m-DAP) (DD) endopeptidase, or m-DAP-m-DAP (LD) endopeptidase. Furthermore, the EAD is having at least one or more of the following activities: lipolytic activity, cutinase, mycolarabinogalactanesterase, or alpha/beta hydrolase. In addition, the endolysins may contain also regions which are enzymatically inactive, and bind to the cell wall of the host bacteria, the so-called CBDs (cell wall binding domains). The endolysin may contain two or more CBDs. Generally, the cell wall binding domain is able to bind different components on the surface of bacteria. Preferably, the cell wall binding domain is a peptidoglycan binding domain and binds to the bacteria's peptidoglycan structure. The different domains of an endolysin can be connected by a domain linker.

The term "domain linker" as used herein refers to an amino acid sequence functioning to connect single protein domains with one another. As a rule domain linkers form no or only few regular secondary structure like α-helices or ß-sheets and can occupy different conformations with the respective structural context. Methods to detect domain linker and properties of linker sequences are well known in the art as e.g. described in Bae et al., 2005, Bioinformatics, 21, 2264-2270 or George & Heringa, 2003, Protein Engineering, 15, 871-879.

The term "protein transduction domain" (PTD) or the term "cell penetrating peptides" (CPP) refers to an amino acid sequence functioning to deliver a cargo from the extracellular to the intracellular space of a cell. This transportation involves a three step process, including first, binding of the PTD to the cellular membrane; second, stimulation of cellular uptake by endocytosis; and third, escape of cargo into the cytoplasm (Van den Berg and Dowdy, Current Opinion in Biotechnology, 22, 2011). PTDs may be cationic. The term "cargo" in the context of the present invention refers to a substance which is transported from the outside to the inside of a cell. The term "cargo" in the context of the present invention refers in particular to peptides, such as AMPs, enzymes, such as endolysins, dyes, such as fluorescent dyes like fluorescein. A person skilled in the art is able to identify amino acid sequences which are PTDs e.g. in form of an experimental setting wherein it is foreseen to use an amino acid sequence which is supposed to be a PTD together with a dye, such as a fluorescent dye, as a cargo. Using e.g. fluorescent microscopy or Fluorescent-activated cell sorting (FACS) analysis, allows assessing whether the putative PTD is able to deliver the fluorescent dye into the inside of a cell or not. PTD in combination with fluorescein as cargo are describe in Van den Berg and Dowdy, Curr Opin Biotech, 22, 2011 and in more detail in Vives at el., J Biol Chem, 272, 1997.

The term "deletion" as used herein refers to the removal of 1, 2, 3, 4, 5 or more amino acid residues from the respective starting sequence.

The term "insertion" or "addition" as used herein refers to the insertion or addition of 1, 2, 3, 4, 5 or more amino acid residues to the respective starting sequence.

The term "substitution" as used herein refers to the exchange of an amino acid residue located at a certain position for a different one.

The term "cell wall" as used herein refers to all components that form the outer cell enclosure in particular of a Mycobacterium and thus guarantee their integrity. In particular, the term "cell wall" as used herein refers to in particular to the arabinogalactan layer an the mycolic acid layer of Mycobacteria, but also to membranes or additional layers deposited or attached to the mycolic acid layer, such as capsule-like material, outer protein layer or slimes.

The term "EAD" as used herein refers to the enzymatically active domain of an endolysin. The EAD is responsible for hydrolysing bacterial peptidoglycans. It exhibits at least one enzymatic activity of an endolysin. The EAD can also be composed of more than one enzymatically active module. The term "EAD" is used herein synonymously with the term "catalytic domain".

As used herein, the term "cationic peptide" refers to a synthetic peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides.

The term "polycationic peptide" as used herein refers to a synthetically produced peptide composed of mostly positively charged amino acid residues, in particular lysine and/or arginine residues. A peptide is composed of mostly positively charged amino acid residues of at least about 20, 30, 40, 50, 60, 70, 75, 80, 85, 90, 95 or about 100% of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term "antimicrobial peptide" (AMP) as used herein refers to any naturally occurring peptide that has microbicidal and/or microbistatic activity in particular against a *Mycobacterium species.* Thus, the term "antimicrobial peptide" as used herein relates in particular to any peptide having anti-bacterial, anti-infectious, anti-infective and/or germicidal, microbicidal, or bactericidal properties.

The antimicrobial peptide may be a member of the RNAse A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphipathic peptides may be e.g. alpha helical, having predominantly non polar side chains along one side of the helix and polar residues along the remainder of its surface.

The term "hydrophobic group" as used herein refers to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a nonaqueous environment. Examples of amino acid residues with hydrophobic side chains are valine, isoleucine, leucine, methionine, phenylalanine, tryptophan, cysteine, alanine, tyrosine, histidine, threonin, serine, proline and glycine residues.

The term "autolysins" refers to enzymes related to endolysins but encoded by bacteria and involved in e.g. cell division. An overview of autolysins is can be found in "Bacterial peptidoglycan (murein) hydrolases. Vollmer W, Joris B, Charlier P, Foster S. FEMS Microbiol Rev. 2008 Mar;32(2):259-86".

The term "bacteriocin" as used herein refers to protein-like, polypeptide-like or peptide-like substances which are able to inhibit the growth of other bacteria. Some bacteriocins are capable of degrading bacterial cell walls like Lysostaphin (degrading *Staphylococcus* cell walls), Mutanolysin (degrading *Streptococcus* cell walls) and Enterolysin (degrading *Enterococcus* cell walls). Preferably said growth inhibition is specifically by means of absorption of said other bacteria to specific receptors of the bacteriocin. A further group of bacteriocins are Nisin-like peptides (Gene encoded antimicrobial peptides, a template for the design of novel anti-mycobacterial drugs. Carroll J, Field D, O'Connor PM, Cotter PD, Coffey A, Hill C, Ross RP, O'Mahony J. Bioeng Bugs. 2010 Nov-Dec;1(6):408-12). In general, bacteriocins are produced by microorganisms. However, the term "bacteriocin" as used herein refers both to an isolated form procuded by a microorganism or to a synthetically produced form, and refers also to variants which substantially retain the activities of their parent bacteriocins, but whose sequences have been altered by insertion or deletion of one or more amino acid residues.

The present invention relates to new antibacterial agents against *Mycobacterium species,* in particular to a composition having the activity of degrading the cell wall of a *Mycobacterium species* comprising: (a) a first fusion protein including (i) a first endolysin or a first domain, both having a first enzymatic activity, the enzymatic activity being at least one or more of the following: N-acetyl-b-D-muramidase (lysozyme, lytic transglycosylase), N-acetyl-b-D-glucosaminidase, N-acetylmuramoyl-L-alanine amidase, L-alanoyl-D-glutamate (LD) endopeptidase, c-D-glutamyl-meso-diaminopimelic acid (DL) peptidase, D-Ala-m-DAP (DD) endopeptidase, or m-DAP-m-DAP (LD) endopeptidase, (ii) at least one peptide stretch fused to the N- or C-terminus of the endolysin having the first enzymatic activity or the domain having the first enzymatic activity, wherein the peptide stretch is selected from the group consisting of synthetic amphipathic peptide, synthetic cationic peptide, synthetic polycationic peptide, synthetic hydrophobic peptide, synthetic antimicrobial peptide (AMP) or naturally occurring AMP; and (iii) a protein transduction domain (PTD) being at the N- or C-terminus of the first fusion protein, wherein the PTD is having the characteristic to deliver a cargo from the extracellular to the intracellular space of a cell; and (b) a second fusion protein including (i) a second endolysin or a second domain, both having a second enzymatic activity, the enzymatic activity being at least one or more of the following: lipolytic activity, cutinase, mycolarabinogalactanesterase, or alpha/beta hydrolase; (ii) at least one peptide stretch fused to the N- or C-terminus of the endolysin having a second enzymatic activity or the domain having the second enzymatic activity, wherein the peptide stretch is selected from the group consisting of synthetic amphipathic peptide, synthetic cationic peptide, synthetic polycationic peptide, synthetic hydrophobic peptide, synthetic antimicrobial peptide (AMP) or naturally occurring AMP; and (iii) a protein transduction domain (PTD) being at the N- or C-terminus of the second fusion protein, wherein the PTD is having the characteristic to deliver a cargo from the extracellular to the intracellular space of a cell.

Accordingly, the present invention relates to a composition comprising two distinct fusion proteins which include distinct enzymatic activities combined with a peptide stretch and a protein transduction domain. This distinct combination of a first and a second fusion protein, wherein the first and the second fusion protein include each a first and a second enzymatic activity, a peptide stretch and the PTD, in a composition according to the present invention is advantageous. The composition according to the present invention provides the distinct advantage that the combination of two fusion proteins exert a strong activity in respect of the degradation of the cell wall of *Mycobacteria species.* Moreover, due to the presence of the PTD in each of the both fusion proteins, the structure of the outer cell membrane can be more easily overcome with the present invention. This is in particular of importance in the case that the mycobacteria are surviving inside of a host cell, such as in a human macrophage. Therefore, the composition of the present invention is able to provoke a strong anti-mycobacterial activity.

In a preferred embodiment of the composition of the present invention the first endolysin is Lysin A (LysA) or the first enzymatic activity of the first domain is exerted by Lysin A (LysA) or Lysin A like enzymes and the second endolysin is Lysin B (LysB) or the second enzymatic activity of the second domain is exerted by Lysin B (LysB) or Lysin B like enzymes.

Examples for the first and second endolysins or the first and second enzymatic activity of the first and second domain are listed in the following table 1.

**Table 1:**

| **Mycobacteriophage** | **endolysin/ domain** | **type of endolysin or domain** | **amino acid sequence** | **nucleic acid sequence** |
|---|---|---|---|---|
| TM4 | TM4gp29 | Lysin A | SEQ ID NO:1 | SEQ ID NO:2 |
| Bxz2 | Bxz2gp11 | Lysin A | SEQ ID NO:3 | SEQ ID NO:4 |
| D29 | D29gp10 | Lysin A | SEQ ID NO:5 | SEQ ID NO:6 |
| L5 | L5gp10 | Lysin A | SEQ ID NO:7 | SEQ ID NO:8 |
| TM4 | TM4gp30 | Lysin B | SEQ ID NO:9 | SEQ ID NO:10 |
| Bxz2 | Bxz2gp 12 | Lysin B | SEQ ID NO:11 | SEQ ID NO:12 |
| D29 | D29gp12 | Lysin B | SEQ ID NO:13 | SEQ ID NO:14 |
| L5 | L5gp12 | Lysin B | SEQ ID NO:15 | SEQ ID NO:16 |

In a preferred embodiment the first and the second enzymatic activity of the first and second fusion protein of the composition is exerted by enzymes derived from mycobacteriophages selected from the group consisting of TM4, D29, L5, and Bxz2.

In a further preferred embodiment the peptide stretch of the first and second fusion protein of the composition of the present invention comprises an antimicrobial peptide (AMP), the AMP being selected from the group consisting of Cathelicidins (hCAP-18/LL37), alpha defensins, beta defensins, hepcidin, NK-2, and Ci-MAM-A24.

Examples for antimicrobial peptides according to the present invention are listed in the following table 2.

**Table 2:**

| **Peptid** | **amino acid sequence** | **nucleic acid sequence** |
|---|---|---|
| | LLGDFFRKSKEKIGKEFKRIVQRIKDFLRNLVP RTES | SEQ ID NO:18 |
| LL-37 | SEQ ID NO:17 | |
| | DCYCRIPACIAGERRYGTCIYQGRLWAFCC | SEQ ID NO:20 |
| alpha-defensin | SEQ ID NO:19 | |
| | NPVSCVRNKGICVPIRCPGSMKQIGTCVGRAV KCCRKK | SEQ ID NO:22 |
| beta-defensin | SEQ ID NO:21 | |
| | DTHFPICIFCCGCCHRSKCGMCCKT | SEQ ID NO:24 |
| Hepcidin | SEQ ID NO:23 | |
| | KILRGVCKKIMRTFLRRISKDILTGKK; | SEQ ID NO:26 |
| NK-2 | SEQ ID NO:25 | |
| | WRSLGRTLLRLSHALKPLARRSGW | SEQ ID NO:28 |
| Ci-MAM-A24 | SEQ ID NO:27 | |

In a preferred embodiment the PTD of the first and second fusion protein of the composition of the present invention is selected from the group consisting of TAT48-60, TAT47-57, TAT47-55, PTD3, PolyArginine, CADY, PepFect6, RXR, Antennapedia, Kala Syn, M918, MAP, Penetratin, PTD5-Syn, Pvec, Poly Arg 8, TAT 48-60, Transportan, Transportan10, and TAT-9.

Examples for PTDs according to the present invention are listed in the following table 3.

**Table 3:**

| **PTD** | **amino acid sequence** | **SEQ ID NO** |
|---|---|---|
| TAT48-60 Ref. 1 | GRKKRRQRRRPPQC | SEQ ID NO:29 |
| TAT47-57 Ref. 1 | YGRKKRRQRRR | SEQ ID NO:30 |
| TAT47-55 Ref. 1 | YGRKKRRQR | SEQ ID NO:31 |
| PTD3 Ref. 1 | YARKARRQARR | SEQ ID NO:32 |
| Poly Arginine Ref. 1 | RRRRRRRRR | SEQ ID NO:33 |
| CADY Ref. 1 | GLWRALWRLLRSLWRLLWRA | SEQ ID NO:34 |
| PepFect6 Ref. 1 | AGYLLGKINLKALAALAKKIL | SEQ ID NO:35 |
| RXR Ref. 1 | RXRRXRRXRRXRXB | SEQ ID NO:36 |
| Antennapedia Ref. 2 | RQIKIWFQNRRMKWKK | SEQ ID NO:37 |
| Kala Syn Ref. 3 | WEAKLAKALAKALAKHLAKALAKALKACEA | SEQ ID NO:38 |
| M918 Ref. 4 | MVTVLFRRLRIRRASGPPRVRV | SEQ ID NO:39 |
| MAP Ref. 5 | KLALKLALKALKAALKLA | SEQ ID NO:40 |
| Penetratin Ref. 6 | RQIKIWFQNRRMKWKK | SEQ ID NO:41 |
| PTD5-Syn Ref. 7 | RRQRRTSKLMKR | SEQ ID NO:42 |
| Pvec Ref. 8 | LLIILRRRIRKQAHAHSK | SEQ ID NO:43 |
| Poly Arg 8 Ref. 2 | RRRRRRRR | SEQ ID NO:44 |
| TAT48-60 Ref. 9 | GRKKRRQRRRPPQ | SEQ ID NO:45 |
| Transportan Ref. 10 | GWTLNSAGYLLGKINLKALAALAKKIL | SEQ ID NO:46 |
| Transportan10 Ref. 11 | AGYLLGKINLKALAALAKKIL | SEQ ID NO:47 |
| TAT-9 Ref. 9 | RKKRRQRRR | SEQ ID NO:48 |

The PTDs are disclosed in the following references:
1. Van den Berg and Dowdy, Curr Opin Biotech, 22, 2011. 2. Kabouridis, P.S. Biological applications of protein transduction technology. Trends Biotechnol. 21, 498-503 (2003).
3. Min, S.H. et al. Gene delivery using a derivative of the protein transduction domain peptide, K-Antp. Biomaterials. 31, 1858-1864 (2010). 4. El-Andaloussi, S., Johansson, H.J., Holm, T., & Langel, U. A novel cell-penetrating peptide, M918, for efficient delivery of proteins and peptide nucleic acids. Mol. Ther. 15, 1820-1826 (2007). 5. Saar, K. et al. Cell-penetrating peptides: a comparative membrane toxicity study. Anal. Biochem. 345, 55-65 (2005). 6. Splith, K. & Neundorf, I. Antimicrobial peptides with cell-penetrating peptide properties and vice versa. Eur. Biophys. J.(2011). 7. Mi, Z. et al. Identification of a synovial fibroblast-specific protein transduction domain for delivery of apoptotic agents to hyperplastic synovium. Mol. Ther. 8, 295-305 (2003). 8. Herbig, M.E. et al. Bilayer interaction and localization of cell penetrating peptides with model membranes: a comparative study of a human calcitonin (hCT)-derived peptide with pVEC and pAntp(43-58). Biochim. Biophys. Acta. 1712, 197-211 (2005). 9. Eguchi, A. et al. Protein transduction domain of HIV-1 Tat protein promotes efficient delivery of DNA into mammalian cells. J. Biol. Chem. 276, 26204-26210 (2001). 10. Jones, S.W. et al. Characterisation of cell-penetrating peptide-mediated peptide delivery. Br. J. Pharmacol. 145, 1093-1102 (2005). 11. Fisher, L. et al. Cellular delivery of a double-stranded oligonucleotide NFkappaB decoy by hybridization to complementary PNA linked to a cell-penetrating peptide. Gene Ther. 11, 1264-1272 (2004).

In a further preferred embodiment the composition of the present invention is having activity of degrading the cell wall of a *Mycobacterium species* which is selected from the group consisting of *Mycobacterium tuberculosis, Mycobacterium microti, Mycobacterium africanum, Mycobacterium bovis, Mycobacterium canettii, Mycobacterium pinnipedii, Mycobacterium caprae, Mycobacterium mungi, Mycobacterium leprae, Mycobacterium ulcerans, Mycobacterium xenopi, Mycobacterium shottsii, Mycobacterium avium, Mycobacterium avium subsp. paratuberculosis, Mycobacterium paratuberculosis, Mycobacterium intracellulare, Mycobacterium smegmatis, Mycobacterium kansasii, Mycobacterium terrae, Mycobacterium nonchromogenicum, Mycobacterium gordonae, and Mycobacterium triviale.*

In a preferred embodiment of the composition of the present invention, the domain with the first enzymatic activity of the first fusion protein exhibits an amino acid sequence selected from the group consisting of SEQ ID NO:1, 3, 5, and 7, wherein the domain with the second enzymatic activity of the second fusion protein exhibits an amino acid sequence selected from the group consisting SEQ ID NO:9, 11, 13, and 15, wherein the peptide stretch of the first and second fusion protein exhibits an amino acid sequence selected from the group consisting SEQ ID NO:17, 19, 21, 23, 25, and 27, and wherein the PTD exhibits an amino acid sequence selected from the group consisting SEQ ID NO:29, 30, 31, 32, 33, 34, 35, 36, 37, 38, 39, 40, 41, 42, 43, 44, 45, 46, 47, and 48.

In a particular preferred embodiment of the composition of the present invention, the first fusion protein is exhibiting an amino acid sequence selected from the group consisting SEQ ID NO:49, 51, 53, 55, 57, 59, and 61, and the second fusion protein is exhibiting an amino acid sequence selected from the group consisting SEQ ID NO:63, 65, 67, 69, 71, 73, and 75.

Specific examples of fusion proteins according to the present invention are listed in the following table.

**Table 4:**

| **First fusion protein** | **amino acid sequence** | **nucleic acid sequence** |
|---|---|---|
| TM4gp29/LL-37/TAT47-57 | SEQ ID NO:49 | SEQ ID NO:50 |
| TM4gp29/LL-37/TAT47-57 | SEQ ID NO:51 | SEQ ID NO:52 |
| Bzx2gp 11/alpha-defensin/PTD3 | SEQ ID NO:53 | SEQ ID NO:54 |
| PTD3/alpha-defensin/Bzx2gp11 | SEQ ID NO:55 | SEQ ID NO:56 |
| PTD3/alpha-defensin/Bzx2gp 11/alpha-defensin | SEQ ID NO:57 | SEQ ID NO:58 |
| beta-defensin/L5gp10/TAT47-57 | SEQ ID NO:59 | SEQ ID NO:60 |
| alpha-defensin/Hepcidin/L5gp10/TAT47-57 | SEQ ID NO:61 | SEQ ID NO:62 |

| **Second fusion protein** | **amino acid sequence** | **nucleic acid sequence** |
|---|---|---|
| TM4gp30/LL-37/TAT47-57 | SEQ ID NO:63 | SEQ ID NO:64 |
| TM4gp30/LL-37/TAT47-57 | SEQ ID NO:65 | SEQ ID NO:66 |
| D29gp 12/alpha-defensin/PTD3 | SEQ ID NO:67 | SEQ ID NO:68 |
| PTD3/alpha-defensin/D29gp 12 | SEQ ID NO:69 | SEQ ID NO:70 |
| PTD3/alpha-defensin/D29gp 12/alpha-defensin | SEQ ID NO:71 | SEQ ID NO:72 |
| beta-defensin/D29gp12/TAT47-57 | SEQ ID NO:73 | SEQ ID NO:74 |
| beta-defensin/Hepcidin/L5gp12/TAT47-57 | SEQ ID NO:75 | SEQ ID NO:76 |

In another preferred embodiment of the present invention the enzymes, such as endolysins, autolysins and bacteriocins of the first and second fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, pegylation, chemical changes of the amino-, SH- or carboxyl- groups. Said endolysins, autolysins and bacteriocins of the fusion protein according to the present invention exhibit the lytic activity of the respective wild-type endolysin, autolysin and bacteriocin. However, said activity can be the same, higher or lower as the activity of the respective wild-type endolysin, autolysin and bacteriocin. Said activity can be about 10, 20, 30, 40, 50, 60, 70, 80, 90, 100, 110, 120, 130, 140, 150, 160, 170, 180, 190 or about 200% of the activity of the respective wild-type endolysin, autolysin and bacteriocin or even more. The activity can be measured by assays well known in the art by a person skilled in the art as e.g. the plate lysis assay or the liquid lysis assay which are e.g. described in Briers et al., J. Biochem. Biophys Methods 70: 531-533, (2007*),* Donovan DM, Lardeo M, Foster-Frey J. FEMS Microbiol Lett. 2006 Dec;265(1*),* Payne KM, Hatfull GF PLoS One, 2012*.*

The peptide stretch and the PTD of the fusion proteins according to the present invention may be linked to the endolysin or the domain having an enzymatic activity by additional amino acid residues e.g. due to cloning reasons. Preferably, said additional amino acid residues may be not recognized and/or cleaved by proteases. Preferably the peptide stretch and the PTD may be linked to the endolysin or the domain having an enzymatic activity by at least 1, 2, 3, 4, 5, 6, 7, 8, 9 or 10 additional amino acid residues. In a preferred embodiment the peptide stretch is fused to the N- or C-terminus of the endolysin or the domain having an enzymatic activity by the additional amino acid residues glycine, serine, and alanine (Gly-Ser-Ala). Moreover, the PTD is located on the N-terminus or on the C-Terminus of the first fusion protein or of the second fusion protein according to the invention.

The PTD may further comprise additional amino acids on its N- or C-terminus. Preferably the peptide stretch or the PTD comprise the amino acid methionine (Met), or methionine, glycine and serine (Met-Gly-Ser). In another preferred embodiment the first peptide stretch is linked to the N-terminus of the enzyme by the additional amino acid residues, in particular glycine and serine (Gly-Ser) and the second peptide stretch is linked to the N-terminus of the first peptide stretch by the additional amino acid residues, in particular glycine and serine (Gly-Ser). In another preferred embodiment the first peptide stretch is linked to the C-terminus of the enzyme by the additional amino acid residues, in particular glycine and serine (Gly-Ser) and the second peptide stretch is linked to the C-terminus of the first peptide stretch by the additional amino acid residues, in particular glycine and serine (Gly-Ser).

Within the first and second fusion protein according to the present invention the peptide stretch and the PTD are preferably covalently bound to the endolysin or to the domain, both having enzymatic activity. Preferably, the peptide stretch and the PTD consist of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 amino acid residues. Especially preferred are peptide stretches and PTDs comprising about 5 to about 100 amino acid residues, about 5 to about 50 or about 5 to about 30 amino acid residues. More preferred are peptide stretches and PTDs comprising about 6 to about 42 amino acid residues, about 6 to about 39 amino acid residues, about 6 to about 38 amino acid residues, about 6 to about 31 amino acid residues, about 6 to about 25 amino acid residues, about 6 to about 24 amino acid residues, about 6 to about 22 amino acid residues, about 6 to about 21 amino acid residues, about 6 to about 20 amino acid residues, about 6 to about 19 amino acid residues, about 6 to about 16 amino acid residues, about 6 to about 14 amino acid residues, about 6 to about 12 amino acid residues, about 6 to about 10 amino acid residues or about 6 to about 9 amino acid residues.

Preferably, the peptide stretches and the PTDs are no tag such as a His-tag, Strep-tag, Avi-tag, Myc-tag, Gst-tag, JS-tag, cystein-tag, FLAG-tag or other tags known in the art and no thioredoxin or maltose binding proteins (MBP). However, the first and second fusion protein according to the present invention may comprise in addition such tag or tags.

More preferably the peptide stretches and in particular the PTDs have the function to lead the first and second fusion protein of the composition of the present invention through the outer membrane but may have activity or may have no or only low activity when administered without being fused to the endolysin or the domain, both having enzymatic activity. The function to lead the first and second fusion protein through the outer membrane of mycobacteria is caused by the potential of the outer membrane or mycolic acid/arabinogalactan or LPS disrupting or permeabilising or destabilizing activity of said peptide stretches in combination with the PTDs and the endolysins or the domains. Such outer membrane or LPS disrupting or permeabilising or destabilizing activity of the peptide stretches may be determined in a method as follows: The bacteria cells to be treated are cultured in liquid medium or on agar plates. Then the bacteria cell concentration in the liquid medium is determined photometrically at OD600 nm or the colonies on the agar plates are counted, respectively. Now, the bacteria cells in liquid medium or on the plates are treated with a first and second fusion protein according to the invention. After incubation the bacteria cell concentration in the liquid medium is determined photometrically at OD600 nm or the colonies on the agar plates are counted again. If the first and second fusion protein exhibits such outer membrane or LPS disrupting or permeabilising or destabilizing activity, the bacteria cells are lysed due to the treatment with the fusion protein and thus, the bacteria cell concentration in the liquid medium or the number of the bacteria colonies on the agar plate is reduced. Thus, the reduction in bacteria cell concentration or in the number of bacteria colonies after treatment with the first and second fusion protein is indicative for an outer membrane or LPS disrupting or permeabilising or destabilizing activity of the first and second fusion protein.

Fusion proteins are constructed by linking at least three nucleic acid sequences using standard cloning techniques as described e.g. by Sambrook et al. 2001, Molecular Cloning: A Laboratory Manual. Such a protein may be produced, e.g., in recombinant DNA expression systems. Such fusion proteins according to the present invention can be obtained by fusing the nucleic acids for endolysin and the respective peptide stretches.

A further subject-matter of the present invention relates to an isolated nucleic acid molecule encoding the first fusion protein of the composition of to the present invention or to an isolated nucleic acid molecule encoding the second fusion protein of the composition of the present invention.

Preferably the isolated nucleic acid molecule encoding the first fusion protein of the composition of to the present invention is selected from the group consisting of SEQ ID NO:50, 52, 54, 56, 58, 60 and 62. Preferably the isolated nucleic acid molecule encoding the second fusion protein of the composition of to the present invention is selected from the group consisting of SEQ ID NO:64, 66, 68, 70, 72, 74, and 76.

The present invention further relates to a vector comprising a nucleic acid molecule according to the present invention. Said vector may provide for the constitutive or inducible expression of said fusion protein according to the present invention.

The invention also relates to a method for obtaining said first and second fusion protein of the composition of the present invention from a micro-organism, such as a genetically modified suitable host cell which expresses said fusion proteins. Said host cell may be a microorganism such as bacteria or yeast or an animal cell as e.g. a mammalian cell, in particular a human cell. In one embodiment of the present invention the host cell is a *Pichia pastoris* cell. The host may be selected due to mere biotechnological reasons, e.g. yield, solubility, costs, etc. but may be also selected from a medical point of view, e.g. a non-pathological bacteria or yeast, human cells.

Another subject-matter of the present invention relates to a method for genetically transforming a suitable host cell in order to obtain the expression of the first and second fusion protein of the composition according to the invention, wherein the host cell is genetically modified by the introduction of a genetic material encoding said fusion proteins into the host cell and obtain their translation and expression by genetic engineering methods well known by a person skilled in the art.

In a further aspect the present invention relates to a pharmaceutical composition, comprising a composition comprising a first and a second fusion protein according to the present invention and/or a host transformed with a nucleic acid molecule or a vector comprising a nucleotide sequence encoding a first fusion protein and a nucleic acid molecule or a vector comprising a nucleotide sequence encoding a second fusion protein of the composition according to the present invention.

The present invention also relates to a composition according to the present invention and/or a host transformed with a nucleic acid comprising a nucleotide sequence encoding a first fusion protein or a nucleic acid molecule encoding a second fusion protein of the composition according to the present invention for use as a medicament.

In a further subject-matter the present invention relates to the use of a composition comprising the first and the second fusion protein according to the present invention and/or a host transformed with a vector comprising the nucleic acid molecule comprising a nucleotide sequence encoding a modified first fusion protein or a nucleotide sequence encoding a modified second fusion protein of the composition according to the present invention in the manufacture of a medicament for the treatment and/or prevention of a disorder, disease or condition associated with a *Mycobacterium species.*

The present invention relates further to a composition comprising of the first and second fusion protein according to the present invention for use as a medicament, a disinfectant, a feed additive, or food additive.

Preferably, a composition comprising the first and second fusion protein according to the present invention is foreseen for use as a medicament, a disinfectant, feed additive, or food additive in the treatment or prevention of diseases caused by an infection with a *Mycobacterium species,* such as tuberculosis, leprosy, pulmonary disease, lymphadenitis or skin disease.

In a further preferred embodiment a composition comprising a first and a second fusion protein of the present invention is foreseen for use as a medicament, a disinfectant, feed additive, or food additive in the treatment or prevention of a Mycobacterial contamination of foodstuff, of food processing equipment, of food processing plants, of surfaces coming into contact with foodstuff, of medical devices, or of surfaces in hospitals and surgeries.

In another preferred embodiment a composition comprising a first and a second fusion protein of the present invention is foreseen for use as a medicament, a disinfectant or food additive, wherein the composition is used in combination with an additional therapeutic agent.

Additional therapeutic agents may be medicaments and pharmaceutical active substances e.g. antibiotics, such as streptomycin, tetracycline, cephalothin, gentamicin, cefotaxime, cephalosporin, ceftazidime, imipenem, isoniazid, ethambutol, or pyrazinamide.

In a further subject-matter of the present invention relates to a method of treating a disorder, disease or condition in a subject in need of treatment and/or prevention, which method comprises administering to said subject an effective amount of a composition comprising a first and a second fusion protein according to the present invention and/or an effective amount of a host transformed with nucleic acids comprising a nucleotide sequences encoding a first and a second fusion protein according to the present invention or a medicament or a pharmaceutical composition comprising the composition of the present invention. The subject may be a human or an animal.

The dosage and route of administration used in a method of treatment or prophylaxis according to the present invention depends on the specific disease/site of infection to be treated. The route of administration may be for example oral, topical, nasopharyngeal, parenteral, intravenous, rectal or any other route of administration.

For application of a composition according to the present invention and/or an effective amount of a host transformed with a nucleic acid comprising a nucleotide sequences encoding a first and second fusion protein according to the present invention or a medicament or a pharmaceutical composition comprising a composition comprising a first and second fusion protein according to the present invention to a site of infection or site endangered to be infected a formulation may be used that protects the active compounds from environmental influences such as proteases, oxidation, immune response etc., until it reaches the site of infection. Therefore, the formulation may be capsule, dragee, pill, powder, suppository, emulsion, suspension, gel, lotion, cream, salve, injectable solution, syrup, spray, inhalant or any other medical reasonable galenic formulation. Preferably, the galenic formulation may comprise suitable carriers, stabilizers, flavourings, buffers or other suitable reagents. For example, for topical application the formulation may be a lotion, cream, gel, salve or plaster, for nasopharyngeal application the formulation may be saline solution to be applied via a spray to the nose. For oral administration in case of the treatment and/or prevention of a specific infection site e.g. in the intestine, it can be necessary to protect a fusion protein according to the present invention from the harsh digestive environment of the gastrointestinal tract until the site of infection is reached. Thus, bacteria as carrier, which survive the initial steps of digestion in the stomach and which secret later on a first and second fusion protein according to the composition of the present invention into the intestinal environment can be used.

In another specific embodiment of the present invention the disorder, disease or condition is caused by a *Mycobacterium species,* in particular *Mycobacterium tuberculosis, Mycobacterium microti, Mycobacterium africanum, Mycobacterium bovis, Mycobacterium canettii, Mycobacterium pinnipedii, Mycobacterium caprae, Mycobacterium mungi, Mycobacterium leprae, Mycobacterium ulcerans, Mycobacterium xenopi, Mycobacterium shottsii, Mycobacterium avium, Mycobacterium avium subsp. paratuberculosis, Mycobacterium paratuberculosis, Mycobacterium intracellulare, Mycobacterium smegmatis, Mycobacterium kansasii, Mycobacterium terrae, Mycobacterium nonchromogenicum, Mycobacterium gordonae, and Mycobacterium triviale,* in particular tuberculosis.

Preferably, a composition comprising a first and second fusion protein according to the present invention is used for medical treatment, if the infection to be treated or prevented is caused by multiresistant bacterial strains, in particular by strains resistant against one or more of the following antibiotics: streptomycin, tetracycline, cephalothin, gentamicin, cefotaxime, cephalosporin, ceftazidime, imipenem, isoniazid, ethambutol or pyrazinamide. Furthermore, a composition according to the present invention can be used in methods of treatment by administering it in combination with conventional antibacterial agents, such as antibiotics, lantibiotics, bacteriocins or endolysins, etc.

The present invention also relates to a pharmaceutical pack comprising one or more compartments, wherein at least one compartment comprises a composition comprising a first and second fusion protein according to the present invention and/or one or more hosts transformed with a nucleic acid comprising a nucleotide sequences encoding a first and second fusion protein according to the present invention or a medicament or a pharmaceutical composition comprising a composition comprising a first and second fusion protein according to the present invention.

In another subject-matter of the present invention relates to a process of preparation of a pharmaceutical composition, said process comprising admixing one or more fusion protein according to the present invention and/or one or more hosts transformed with a nucleic acid comprising a nucleotide sequence encoding a first and second fusion protein according to the composition of the present invention with a pharmaceutically acceptable diluent, excipient or carrier.

In a further subject-matter of the present invention relates to the use of the composition comprising a first and second fusion protein according to the present invention for the treatment, removal, reduction or prevention of mycobacterial contamination of foodstuff, of food processing equipment, of food processing plants, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries.

In a subject-matter of the present invention relates to the use of the composition comprising a first and second fusion protein according to the present invention as a food and feed additive.

Further scope of applicability of the present invention will become apparent from the detailed description given hereinafter, however, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

The following examples explain the present invention but are not considered to be limiting. Unless indicated differently, molecular biological standard methods were used, as e.g., described by Sambrock et al., 1989, Molecular Cloning: A Laboratory Manual, 2nd edition, Cold Spring Harbor Laboratory Press, Cold Spring Harbor, New York.

### EXAMPLE 1: Cloning, expression and purification of the respective proteins modified with various peptide stretches and protein transduction domains at the N-terminus or the C-terminus.

### Proteins

TM4gp29 according to SEQ ID NO:1 is a Lys A-type endolysin originating from *Mycobacteria* phage TM4. The endolysin TM4gp29 is encoded by the nucleic acid molecule according to SEQ ID NO:2. The nucleic acid molecule according to SEQ ID NO:2 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

Bxz2gp11 according to SEQ ID NO:3 is a Lys A-type endolysin originating from *Mycobacteria* phage Bxz2. The endolysin Bxz2gp11 is encoded by the nucleic acid molecule according to SEQ ID NO:4. The nucleic acid molecule according to SEQ ID NO:4 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

D29gp10 according to SEQ ID NO:5 is a Lys A-type endolysin originating from *Mycobacteria* phage D29. The endolysin D29gp10 is encoded by the nucleic acid molecule according to SEQ ID NO:6. The nucleic acid molecule according to SEQ ID NO:6 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

L5gp10 according to SEQ ID NO:7 is a Lys A-type endolysin originating from *Mycobacteria* phage L5. The endolysin L5gp10 is encoded by the nucleic acid molecule according to SEQ ID NO:8. The nucleic acid molecule according to SEQ ID NO: 8 was synthetically produced with a BamH I (5'-GGA TCC-3') restriction site at the 5'-end of the nucleic acid molecule and an Xho I (5'-CTC GAG-3 ') restriction site at the 3'-end of the nucleic acid molecule.

TM4gp30 according to SEQ ID NO: 9 is a Lys B-type endolysin originating from *Mycobacteria* phage TM4. The endolysin TM4gp30 is encoded by the nucleic acid molecule according to SEQ ID NO: 10. The nucleic acid molecule according to SEQ ID NO: 10 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

Bxz2gp12 according to SEQ ID NO: 11 is a Lys B-type endolysin originating from *Mycobacteria* phage Bxz2. The endolysin Bxz2gp12 is encoded by the nucleic acid molecule according to SEQ ID NO: 12. The nucleic acid molecule according to SEQ ID NO: 12 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

D29gp12 according to SEQ ID NO: 13 is a Lys B-type endolysin originating from *Mycobacteria* phage D29. The endolysin D29gp12 is encoded by the nucleic acid molecule according to SEQ ID NO:14. The nucleic acid molecule according to SEQ ID NO: 14 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

L5gp12 according to SEQ ID NO: 15 is a Lys B-type endolysin originating from *Mycobacteria* phage L5. The endolysin L5gp12 is encoded by the nucleic acid molecule according to SEQ ID NO: 16. The nucleic acid molecule according to SEQ ID NO: 16 was synthetically produced with a BamH I (5'-GGA TCC-3') restriction site at the 5'-end of the nucleic acid molecule and an Xho I (5'-CTC GAG-3') restriction site at the 3'-end of the nucleic acid molecule.

The following peptide stretches in table 5 were used for production of fusion proteins with the endolysins above:

**Table 5:**

| **Peptide stretch** | **amino acid sequence** | **nucleic acid sequence** |
|---|---|---|
| LL-37 | SEQ ID NO: 17 | SEQ ID NO: 18 |
| alpha-defensin | SEQ ID NO: 19 | SEQ ID NO: 20 |
| beta-defensin | SEQ ID NO: 21 | SEQ ID NO: 22 |
| Hepcidin | SEQ ID NO: 23 | SEQ ID NO: 24 |

The following protein transduction domains in table 6 were used for production of fusion proteins with the endolysins above:

**Table 6:**

| **PTD** | **amino acid sequence** | **SEQ ID NO** |
|---|---|---|
| TAT47-57 | YGRKKRRQRRR | SEQ ID NO:30 |
| PTD3 | YARKARRQARR | SEQ ID NO:32 |

The nucleic acid molecules encoding the respective peptide stretches and protein transduction domains were synthetically produced with a Nde I (5'-CAT ATG-3') restriction site at the 5'-end of the nucleic acid molecule and a BamH I (5'-GGA TCC-3') restriction site at the 3'-end of the nucleic acid molecule.

Fusion proteins are constructed by linking at least two nucleic acid sequences using standard cloning techniques as described e.g. by Sambrook et al. 2001, Molecular Cloning: A Laboratory Manual. Accordingly the nucleic acid molecules encoding the peptide stretches were cleaved in a restriction digestion with the respective restriction enzymes Nde I and BamH I and in case of the nucleic acid molecule encoding the peptide stretch and PTD for ligation with the proteins the digestion was performed with the restriction enzymes Nco I and BamH I. Subsequently the cleaved nucleic acids encoding the peptide stretches were ligated into the pET21 b expression vector (Novagen, Darmstadt, Germany), which was also cleaved in a digestion with the respective restriction enzymes Nde I and BamH I before. The cleaved nucleic acid molecule encoding the peptide stretch and PTD for ligation with toxic proteins was ligated into a modified pET32 b expression vector (unmodified vector obtainable from Novagen, Darmstadt, Germany), which was also cleaved in a digestion with the respective restriction enzymes Nco I and BamH I before. The modification of the pET32b expression vector refers to the deletion of the sequence encoding an S-tag and the central His-tag.

Afterwards, the nucleic acid molecules encoding the proteins were cleaved in a digestion with the restriction enzyme BamH I and Xho I, so that the proteins could be ligated into the pET21b expression vector (Novagen, Darmstadt, Germany) and the modified pET32 b expression vector, respectively, which were also cleaved in a digest with the respective restriction enzymes BamH I and Xho I before.

In the case of the peptide stretch, which was introduced by PCR to the C-terminus of the proteins, the resulting fusion protein has a His-tag on the N-terminus, wherein the His-tag is linked to the N-terminus by a linker. For the cloning of the respective nucleic acid molecules the pET32 b expression vector (Novagen, Darmstadt, Germany) was used.

Thus, the nucleic acid molecule encoding the peptide stretch is ligated into the respective vector at the 5'-end of the nucleic acid molecule encoding the respective enzyme. Moreover, the nucleic acid molecule encoding the respective enzyme is ligated into the respective plasmid, so that a nucleic acid molecule encoding a His-tag consisting of six histidine residues is associated at the 3'-end of the nucleic acid molecule encoding the endolysin.

As some fusion proteins may either be toxic upon expression in bacteria, or not homogenous due to protein degradation, the strategy might be to express these fusion proteins fused or linked to other additional proteins. Example for these other additional protein is thioredoxin, which was shown to mediate expression of toxic antimicrobial peptides in *E.coli* (TrxA mediating fusion expression of antimicrobial peptide CM4 from multiple joined genes in Escherichia coli. Zhou L, Zhao Z, Li B, Cai Y, Zhang S. Protein Expr Purif. 2009 Apr;64(2):225-230). In the case of the fusion protein consisting of the N-terminal PTD or peptide stretch and the protein, the peptide was ligated into the modified pET32 b expression vector, so that an additional thioredoxin is associated at the 5'-end of the peptide. The thioredoxin could be removed from the expressed fusion protein by the use of enterokinase, therefore between the nucleic acid molecule encoding the peptide and the one encoding the thioredoxin is an enterokinase restriction site introduced.

The sequence of the endolysin-peptide stretch-PTD-fusions was controlled via DNA-sequencing and correct clones were transformed into *E.coli* BL21(DE3) or *E.coli* BL21(DE3) pLysS (Novagen, Darmstadt, Germany) for protein expression.

Recombinant expression of the fusion proteins according to SEQ ID NO: 49, 51, 53, 55, 57, 59, 61, 63, 65, 67, 69, 71, 73, and 75 is performed in *E. coli* BL21 (DE3) cells (Novagen, Darmstadt, Germany). The cells were growing until an optical density of OD600nm of 0.5 to 0.8 was reached. Then the expression of the fusion protein was induced with 1 mM IPTG (isopropylthiogalactoside) and the expression was performed at 37°C for a period of 4 hours, alternatively an overnight expression at 16°C was performed.

*E.coli* BL21 cells were harvested by centrifugation for 20 min at 6000g and disrupted via sonication on ice. Soluble and insoluble fraction of the E.coli crude extract were separated by centrifugation (Sorvall, SS34, 30 min, 15 000 rpm). All proteins were purified by Ni²⁺ affinity chromatography (Akta FPLC, GE Healthcare) using the C-terminal 6xHis-tag, encoded by the pET21b or pET32b vectors.

Toxic proteins were expressed using a modified pET32b vector (S-tag and central His-tag deleted), which fuses thioredoxin on the N-terminus of the proteins of interest. The vector also contains an enterokinase cleavage site right before the protein of interest. This site allows the proteolytic cleavage between thioredoxin and the protein of interest, which can purified via the remaining C-terminal His-tag. Expressed fusion proteins were not toxic to the host resulting in high yields of produced protein. For antimicrobial function of the fusion protein it was necessary to remove the thioredoxin by proteolytic cleavage. Therefore the fusion protein was cleaved with 2-4 units/mg recombinant enterokinase (Novagen, Darmstadt, Germany) to remove the thioredoxin following the protocol provided by the manufacturer. After enterokinase cleavage the fusion protein was purified via His-tag purification as described below.

The Ni²⁺ affinity chromatography is performed in 4 subsequent steps, all at room temperature:
1. *Equilibration* of the *Histrap FF 5 ml* column (GE Healthcare) with up to 10 column volumes of Washing Buffer (20 mM imidazole, 1 M NaCl and 20 mM Hepes on pH 7.4) at a flow rate of 3-5 ml/min.
2. *Loading* of the total lysate (with wanted fusion protein) on the *Histrap FF 5 ml* column at a flow rate of 3-5 ml/min.
3. Washing of the column with up to 10 column volumes of Washing Buffer to remove unbound sample followed by a second washing step with 10% Elution buffer (500 mM imidazole, 0.5 M NaCl and 20 mM Hepes on pH 7.4) at a flow rate of 3-5 ml/min.
4. Elution of bounded fusion proteins from the column with a linear gradient of 4 column volumes of Elution Buffer (500 mM imidazole, 0.5 M NaCl and 20 mM Hepes on pH 7.4) to 100% at a flow rate of 3-5 ml/min.

Purified stock solutions of fusion proteins in Elution Buffer (20 mM Hepes pH 7.4; 0.5 M NaCl; 500 mM imidazole) were at least 90% pure as determined visually on SDS-PAGE gels (data not shown).

Lysin A like activity was controlled in a Chloroform assay. Escherichia coli BL21 transformed with the respective Lysin A variant were grown at 37°C in LB broth supplemented with 100 mg/mL ampicillin to an OD600 nm of 0.5 and then induced with a final concentration of 1mM IPTG. One hour after induction, 2% chloroform was added to the cell suspension and OD600nm was monitored. Chloroform permeabilizes the inner membrane, thus replacing the holin function, and allows the putative lysin to reach its target in the peptidoglycan layer. The reduction in OD600nm after addition of chloroform to 10mL of induced clones was recorded.

Lysin B like activity was controlled by enzymatic assays for lipolytic activity like from those described by Payne et al. 2009. Briefly one milliliter of p-nitrophenyl substrates (50 mM) (Sigma) was incubated with 1 mg of the lysine B variants, or 5 ml of a mock purified sample (derived from pET21 or pET32 containing cells) in buffer (20 mM Tris-HCl pH 8.0, 100 mM NaCl, 0.1% Triton X-100) at room temperature for 30 min. Release of p-nitrophenol was determined by measuring absorbance at 420 nm (A420).

### EXAMPLE 2: Antimicrobial activity of proteins modified with various peptide stretches and PTDs on the N-terminus or the C-terminus.

Mycobacteria were grown to an OD600 of 1.0. If necessary, clumps were dispersed by passing the bacterial suspension several times through a 25-gauge needle. A volume of 500 ml was added to 3 ml top agar containing 1 mM CaCl₂ and poured onto 7H10 agar plates enriched with 1 mM CaCl₂ and OADC (oleic acid, BSA, dextrose and catalase; Difco). For each modified protein, a serial dilution was prepared in storage buffer. Twenty-microlitre volumes of the original stock and of each dilution were pipetted onto the bacterial lawn, and the spots were allowed to dry completely. Plates were incubated for 4 days for the fast-growing mycobacteria, and for up to 6 weeks for the slow growing strains, at the optimal temperature for the individual strain.

**Table 7:**

| **First fusion protein** | **Second fusion protein** | **Composition comprising the first and second fusion protein** | **control** |
|---|---|---|---|
| SEQ ID NO 49: - | SEQ ID NO 63: - | SEQ ID NO 49 + SEQ ID NO 63: ++ | - |
| SEQ ID NO 51: - | SEQ ID NO 67: - | SEQ ID NO 51 + SEQ ID NO 67: ++ | - |
| SEQ ID NO 53:- | SEQ ID NO 67: - | SEQ ID NO 53 + SEQ | - |
| | | ID NO 67: + | |
| SEQ ID NO 55: - | SEQ ID NO: 71: - | SEQ ID NO 55 + SEQ ID NO 73: + | - |
| SEQ ID NO 59: - | SEQ ID NO 75: - | SEQ ID NO 59 + SEQ ID NO 75: ++ | - |
| SEQ ID NO 61: - | SEQ ID NO 73: - | SEQ ID NO 61 + SEQ ID NO 73: + | - |
| SEQ ID NO 61: - | SEQ ID NO 69: - | SEQ ID NO 61 + SEQ ID NO 69: ++ | - |

| | | | |
|---|---|---|---|
| Abbreviations: - no activity; +: 1 log; ++: 2-3 log; +++: 4 log. | | | |

The results as shown in table 7 above provide evidence that the first fusion protein and the second fusion protein alone do not exert an anti-mycobacterial activity. In contrast to this, the combination of the first and the second fusion protein in the composition according to the present invention provides a good in some examples very good activity against mycobacteria.

## Claims

1. A composition having the activity of degrading the cell wall of a *Mycobacterium species* comprising:
(a) a first fusion protein including
(i) a first endolysin or a first domain, both having a first enzymatic activity, the enzymatic activity being at least one or more of the following: N-acetyl-b-D-muramidase (lysozyme, lytic transglycosylase), N-acetyl-b-D-glucosaminidase, N-acetylmuramoyl-L-alanine amidase, L-alanoyl-D-glutamate (LD) endopeptidase, c-D-glutamyl-meso-diaminopimelic acid (DL) peptidase, L-alanyl-D-iso-glutaminyl-meso-diaminopimelic acid (D-Ala-m-DAP) (DD) endopeptidase, or m-DAP-m-DAP (LD) endopeptidase;
(ii) at least one peptide stretch fused to the N- or C-terminus of the endolysin having the first enzymatic activity or the domain having the first enzymatic activity, wherein the peptide stretch is selected from the group consisting of synthetic amphipathic peptide, synthetic cationic peptide, synthetic polycationic peptide, synthetic hydrophobic peptide, synthetic antimicrobial peptide (AMP) or naturally occurring AMP; and
(iii) a protein transduction domain (PTD) being at the N- or C-terminus of the first fusion protein, wherein the PTD is having the characteristic to deliver a cargo from the extracellular to the intracellular space of a cell; and
(b) a second fusion protein including
(i) a second endolysin or a second domain, both having a second enzymatic activity, the enzymatic activity being at least one or more of the following: lipolytic activity, cutinase, mycolarabinogalactanesterase, or alpha/beta hydrolase;
(ii) at least one peptide stretch fused to the N- or C-terminus of the endolysin having a second enzymatic activity or the domain having the second enzymatic activity, wherein the peptide stretch is selected from the group consisting of synthetic amphipathic peptide, synthetic cationic peptide, synthetic polycationic peptide, synthetic hydrophobic peptide, synthetic antimicrobial peptide (AMP) or naturally occurring AMP; and
(iii) a protein transduction domain (PTD) being at the N- or C-terminus of the second fusion protein, wherein the PTD is having the characteristic to deliver a cargo from the extracellular to the intracellular space of a cell.

2. The composition according to claim 1, wherein the first endolysin is Lysin A (LysA) or the first enzymatic activity of the first domain is exerted by Lysin A (LysA) or Lysin A like enzymes and the second endolysin is Lysin B (LysB) or the second enzymatic activity of the second domain is exerted by Lysin B (LysB) or Lysin B like enzymes.

3. The composition according to claim 1 or 2, wherein the first and second enzymatic activity is exerted by enzymes derived from mycobacteriophages selected from the group consisting of TM4, D29, L5, and Bxz2.

4. The composition according to any one of claims 1 to 3, wherein the AMP selected from the group consisting of cathelicidins (hCAP-18/LL37), alpha defensins, beta defensins, hepcidin, NK-2, and Ci-MAM-A24.

5. The composition according to any one of claims 1 to 4, wherein the PTD is selected from the group consisting of TAT48-60, TAT47-57, TAT47-55, PTD3, PolyArginine, CADY, PepFect6, RXR, Antennapedia, Kala Syn, M918, MAP, Penetratin, PTD5-Syn, Pvec, Poly Arg 8, TAT 48-60, Transportan, Transportan10, and TAT-9.

6. The composition according to any one of claims 1 to 5, wherein the *Mycobacterium species* is selected from the group consisting of *Mycobacterium tuberculosis, Mycobacterium microti, Mycobacterium africanum, Mycobacterium bovis, Mycobacterium canettii, Mycobacterium pinnipedii, Mycobacterium caprae, Mycobacterium mungi, Mycobacterium leprae, Mycobacterium ulcerans, Mycobacterium xenopi, Mycobacterium shottsii, Mycobacterium avium, Mycobacterium avium subsp. paratuberculosis, Mycobacterium paratuberculosis, Mycobacterium intracellulare, Mycobacterium smegmatis, Mycobacterium kansasii, Mycobacterium terrae, Mycobacterium nonchromogenicum, Mycobacterium gordonae, and Mycobacterium triviale.*

7. The composition according to any one of claims 1 to 6, wherein the endolysin or the domain having the first enzymatic activity of the first fusion protein exhibits an amino acid sequence selected from the group consisting of SEQ ID NO:1, 3, 5, and 7, wherein the endolysin or the domain having the second enzymatic activity of the second fusion protein exhibits an amino acid sequence selected from the group consisting SEQ ID NO:9, 11, 13, and 15, wherein the peptide stretch of the first and second fusion protein exhibits an amino acid sequence selected from the group consisting SEQ ID NO:17, 19, 21, 23, 25, and 27, and wherein the PTD exhibits an amino acid sequence selected from the group consisting SEQ ID NO:29, 30, 31, 32, 33, 34, 35, 36, 37, 38, 39, 40, 41, 42, 43, 44, 45, 46, 47, and 48.

8. The composition according to any one of claims 1 to 7, wherein the first fusion protein exhibits an amino acid sequence selected from the group consisting SEQ ID NO:49, 51, 53, 55, 57, 59, and 61, and wherein the second fusion protein exhibits an amino acid sequence selected from the group consisting SEQ ID N0:63, 65, 67, 69, 71, 73, and 75.

9. Isolated nucleic acid molecule encoding the first fusion protein of the composition according to any one of claims 1 to 8 or isolated nucleic acid molecule encoding the second fusion protein of the composition according to any one of claims 1 to 8.

10. A vector comprising a nucleic acid molecule according to claim 9.

11. A host cell comprising a nucleic acid molecule according to claim 9 or the vector according to claim 10.

12. The composition according to any one of claims 1 to 8 for use as a medicament, a disinfectant, feed additive, or food additive.

13. The composition according to any one of claims 1 to 8 for use according to claim 12 in the treatment or prevention of diseases caused by an infection with a *Mycobacterium species,* such as tuberculosis, leprosy, pulmonary disease, lymphadenitis or skin disease.

14. The composition according to any one of claims 1 to 8 for use according to claim 12 in the treatment or prevention of a contamination caused by a *Mycobacterium species* of foodstuff, of food processing equipment, of food processing plants, of surfaces coming into contact with foodstuff, of medical devices, or of surfaces in hospitals and surgeries.

15. The composition according to any one of claims 1 to 8 for use according to claim 12, wherein the composition is used in combination with an additional therapeutic agent.
